# EUROPÄISCHE PATENTSCHRIFT

(11) **EP 1 556 411 B1**
(45) Veröffentlichungstag und Bekanntmachung des Hinweises auf die Patenterteilung: **02.10.2013**
(21) Anmeldenummer: 03757951.3
(22) Anmeldetag: 10.10.2003
(51) Int. Cl.: C07K 14/54, C07K 14/705, C12N 15/861, A61K 38/17, A61K 38/20, A61K 48/00

(54) **Adenovirale Vektoren die Einzelketten Interleukin-12 und 4-1BB Ligand exprimieren**
Adenoviral vectors expressing single chain interleukin-12 and 4-1BB ligand
Vecteurs adenoviraux exprimant Interleukine-12 simple brin et ligand 4-1BB

(30) Priorität: 11.10.2002 DE 10248141
(43) Veröffentlichungstag der Anmeldung: 27.07.2005
(73) Patentinhaber: Universitätsklinikum Hamburg-Eppendorf, 20246 Hamburg (DE)
(72) Erfinder: WÄHLER, Reinhard, Birmingham, AL 35205 (US); SCHNIEDERS, Frank, 22307 Hamburg (DE)
(74) Vertreter: Weiss, Wolfgang
(86) Internationale Anmeldenummer: PCT/EP2003/011252
(87) Internationale Veröffentlichungsnummer: WO 2004/035799

(56) Entgegenhaltungen:
- WO-A-00/41508
- US-A1- 2002 018 767
- PUETZER B M ET AL: "INTERLEUKIN 12 AND B7-1 COSTIMULATORY MOLECULE EXPRESSED BY AN ADENOVIRUS VECTOR ACT SYNERGISTICALLY TO FACILITATE TUMOR REGRESSION" PROCEEDINGS OF THE NATIONAL ACADEMY OF SCIENCES OF USA, NATIONAL ACADEMY OF SCIENCE. WASHINGTON, US, Bd. 94, 1997, Seiten 10889-10894, XP000887254 ISSN: 0027-8424
- CARROLL M W ET AL: "CONSTRUCTION AND CHARACTERIZATION OF A TRIPLE-RECOMBINANT VACCINIA VIRUS ENCODING B7-1, INTERLEUKIN 12, AND A MODEL TUMOR ANTIGEN" JOURNAL OF THE NATIONAL CANCER INSTITUTE, US DEPT. OF HEALTH, EDUCATION AND WELFARE, PUBLIC HEALTH, US, Bd. 90, Nr. 24, 1998, Seiten 1881-1887, XP000909585 ISSN: 0027-8874
- MARTINET O ET AL: "T cell activation with systemic agonistic antibody versus local 4-1BB ligand gene delivery combined with interleukin-12 eradicate liver metastases of breast cancer" GENE THERAPY, Bd. 9, Nr. 12, Juni 2002 (2002-06), Seiten 786-792, XP002275865 ISSN: 0969-7128 in der Anmeldung erwähnt
- LIESCHKE G ET AL: "Bioactive murine and human interleukin-12 fusion proteins which retain antitumor activity in vivo" NATURE BIOTECHNOLOGY, NATURE PUBLISHING, US, Bd. 15, Nr. 1, Januar 1997 (1997-01), Seiten 35-40, XP002106574 ISSN: 1087-0156 in der Anmeldung erwähnt
- MELERO I ET AL: "IL-12 gene therapy for cancer: in synergy with other immunotherapies." TRENDS IN IMMUNOLOGY. ENGLAND MAR 2001, Bd. 22, Nr. 3, März 2001 (2001-03), Seiten 113-115, XP004255858 ISSN: 1471-4906
- EMTAGE P C ET AL: "A double recombinant adenovirus expressing the costimulatory molecule B7-1 (murine) and human IL-2 induces complete tumor regression in a murine breast adenocarcinoma model." JOURNAL OF IMMUNOLOGY (BALTIMORE, MD.: 1950) UNITED STATES 1 MAR 1998, Bd. 160, Nr. 5, 1. März 1998 (1998-03-01), Seiten 2531-2538, XP002959615 ISSN: 0022-1767 in der Anmeldung erwähnt
- GUINN B A ET AL: "4-1BBL cooperates with B7-1 and B7-2 in converting a B cell lymphoma cell line into a long-lasting antitumor vaccine." JOURNAL OF IMMUNOLOGY (BALTIMORE, MD.: 1950) UNITED STATES 15 APR 1999, Bd. 162, Nr. 8, 15. April 1999 (1999-04-15), Seiten 5003-5010, XP000887237 ISSN: 0022-1767 in der Anmeldung erwähnt
- ADDISON C L ET AL: "Intratumoral coinjection of adenoviral vectors expressing IL-2 and IL-12 results in enhanced frequency of regression of injected and untreated distal tumors" GENE THERAPY, Bd. 5, Nr. 10, Oktober 1998 (1998-10), Seiten 1400-1409, XP002275866 ISSN: 0969-7128
- ANDERSON R. ET AL: "Construction and biological characterisation of an interleukin-12 fusion protein (Flexi-12): delivery to acute myeloid leukemic blasts using adeno-associated virus", HUMAN GENE THERAPY, vol. 8, 10 June 1997 (1997-06-10), pages 1125-1135,

## Beschreibung

Die vorliegende Erfindung betrifft adenovirale Vektoren, die Nukleinsäure-Sequenzen umfassen, welche für Einzelketten-Interleukin (IL)-12 (Einzelketten-IL-12, "single chain IL-12" oder scIL-12) und das Kostimulator-Protein 4-1BB kodieren, sowie die Verwendung dieser Vektoren für die Gentherapie, insbesondere für die Therapie von Tumoren.

Krebserkrankungen stellen nach wie vor eine der häufigsten Todesursachen von Menschen in Industriestaaten dar. Das hepatozelluläre Karzinom (HCC) ist beispielsweise eine Krebserkrankung mit einer mittleren Überlebensdauer von 6 Monaten bei Diagnose eines oder mehrerer größerer Tumore (Llovet J.M. et al., Hepoatology, 1999, 29:62-67). Die gegenwärtig verwendeten Therapieansätze, welche Radiofrequenzablation, Chemotherapie und perkutane Ethanolinjektion (PEI) umfassen, weisen zwar bei kleinen Tumoren gewisse Erfolge auf, erweisen sich jedoch bei der Bekämpfung großer Tumore als unzureichend.

Im Stand der Technik wurde daher vorgeschlagen, HCC durch Gentherapie zu behandeln. Gentherapeutische Behandlungsverfahren basieren auf der Verabreichung einer Nukleinsäure, die üblicherweise in die Tumorzelle aufgenommen wird und die Sequenzen aufweist, welche die Tumorzelle zerstören. Dabei wurden eine Vielzahl alternativer Strategien entwickelt, mittels derer die übertragenen Nukleinsäuresequenzen eine Zerstörung der Tumorzellen bewirken können. Eine Übersicht entsprechender Strategien zur Behandlung des HCC findet sich Ruiz et al., (Dig.Dis. 2001, 19:324-332). In dieser Veröffentlichung werden die Nukleinsäuren, die derzeit in klinischen Versuchen für eine Behandlung des HCC beim Menschen untersucht werden, nach der Behandlungsstrategie eine der folgenden 4 Gruppen zugeordnet:
(1) Übertragung von Tumorsuppressorgen:
   Diese Strategie basiert darauf, daß die für die Gentherapie verwendete Nukleinsäure ein Gen enthält, dessen Genprodukt das Wachstums des Tumors hemmt oder in den Tumorzellen Apoptose induziert. Die meisten klinischen Versuche basieren auf einer Übertragung des p53 Gens.
(2) Immungentherapie:
   Diese Strategie basiert darauf, daß die für die Gentherapie verwendete Nukleinsäure Sequenzen umfaßt, deren Genprodukte das Immunsystem des Patienten aktivieren und eine gegen die Tumorzellen gerichtete Immunreaktion auslösen. Die Immunreaktion selbst führt dann zur Zerstörung des Tumors. Zahlreiche Zytokine, Kostimulator-Moleküle und Tumor-spezifische Moleküle wurden für eine Immungentherapie vorgeschlagen worden.
      Lieschke et al. exprimieren Interleukin-12 in CMS-5 Tumorzellen.
(3) Suizid-Gentherapie:
   Bei dieser Vorgehensweise kodiert die für die Gentherapie verwendete Nukleinsäure für ein Genprodukt, beispielsweise für ein Enzym, das einen nicht-toxischen Wirkstoff in ein für die Tumorzelle zytotoxisches Agens umwandelt.
(4) Übertragung onkolytischer Viren:
   Für diese Form der Gentherapie werden Nukleinsäurevektoren verwendet, die auf viralen Sequenzen basieren. Vektoren mit onkolytischen, viralen Sequenzen weisen einen Tumor-spezifischen Promotor auf, welcher die Replikation des Virus steuert, so daß ein selektives Wachstum der Viren in den Tumorzellen ermöglicht wird.

Bei der für die vorliegende Anmeldung relevanten Immungentherapie (auch als Immuntherapie bezeichnet) werden somit Nukleinsäuren verabreicht, die Sequenzen umfassen, welche das Immun-system aktivieren und auf den Tumor richten. Grundsätzlich erkennt das Immunsystem neben Antigenen auch Tumor-spezifische Strukturen auf Tumorzellen. Die Aktivierung des Immunsystems kann daher zu einer Zerstörung des Tumors durch die Bestandteile des Immunsystems führen.

WO 00/41508 beschreibt eine Kombinationstherapie von Krebs durch Aktivierung von Kostimulatorischen Molekülen der Immunzellen, mittels zwei adenoviraler Vektoren die entweder für die zwei verschiedene Untereinheiten von IL-12 oder für 4-1BB Ligand kodieren.

Im Stand der Technik sind zahlreiche Moleküle bekannt, die das Immunsystem stimmulieren oder eine Immunreaktion modulieren, insbesondere die Zytokine. Schon früh wurde festgestellt, daß Zytokine auch antitumorale Aktivitäten aufweisen. Beispielsweise wurde berichtet, daß IL-12 ein Stimulator der zellulären Immunität ist und starke Antitumor-Aktivität aufweist (Brunda et al, J. Exp. Med. 1993, 178:1223-1230). Die Verabreichung des rekombinanten IL-12 Proteins selbst als Antitumormittel scheiterte jedoch daran, daß das Zytokin in therapeutischer Dosierung toxische Nebenwirkungen aufweist (Lotze et al., Ann.N.Y.Acad.Sci., 1997, 795:440-454; und Cohen J. Science, 1995, 270:908).

Es wurde daher vorgeschlagen, eine für ein Zytokin kodierende Nukleinsäure in den Tumor einzubringen und somit eine lokale Aktivierung des Immunsystems zu ermöglichen. Hock et al. (Proc. Natl. Acad.Sci. USA, 1993, 90:2774-2778) beschreiben beispielsweise den Transfer des Interleukin-2 (IL-2), Interleukin-4 (IL-4), Interleukin-7 (IL-7), TNF oder IFN-γ Gens in Tumorzellinien und die Verwendung der Tumorzellinien zur Induktion von Tumoren in Tieren. Alle transgenen Tumorzellinien erzeugten eine Abstoßungsreaktion gegen die Tumorzellen, wobei in Abhängigkeit des verwendeten Zytokins verschiedene Zellen des Immunsystems der Versuchstiere an der Abstoßungsreaktion beteiligt waren (CD4⁺, CD8⁺, CD3⁺).

Auch Vektoren, die für IL-12 kodieren, wurden auf deren Eignung für die Immuntherapie hin untersucht. IL-12, das auch als CMLF ("cytotoxic lymphocyte maturation factor") oder NKSF ("natural killer cell stimulatory factor") bezeichnet wird, ist ein heterodimeres Zytokin, das natürlicherweise von peripheren B-Lymphozyten nach Aktivierung gebildet wird. Das Protein besteht aus zwei Untereinheiten mit relativen Molekulargewichten von 40 und 35 kDa, die über Disulfidbrücken miteinander verbunden sind. Die Disulfidbrücken sind für die biologische Aktivität essentiell. Wie bereits durch die verschiedenen Namen angedeutet, stimuliert das Protein die Proliferation aktivierter humaner Lymphoblasten und aktiviert Natural-Killer-Zellen.

In Pützer et al. wurden Interleukin-12 und B7-1 durch einen adenoviralen Vektor in Tumorzellen exprimiert. In Carrol et aL wurden Vaccina-Viren zur Expression von B7-1 und Interleukin-12 eingesetzt.

Vektoren, die für die verschiedenen Untereinheiten dieses Proteins kodieren, wurden für die Behandlung von Tumoren verwendet (Barajas et al., Hepatology, 2001, 33:52-61; Mazzolini et al., Cancer Gene Therapy, 1999, 6:514-522). Ferner wurden diese Vektoren in Kombination mit anderen Sequenzen für die Immuntherapide, insbesondere in Kombination mit Sequenzen für ein Kostimulator-Protein, welche auf demselben oder einem anderem Vektor vorlagen, für die Behandlung von Tumoren verwendet (Gyorffy et al., J.Immunology, 2001, 166:6212-6217; Martinet et al., Gene Therapy, 2002, 9:786-792; Martinet et al., Journal of National Cancer Institute, 2000, 92:931-936; Guinn et al., J. Immunology, 1999, 162:5003-5010; und Emtage et al., J. Immunology, 1998, 160:2531-2538).

Anderson et al. verwendeten Adeno-assoziierte Viren, die in den Zellen ein spezifisches Interleukin-12 Fusionsprotein exprimieren.

IL-12 ist ferner bereits als Einzelketten-IL-12 mit guter Aktivität exprimiert worden, also als Protein, bei dem die verschiedenen Untereinheiten zu einem Fusionsprotein verknüpft wurden (Lieschke et al., Nature Biotechnology, 1997, 15:35-40). In einem weiteren Therapieverfahren wurde vorgeschlagen, dem Patienten Tumorzellen zu entnehmen und diese in vitro mit einem Plasmid zu behandeln, das für Einzelketten-IL-12 oder IL-12 und einen Kostimulator kodiert (US2002/0018767). Nach dieser in vitro Behandlung sollen die Tumorzellen reimplantiert werden. Das Verfahren umfaßt somit mehrere Eingriffe am Patienten und eine Reimplantation von Tumorzellen in den Patienten, was viele Patienten von einer entsprechenden Behandlung abhalten dürfte.

Keine der bisher verwendeten Nukleinsäuren konnte sich für die Behandlung von Säugetieren, vorzugsweise für die Behandlung des Menschen durchsetzen. Obwohl beispielsweise die in der Veröffentlichung von Ruiz et al. (a.a.O.) beschriebenen Behandlungsverfahren von einer sehr hohen Dosierung des für die Therapie verwendeten Vektors ausgehen (3×10⁹-2,5×10¹³ "plaque forming units", PFU, pro Dosierung), wird festgestellt, daß entweder keine oder nur negative Ergebnisse durch die entsprechenden klinischen Versuche erhalten wurden. Gerade die Dosierung der Nukleinsäuren ist jedoch für die Gentherapie ein kritischer Faktor, da bei zu hoher Dosierung Nebenwirkungen oder eine Freisetzung des Vektors durch das Austreten des Vektors aus dem Tumor zu erwarten sind.

Der vorliegenden Erfindung lag somit die Aufgabe zugrunde, Vektoren zur Verfügung zu stellen, die mit verbesserter Wirksamkeit für die Immuntherapie verwendet werden können.

Diese Aufgabe wurde nunmehr überraschenderweise durch virale Vektoren gelöst, die Nukleinsäuresequenzen umfassen, welche für Einzelketten-IL-12 und das Kostimulator-Protein 4-1BB kodieren, wobei der Vektor ein adenoviraler Vektor ist.

Erfindungsgemäß wurde somit überraschenderweise festgestellt, daß Vektoren, welche für Einzelketten-IL-12 und das Kostimulator-Protein 4-1BB kodieren, in besonderem Maße zur Behandlung von Tumoren im Rahmen einer Immuntherapie geeignet sind. Durch die Immuntherapie werden nicht nur der Primärtumor sondern auch die Metastasen eliminiert. Die synergistische Wirkung der durch die erfindungsgemäßen Konstrukte kodierten Proteine bei der Immunstimulierung ermöglicht den Einsatz der Nukleinsäuren in geringeren Dosierungen, als dies im Stand der Technik vorgeschlagen wurde. Die geringere Dosierung weist geringere Nebenwirkungen, wie ein geringeres Risiko einer Autoimmunerkrankung für den Patienten, und gleichzeitig verbesserte Sicherheit beim klinischen Einsatz auf. Die Wahrscheinlichkeit einer Verbreitung des Virus ist geringer als bei den bekannten Vektoren. Die geringere Dosierung ermöglicht es ferner, größere Tumormassen oder mehrere Tumorherde gleichzeitig zu behandeln, ohne Nebenwirkungen zu erzeugen.

Die Verwendung des Einzelketten-IL-12-Gens spart im Vergleich zu der Verwendung von Genen, die für die beiden Untereinheiten des IL-12 kodieren Platz in dem Vektor für die Expression der Nukleinsäuren. Diese Sequenz ermöglicht somit die Verwendung relativ kleiner Vektoren, wie beispielsweise der adenoviralen Vektoren, in denen trotzdem zwei oder mehr Fremdgene, welche die Immuntherapie verstärken, neben dem Gen für Einzelketten-IL-12 vorliegen.

Die erfindungsgemäßen Vektoren sind zur Behandlung von Säugetieren, insbesondere zur Behandlung von Menschen, bestimmt. Die folgenden Verweise auf bestimmte Gensequenzen beziehen sich demgemäß vorzugsweise auf humane Sequenzen. Die Gensequenzen können jedoch auch von anderen Arten abstammen oder im Rahmen der angegebenen Homologiebereiche (die für die vorliegende Anmeldung mittels der BLAST-Software ermittelt werden) mit im Stand der Technik bekannten Verfahren modifiziert werden, sofern die Aktivität der Proteine (immunstimulierend und/oder T-Zellbindung) im Bereich von mindestens 50%, vorzugsweise mindestens 70% der entsprechenden Aktivität des humanen Genprodukts bleibt. Die angegebenen Homologiebereiche beziehen sich auf den Bereich, der im nativen Gen für die biologische Aktivität kodiert. Sofern Nukleinsäuresequenzen genutzt werden, die für Fusionsproteine kodieren, bezieht sich der Homologiebereich somit auf den Teil, der für die genannte biologische Aktivität (IL-12, Kostimulator 4-1BB) kodiert. Veränderungen der Gensequenz, welche zu einer Verstärkung der Aktivität der Proteine führen, sind umfaßt.

Im Rahmen der vorliegenden Erfindung wird der Begriff "Sequenzen, die für ein Kostimulator-Protein kodieren" dazu benutzt, um Sequenzen zu bezeichnen, welche bei Expression in humanen Zellen Proteine erzeugen, die als Zelloberflächenproteine vorliegen und spezifisch von Rezeptoren der T-Zellen gebunden werden. Bei Bindung einer T-Zelle verstärken die Kostimulator-Proteine die Immunreaktion. Entsprechende Kostimulator-Proteine, beispielsweise 4-1BB Ligand (4-1BBL), B7-1 (auch als CD80 bezeichnet) und B7-2, sind im Stand der Technik bekannt.

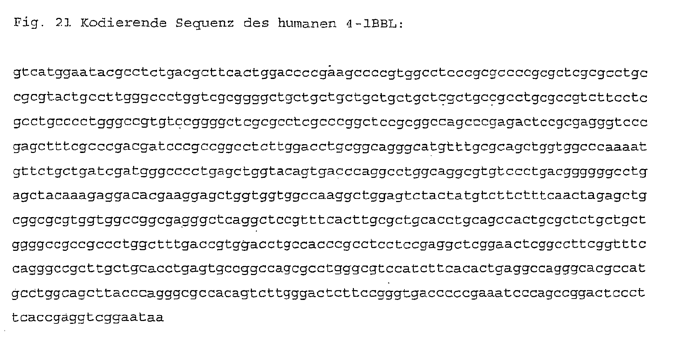

Gemäß einer bevorzugten Ausführungsform der vorliegenden Erfindung umfaßt der Vektor Sequenzen, welche für das Kostimulator-Protein 4-1BB Ligand kodieren, insbesondere Sequenzen mit einer Sequenzhomologie von mindestens 40%, vorzugsweise mindestens 70%, mindestens 80% oder mindestens 90%, zu der in Fig. 21 gezeigten Sequenz, wobei das von der Sequenz kodierte Protein die Fähigkeit aufweist, T-Zellen spezifisch zu binden. Weitere Aktivitätstest für 4-1BBL sind im Stand der Technik bekannt (Vinay DS, Kwon BS. Semin. Immunol., 1998, 10:481-9. Review; Kwon et al., Mol Cells., 2000, 10:119-26).

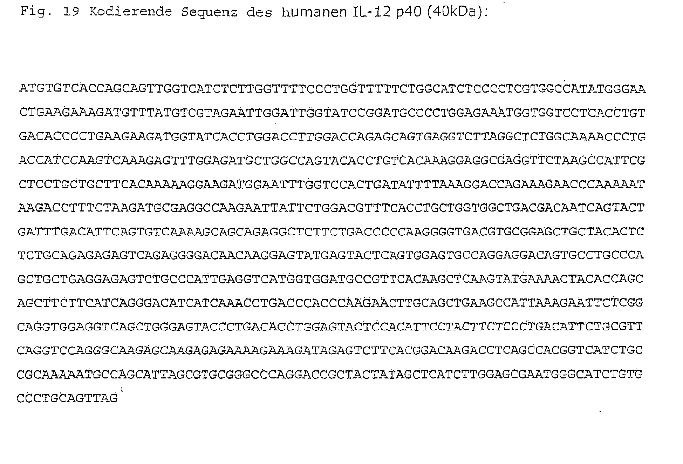

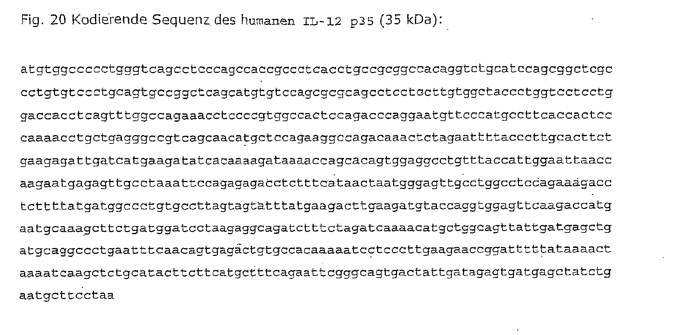

Im Rahmen der vorliegenden Erfindung wird ein Protein als Einzelketten-IL-12 bezeichnet, wenn das Protein aus einer Aminosäuresequenz besteht, welche die beiden Untereinheiten des natürlichen IL-12 als Fusionsprotein umfaßt. Nukleinsäuresequenzen, die für ein Einzelketten-IL-12 kodieren, werden üblicherweise eine Sequenzhomologie von mindestens 40%, vorzugsweise mindestens 70%, mindestens 80% oder mindestens 90%, zu den in Fig. 19 und 20 gezeigten Sequenzen aufweisen. Die gezeigten Sequenzen stellen den IL-12 Anteil des Fusionsgens dar. Sequenzen, welche die Untereinheiten verknüpfen, sind dem Fachmann bekannt und werden bei der Ermittlung der Homologie nicht berücksichtigt. Das Einzelketten-IL-12 weist ferner eine immunstimulierende Aktivität auf, die nicht wesentlich schlechter als die entsprechende Aktivität des natürlichen IL-12 in heterodimerer Form ist. Eine der Wirkungen des humanen IL-12 bei der Immunstimulierung besteht darin, die Ausschüttung von gamma-Interferon zu initiieren. Die immunstimulierende Aktivität des Einzelketten-IL-12 beträgt mindestens 50%, vorzugsweise mindestens 70% der entsprechenden Aktivität des natürlichen IL-12. Die Aktivität der Proteine kann durch bekannte in vitro Testverfahren verglichen werden (beispielsweise unter Verwendung der vitro Tests zum Aktivitätsvergleich von IL-12 in Lieschke et al., a.a.O.). Gemäß einer besonders bevorzugten Ausführungsform der Erfindung ist die immunstimulierende Aktivität des Einzelketten-IL-12 sogar höher.oder wesentlich höher als die entsprechende Aktivität des natürlichen IL-12.

In einer weiteren Ausführungsform umfaßt die vorliegende Erfindung ferner Vektoren, welche für weitere Zytokine, für Proteine mit Zytokin-Aktivität und/oder für Kostimulator-Proteine kodieren. Als Proteine mit Zytokin-Aktivität werden Proteine bezeichnet, welche die immunstimulatorische Aktivität eines Zytokins aufweisen, aber keine strukturelle Verwandschaft zu den Zytokinen aufweisen. Entsprechende Zytokin-Agonisten, beispielsweise agonistische Zytokin-Rezeptor-Antikörper, sind im Stand der Technik bekannt.

Neben den spezifisch genannten Sequenzen, welche für Einzelketten-IL-12 und das Kostimulator-Protein 4-1BB kodieren, können die erfindungsgemäßen Vektoren somit Sequenzen aufweisen, welche für ein oder mehrere weitere Zytokine, welches T- und/oder BZellen aktiviert, oder eine oder mehrere weitere Kostimulator-Proteine kodieren.

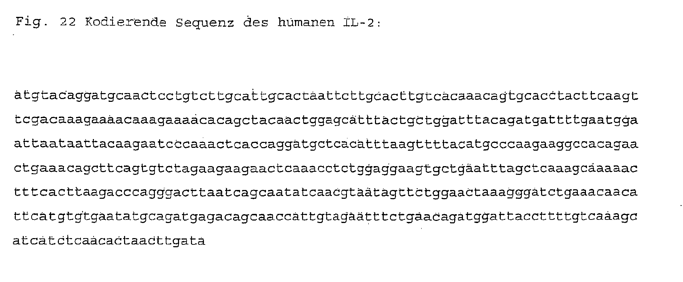

Die Erfindung betrifft insbesondere Vektoren, welche Sequenzen umfassen, die für Einzelketten-IL-12, 4-1BB Ligand und IL-2 kodieren. Ein Protein wird im Rahmen der vorliegenden Erfindung als IL-2 bezeichnet, wenn es von einer Sequenz kodiert wird, welche eine Sequenzhomologie von mindestens 40%, vorzugsweise von mindestens 70%, mindestens 80% oder mindestens 90%, zu der in Fig. 22 gezeigten Sequenz aufweist. Die im Rahmen der vorliegenden Erfindung eingesetzten für IL-2 kodierenden Sequenzen weisen ferner im wesentlichen die immunstimulierende Aktivität des natürlichen IL-2 auf, d.h. im Rahmen der vorliegenden Erfindung werden für IL-2 kodierende Sequenzen eingesetzt, die in vitro eine immunstimulierende Aktivität aufweisen, welche mindestens etwa 70% der Aktivität des natürlichen IL-2 entspricht. Entsprechende in vitro Testverfahren zur Bestimmung der IL-2 Aktivität sind im Stand der Technik bekannt. Vorzugsweise wird das in Gillis et al. (J Immunol., 1978, 120(6):2027-32) beschriebene Verfahren für die Aktivitätsbestimmung verwendet.

In einer weiteren Ausführungsform umfassen die erfindungsgemäßen Vektoren neben den Sequenzen, welche für Einzelketten-IL-12, 4-1BB Ligand und IL-2 kodieren, ferner Sequenzen, die für das Kostimulator-Protein B7-1 und/oder B7-2 kodieren.

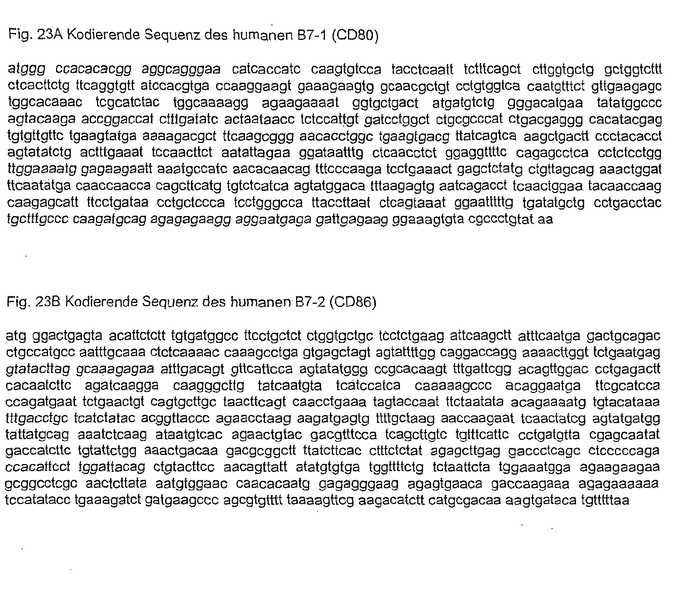

Im Rahmen der vorliegenden Erfindung wird ein Kostimulator-Protein als B7-1 (oder B7-2) bezeichnet, wenn es von einem Gen kodiert wird, das eine Sequenzhomologie von mindestens 40%, vorzugsweise mindestens 70%, mindestens 80% oder mindestens 90% zu der in Fig. 23A (oder B) gezeigten Sequenz aufweist.

Gemäß einer Ausführungsform der vorliegenden Erfindung umfassen die Vektoren ferner Sequenzen, welche die Expression der kodierenden Sequenzen ermöglichen. Die erfindungsgemäßen Vektoren können somit einen Promotor und eine oder mehrere interne Ribosomeneintrittsstellen (IRES) umfassen. Die Promotoren können Tumor-Spezifität aufweisen, also nur in dem Tumor exprimiert werden, oder nicht in allen Zellen aktiv sein.

In bestimmten Ausführungsformen der Erfindung sind nicht-spezifische Promotoren bevorzugt, da entsprechende Promotoren in der Regel besser exprimiert werden und diese Vektoren zur Behandlung verschiedener Tumore eingesetzt werden können.

Besonders hohe Expression der immunstimulierenden Gene wurde erfindungsgemäß mit Vektoren erhalten, die mindestens tricistronisch und ferner dadurch gekennzeichnet sind, daß sie pro Expressionskassette nur einen Promotor enthalten und für jedes Cistron, das sich nicht unmittelbar hinter dem Promotor beindet, eine IRES Sequenz aufweisen. Bei Verwendung mehrer Promotoren in einer Expressionskassette wurde nämlich festgestellt, daß diese sich gegenseitig inhibieren können. Eine Kombination von Promotoren und IRES-Sequenzen führte zu besserer Expression. Bei Verwendung voneinander verschiedener IRES-Sequenzen in einem Vektor ergibt sich der weitere Vorteil, daß die Rekombinationsfrequenz unter diesen Sequenzen minimiert werden kann.

Bei Verwendung tetracistronischer Vektoren kann es vorteilhaft sein, die Cistrons auf mehrere Expressionskassetten aufzuteilen (vgl. Beispiele). In diesem Fall liegt vorzugsweise pro Expressionskassette ein Promotor vor. Durch die Aufteilung in zwei Expressionskassetten, die vorzugsweise in dem Vektor maximalen Abstand voneinander aufweisen, werden die Promotoren räumlich getrennt und die gegeneseitige Hemmung somit verringert.

Die beschriebenen Sequenzen weisen den besonderen Vorteil auf, das die Proteine besonders gut in humanen Zellen exprimiert werden. Die vorteilhafte Wirkung der beschriebenen Sequenzen für die Immuntherapie ist somit gemäß dieser Ausführungsform auch in der hohen Expression der kodierenden Sequenzen begründet.

Im Rahmen der vorliegenden Erfindung bestehen die Vektoren vorzugsweise aus DNA.

Im Rahmen der vorliegenden Erfindung wird ein Vektor als "viraler Vektor" bezeichnet, wenn es sich um eine Nukleinsäuresequenz handelt, die Sequenzen adenoviralen Ursprungs umfaßt, welche die Verpackung der Nukleinsäure in Virushüllen erlauben.

In Abhängigkeit des viralen Ursprungs der Sequenzen können die Vektoren als adenovirale Vektoren, adeno-assoziierte Vektoren, lentivirale Vektoren, HSV-Vektoren, retrovirale Vektoren, baculovirale Vektoren oder Semliki-Forrest-Virus Vektoren vorliegen. Bei den adenoviralen Vektoren kann es sich um adenovirale Vektoren der ersten (Deletionen in den Regionen E1 und E3 des AdEasy-Klonierungssystems; beispielsweise erhältlich von QBiogene GmbH, Heidelberg) oder zweiten Generation (Deletionen in E1, E2, E3, E4, etc.) oder Helfer-abhängige adenovirale Vektoren handeln. Entsprechende Vektoren sind im Stand der Technik umfassend bekannt ( Nicklin SA, Baker AH. Curr Gene Ther., 2002, 2:273-93; Mah et al., Clin Pharmacokinet., 2002, 41:901-11).

In einer besonders bevorzugten Ausführungsform betrifft die Erfindung einen adenoviralen Vektor, der Sequenzen umfaßt, welche für Einzelketten-IL-12, 4-1BB Ligand und IL-2 kodieren. Adenovirale Vektoren weisen den besonderen Vorteil auf, dass es entsprechende Vektoren gibt, welche für die Verwendung in der Gentherapie des Menschen zugelassen sind. Die Vektoren sind für bestimmte Anwendungen (z.B Tumorbehandlung bei lokaler Verabreichung) somit sicher. Adenovirale Vektoren gehören zu den Vektorsystemen, die in der Klinik am häufigsten verwendet werden und zu denen die meisten Daten über die Sicherheit der Verwendung vorliegen.

Die Menge der fremden Nukleinsäuresequenzen, die in adenovirale Vektoren aufgenommen werden kann, ist jedoch beschränkt. Bisher war es daher nicht möglich, mehr als zwei für immunstimulierende Proteine kodierende Gene in einen entsprechenden Vektor aufzunehmen. Die nachfolgend im Detail beschriebene Vektorentwicklung löst dieses Problem erstmals und ermöglicht somit adenovirale Vektoren, welche im Vergleich zu den bekannten Vektoren für mehr immunstimulierende Proteine kodiert. Durch die vorliegende Erfindung werden somit besonders vorteilhafte adenovirale 3-Genvektoren, die auch als tricistronische Vektoren bezeichnet werden können, zu Verfügung gestellt. Ferner werden erstmals adenovirale Vektoren zur Verfügung gestellt, die 4 Gene exprimieren, wobei diese in den Beispielen in zwei Expressionskassetten aufgeteilt wurden.

Die vorliegende Erfindung betrifft ferner Viruspartikel, welche die erfindungsgemäßen Vektoren umfassen. Als Viruspartikel oder. Virionen werden Nukleinsäuren bezeichnet, die von den Hüllproteinen eines Virus umschlossen sind.

Gemäß einer weiteren Ausführungsform betrifft die vorliegende Erfindung Arzneimittel, welche die erfindungsgemäßen Vektoren oder Viruspartikel umfassen. Die erfindungsgemäßen Vektoren oder Viruspartikel können einfach mit einem Träger vermischt oder zusammen mit weiteren Hilfsstoffen verabreicht werden. Die erfindungsgemäßen Vektoren oder Viruspartikel können beispielsweise in Liposomen oder Liposomen mit Replikations-kompetenten Adenoviren (RCAs; vgl. Yoon et al., Curr Cancer Drug Targets, 2001, 1:85-107) eingebracht werden, als Polyethylenglykol-ummantelte Adenoviren, als Antikörper-verbrückte Adenoviren (also als Viren, die an einen Antikörper gebunden sind, welcher Spezifität für das Virus und einen Zellmarker, vorzugsweise einen Tumor-Zellmarker, aufweist), mit RCAs vermischt, als Kassette in einem RCA oder als für die Vermehrung im Tumor konditionelles RCA vorliegen (konditionelles RCA: RCA mit der E1-Funktion unter der Regulation eines Tumor-spezifischen Promotors; van der Poel et al., J Urol., 2002, 168:266-72).

Die genaue Dosierung der Viruspartikel hängt von der zu behandelnden Erkrankung, der Art der Applikationsform und der Struktur des verwendeten Vektors ab und kann im Einzelfall vom Fachmann durch Standardverfahren bestimmt werden. Die erfindungsgemäßen Nukleinsäuren ermöglichen eine Zerstörung oder signifikante Reduzierung des Tumors bereits bei besonders geringer Dosierung. Das Arzneimittel weist vorzugsweise eine Konzentration pro Dosiereinheit von nicht mehr als 1×10¹¹, vorzugsweise nicht mehr als 1×10¹⁰, nicht mehr als 1×10⁹ oder nicht mehr als 1×10⁷ auf. Die Dosierung kann jedoch auch deutlich unter den genannten Bereichen liegen und sogar nicht mehr als 1x10⁶ betragen. Die Dosierungangaben beziehen sich hier auf die Anzahl infektiöser Viruspartikel. In einem Ratten Tumormodel konnte gezeigt werden, daß alle Tiere die Tumorinjektion über eine Zeit von mehr als einem Jahr bei einer Behandlung mit einer Dosierung von 5x10⁷ infektiösen Viruspartikeln überlebten. Eine Behandlung mit einer Dosierung von 5x10⁶ infektiösen Viruspartikeln überlebten noch etwa 90% der Tiere. Demgegenüber starben alle Tiere der Kontrollgruppe in den ersten 50 Tagen nach Tumorinjektion. Diese Dosierungen liegen um mehrere Größenordnungen unter den Dosierungen, die im Stand der Technik für entsprechende Behandlungen vorgeschlagen wurden.

Das Arzneimittel ist so formuliert, daß die Vektoren gut an den Tumor abgegeben werden können. Vorzugsweise liegt das Arzneimittel als Lösung für die intratumorale Injektion vor. Die Herstellung entsprechender Lösungen ist im Stand der Technik bekannt. Alternativ dazu kann das Arzneimittel als Trägermaterial formuliert sein, welches den Vektor nach Implantation in den Tumor über einen gewissen Zeitraum freisetzt. Entsprechende Trägermaterialien, wie beispielsweise Zellulosesulfat oder ähnliches, sind im Stand der Technik bekannt.

Die vorliegende Erfindung betrifft schließlich die Vektoren oder Viruspartikel zur Behandlung von Tumoren, insbesondere zur Behandlung solider Tumore, wie HCC, Darmkrebs, Brustkrebs, etc. beim Menschen.

Gemäß einer alternativen Ausführungsform betrifft die vorliegende Erfindung die Verwendung der Vektoren oder Viruspartikel zur Behandlung von Infektionskrankheiten oder Prionenerkrankungen. Eine immunstimulierende Therapie, auch in Form einer Gentherapie, ist für die Behandlung entsprechender Infektionserkrankungen bereits vorgeschlagen worden (vgl. van der Meide et al., Vaccine., 2002, 20:2296-302).

Die immunstimulatorische Wirkung der erfindungsgemäßen Vektoren und Viruspartikel weisen somit ferner therapeutisches Potenzial für der Behandlung von Infekitonskrankheiten auf, wie zum Beispiel für die Behandlung von Infektionen durch das humane Immundefizienzvirus (HIV), durch Hepatitisviren Typ A, B, C (HAV, HBV, HCV), durch Cytomegalieviren (CMV) und durch humane Papillomaviren HPV, die unter anderem zu Cervicalkarzinomen führen können. Auch für die Therapie von Prionerkrankungen können die Vektoren oder Viruspartikel vorteilhaft eingesetzt werden, da hier unspezifische Immunstimulation bereits im Tiermodell zu Heilungserfolgen geführt hat (Sethi et al., Lancet, 2002, 360:229-30).

Für die erfindungsgemäße medizinische Verwendung liegt der Vektor in einer Konzentration von nicht mehr als 1×10¹¹, vorzugsweise nicht mehr als 1×10¹⁰, nicht mehr als 1×10⁹ oder nicht mehr als 1×10⁷ vor. Die Dosierung kann jedoch auch deutlich unter den genannten Bereichen liegen und sogar nicht mehr als 1x10⁶ betragen. Die Dosierungangaben beziehen sich hier wiederum auf die Anzahl infektiöser Viruspartikel.

Die folgenden Beispiele illustrieren die Erfindung. Detailierte Angaben zur Herstellung und Verwendung der in den Beispielen genannten Vektoren finden sich ferner in der Dissertation von Reinhard Wähler mit dem Thema "Adenovirale Immuntherapie solider Tumore am HCC-Modell der Ratte (Rattus norvegicus, Berkenhout 1769)" des Fachbereichs Biologie der Universität Hamburg.

### Beispiele

I. Konstruktion der Vektoren Ad-1, Ad-2, Ad-3 und deren Testung in vitro und in vivo
II. Konstruktion des Vektors Ad-4

### I. Konstruktion der Vektoren Ad-1, Ad-2, Ad-3 und deren Testung in vitro und in vivo

### 1. Herstellung der Vektoren

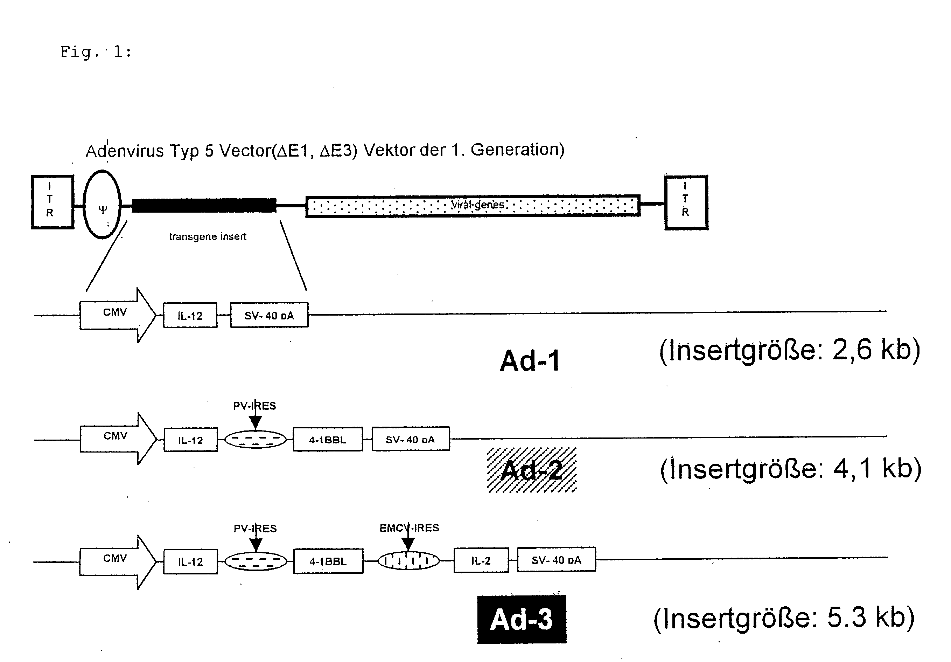

Zunächst wurden die murinen cDNAs von scIL-12, 4-1BBL und IL-2 in das Plasmid pTrident3 (Fig. 1) kloniert. Das Resultat heißt pT3 scIL12[IRES]4-1BBL[IRES]IL2 (ohne Abbildung). Zuvor wurden die 5'-Bereiche der Leserahmen der drei Komponenten und deren Abstände zu den IRES-Elementen zur Bildung optimierter Translationsinitiation modizifiert.

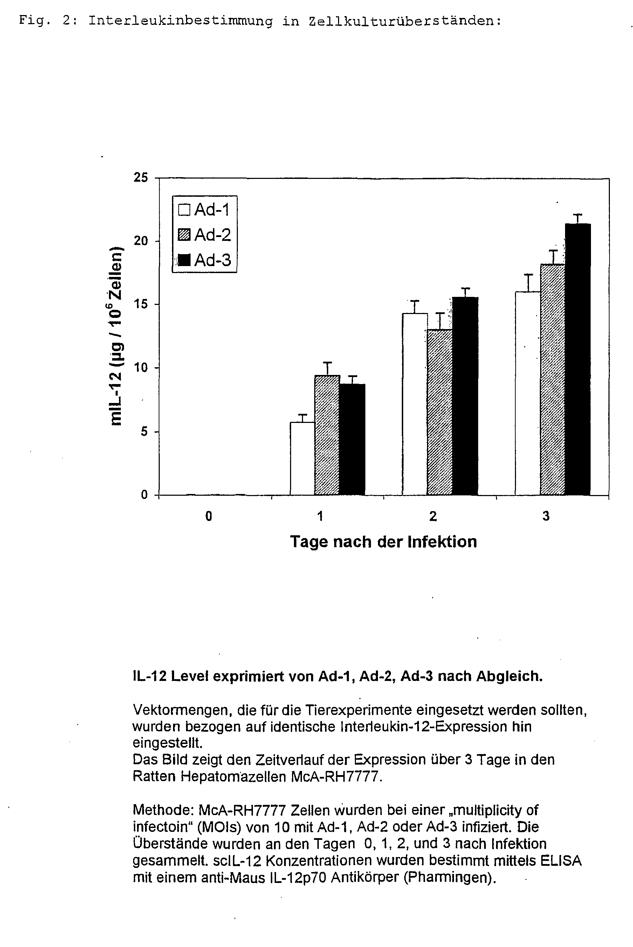

Die so konstruierte tricistronische Expressionskassette wurde ohne den Promoter und die 3'-untranslatierten Sequenzen in das pShuttle-CMV Plasmid des AdEasy-Systems (QBiogene GmbH, Heidelberg)kloniert. Das Resultat ist das Plasmid pShuttle [CMV]scIL12[IRES]4-1BBL [IRES] IL2 (Fig. 2). Die Kassette [CMV]scIL12[IRES]4-1BBL[IRES]IL2 aus dem letztgenannten Plasmid wurde komplett per DNA-Sequenzierung auf ihre Korrektheit hin überprüft.

Dieses Konstrukt wurde dann nach Kotransformation mit dem Plasmid pAdEasyl in dem E. coli Stamm Bj5183 zu dem Plasmid pAd-3 (siehe Fig. ) rekombiniert, das die gesamte rekombinante DNA für Ad-3 enthält benannt ist.

Ausgehend von pShuttle-[CMV]scIL12[IRES]4-1BBL[IRES]IL2 wurden die Plasmide für Ad-2, pShuttle-[CMV]scIL12[IRES]4-1BBL (vgl. Fig. ) und für Ad-1, pShuttle-[CMV]scIL12 (vgl. Fig.) kloniert und in analoger Weise zur Herstellung von pAd-3 wurden die Plasmide pAd-2 und pAd-1 generiert.

Die Adenoviren wurden aus den Plasmidvorstufen nach Freisetzung mittels PacI-Verdau in HEK293 Zellen transfiziert und die entstandenen viralen Plaques (also als Viruspartikel) isoliert und vermehrt.

Übersicht zu den Expressionskassetten: Fig. 1.

### Expressionstests

### 2.1. IL-12

Ratten Hepatomazellen, McA-RH7777, wurden mit den Viren (Viruspartikeln) Ad-1, Ad-2, und Ad-3 infiziert und die IL-12 Expression nach verschiedenen Zeiten im Zellkulturüberstand mittels IL-12 p70-ELISA quantifiziert.

Übersicht: Fig. 2.

### 2.2 4-1BBL

Ratten Hepatomazellen, McA-RH7777, wurden mit den Viren (Viruspartikeln) Ad-1, Ad-2, und Ad-3 infiziert und die IL-12 Expression nach verschiedenen Zeiten mittels Durchflußzytometrie nach Anfärbung des 4-1BBL mit dem Antikörper TKS-1 (BD Pharmingen, Heidelberg) nachgewiesen. Ad-1 diente hier als Kontrolle, da keine 4-1BBL Expression erwartet werden kann.

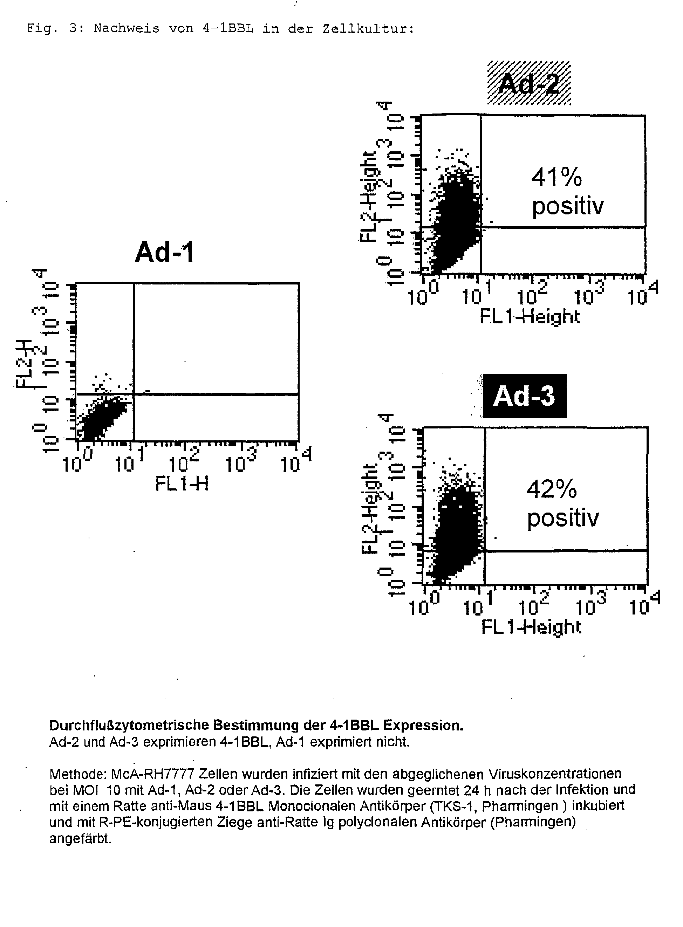

Übersicht: Fig. 3.

### 2.3 IL-2

Ratten Hepatomazellen, McA-RH7777, wurden mit den Viren (Viruspartikeln) Ad-1, Ad-2, und Ad-3 infiziert und die IL-12 Expression nach verschiedenen Zeiten im Zellkulturüberstand mittels IL-2 ELISA quantifiziert. Ad-1 und Ad-2 dienten hier als Kontrollen, da keine IL-2 Expression erwartet werden kann (nicht gezeigt).

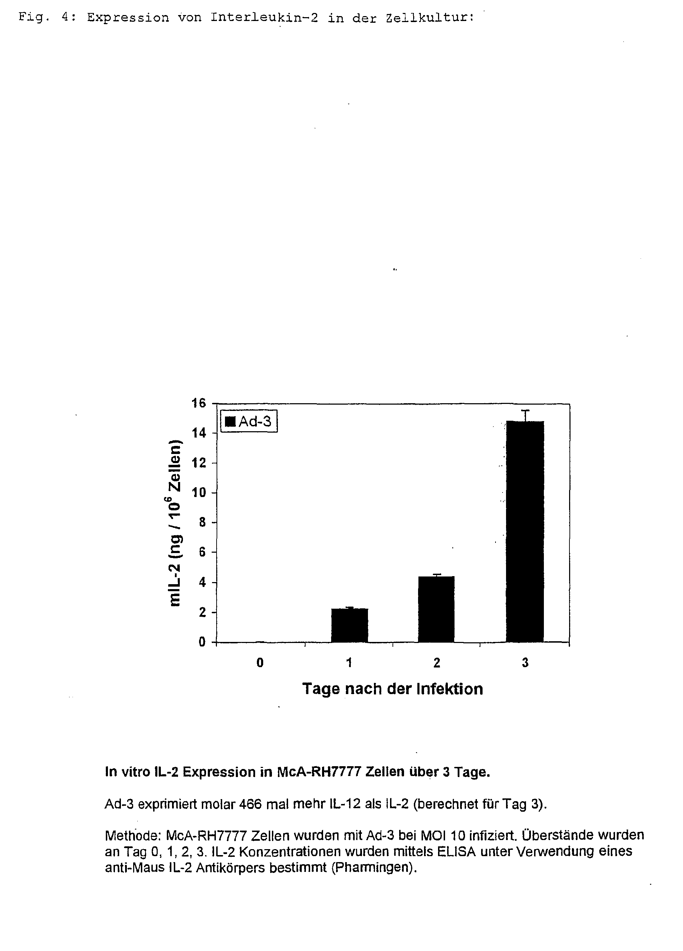

Übersicht: Fig. 4.

### 3. Testung in vivo

### 3.1 Dosiseskalation 1

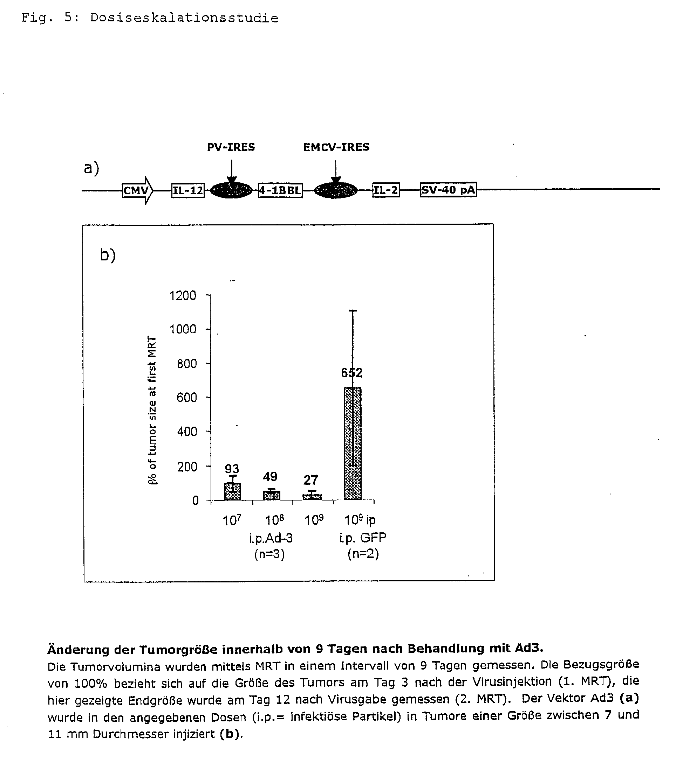

Der Vektor Ad-3 (Fig. 5 a) wurde in unserem Rattenmodell für das hepatozelluläre Carcinom (HCC) getestet. Die Zellinie McA-RH7777 (hepatozelluläres Carcinom der Ratte), die mit der Buffalo-Ratte syngen ist, wird zur subcapsulären hepatischen Transplantation von Tumoren verwendet.

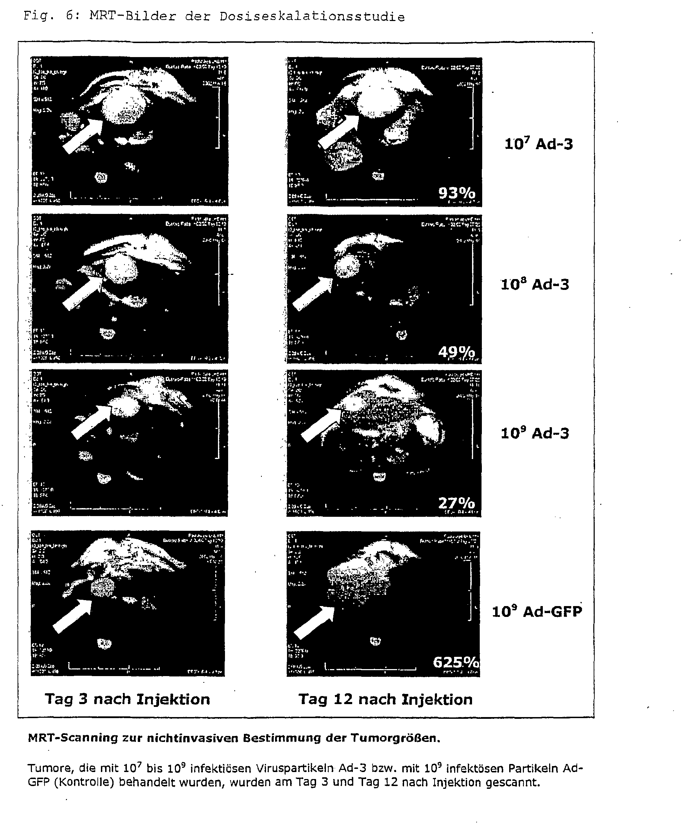

Das Tumorwachstum nach Implantation von 1 Millionen Zellen wird mittels Magnetresonanztomographie (MRT) in Zusammenarbeit mit Herrn Prof. Dr. Gerrit Krupski-Berdien aus der radiologischen Klinik des UKE vor und nach Injektion der Viruspartikel überwacht. Fig. 5b zeigt den Verlauf der Tumorvolumina zwischen dem 3. und 12. Tag nach Virusinjektion. Der in diesem kurzen Zeitraum sehr deutliche Effekt des Vektors ist dosisabhängig und erreicht in der höchsten Dosis eine Reduktion der Tumorvolumina auf 27% der Größe bei Virusinjektion. Dieses Ergebnis ist in Fig. 6 mit ausgewählten MRT-Bildern einzelner Tiere dokumentiert, um die Tumorgröße im Verhältnis zur Größe des Tieres besser zu illustrieren.

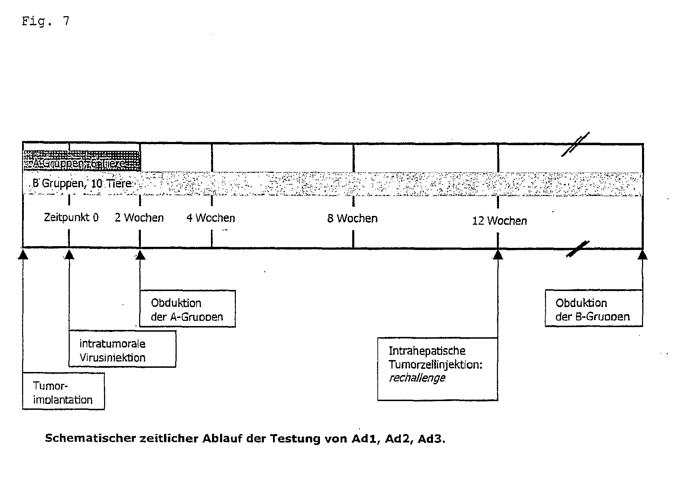

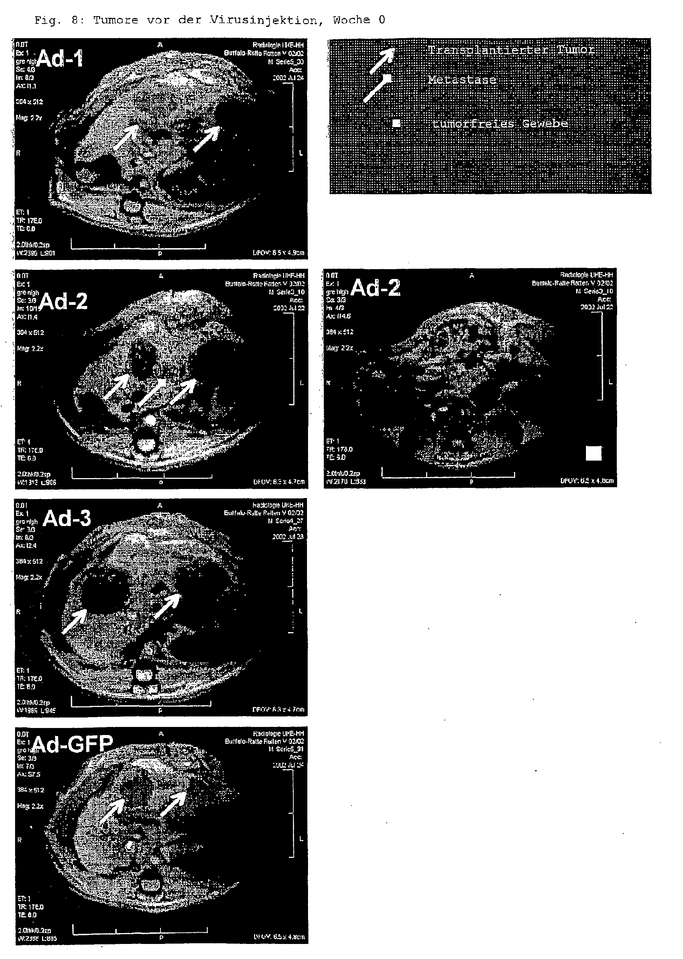

Aufgrund dieser Daten wurde die Dosis für die Langzeitbehandlung größerer Tiergruppen mit auf 5 x 10⁷ infektiöse Partikel pro Tumor festgelegt. In folgenden Studie wurde nun der Effekt der einzelnen Vektoren (Ad-1 bis Ad-3) bei dieser geringen Dosis ermittelt. Wie in dem Schema in Fig. 7 gezeigt, wurden Tumoren implantiert, nach 14 Tagen dann 6 Tiere in den A-Gruppen mit dem Vektor behandelt und die Parameter der antitumoralen Immunantwort nach weiteren 2 Wochen analysiert. Je 10 Tiere bilden die B-Gruppen (Langzeitpruppe). In Abweichung zur Dosiseskalationsstudie (Fig. 5 u. 6) wurden nun zwei Tumoren gesetzt, von denen nur einer mit dem Virus behandelt wurde, um die distalen Effekte der Immunstimulation zu ermitteln. Die B-Gruppen dienten der Analyse der Langzeitkinetik der Tumorreduktion und der Überlebensrate. Nach drei Monaten folgt in diesem Schema bei den überlebenden Tieren eine weitere intrahepatische Tumorimplantation zur Testung des immunologischen Gedächtnisses in Bezug auf die Erkennung und Bekämpfung von Tumorrezidiven. Für weitere drei Monate wird erneut die Überlebensrate bestimmt. Unsere Daten zeigen auch bei der gewählten Dosis von 5 x 10⁷ eine Eindämmung des Tumorwachstums im ersten 14-Tage-Intervall. Die B-Gruppen wurden bereits für 100 Tage beobachtet. Die Ergebnisse der Tumorreduktion durch unsere Behandlung sind in Form der MRT-Aufnahmen in den Fig. 8 bis 10 illustriert.

Die Figuren dokumentieren, dass bei der gewählten Dosis innerhalb von 7 Wochen nach Virusgabe eine komplette Ausräumung der hepatischen injizierten aber auch der nichtinjizierten Tumoren erreicht wurde. Eindrucksvoll ist der weitere Effekt (Siehe Ad-2 Reihe in Fig. 8-10), dass Metastasen in der Leber und im Bauchraum (Ad-2, Woche 3) ebenfalls komplett ausgeräumt wurden.

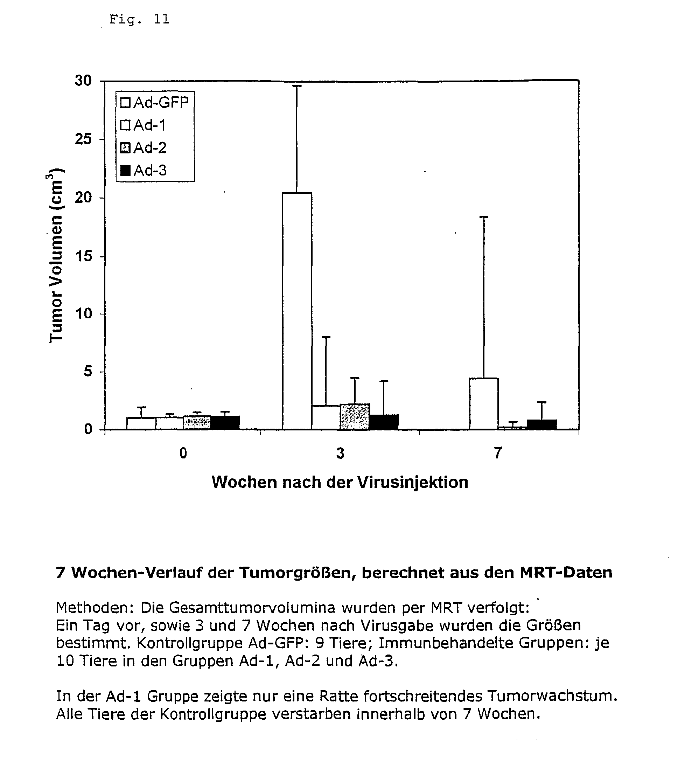

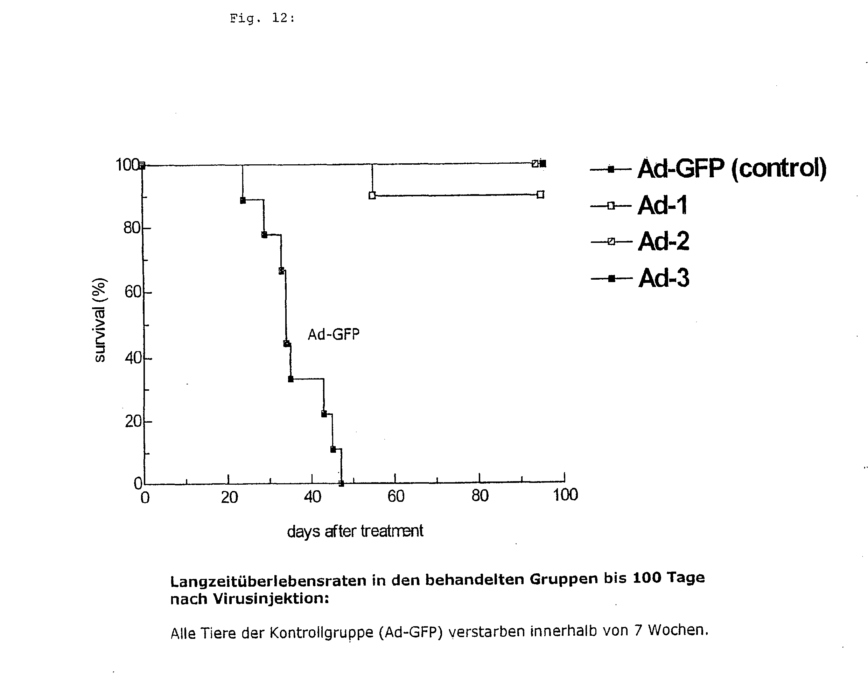

Die Entwicklung der aus den MRT-Daten bestimmten Tumorvolumina ist in Fig. 11 dargestellt und erläutert.
Der Verlauf der Studie ausgedrückt als die Überlebensrate in % innerhalb der behandelten Gruppen ist in Fig. 12 illustriert.
Im Rahmen der Tierexperimente wurde IL-12 im Serum der Ratten nachgewiesen. Ebenfalls wurde Interferon-gamma bestimmt, das nach IL-12-Stimulation von Immunzellen ausgeschüttet wird und einen Großteil der antitumoralen Effekte verantwortet. Interferon-gamma war ebenfalls deutlich nachweisbar. Zusätzlich zu diesen Bestimmungen wurden spezifisch gegen den Tumor gerichtete T-Zellen in einem so genannten Zytotoxizitätstest nachgewiesen. Die Immunzellantwort wurde gegenwärtig auch an Gewebepräparaten der behandelten Tiere charakterisiert. In behandeltem Tumorgewebe konnten CD8+ Zellen, CD4+ Zellen, Makrophagen und natürliche Killerzellen in erhöhter Menge im Vergleich zu mit Kontrolvektor behandelten Gewebe nachgewiesen werden.

### 3.2 Protokoll zur Durchführung der Langzeitstudie:

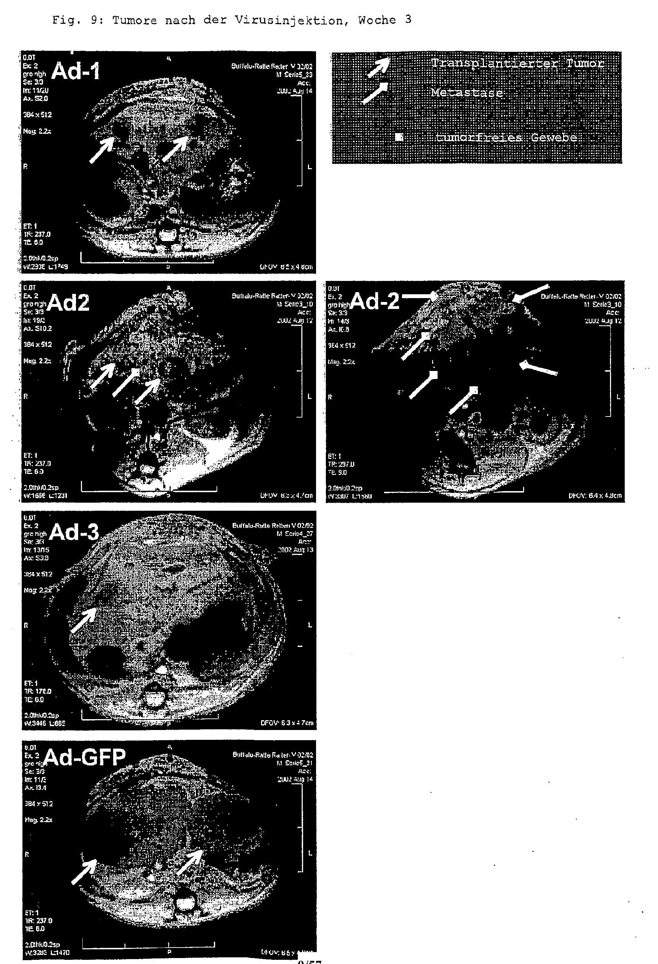

Es wurden zwei Tumoren durch Injektion von McA-RH7777-Zellen gesetzt. Die Zellen wurden in zwei verschiedene Leberlappen injiziert. Für einen Tumor wurden 1 Million Zellen verwendet. In diesem linksseitigen Tumor wurde später die Virusinjektion vorgenommen. Ein weiterer Tumor wurde rechtsseitig mit 650.000 Zellen gesetzt. Dieser Tumor diente uns als intrahepatisches Metastasenmodell. An diesem Tumor wurde die Wirksamkeit der stimulierten Immunzellen an einem distalen Tumorherd überprüft. Die Ergebnisse werden in den Figuren 9 bis 12 und 28 bis 30 gezeigt.

### II. Konstruktion von Ad-4:

Mit einem anderen Insertionssystem als der homologen Rekombination in E. coli-Zellen wird eine Expressionskassette für das B7-1 in die Position der adenoviralen E3-Region eingefügt. Diese Region ist funktionell inaktiv, da große Anteile dieser Region in den hier benutzten Vektoren deletiert sind. Die Expressionskassette verfügt über einen eigenen Promoter der humanen Phosphoglyceratkinase bzw. einem Promoter ähnlicher Stärke.

Die Herstellung der Viren wird auf äquivalente Weise wie in dem vorbeschriebenen Protokoll durchgeführt.

### Kurze Beschreibung der Figuren

- Fig.1: Übersichtsdarstellung der Vektoren Ad-1 bis Ad-3.
- Fig. 2: Bestimmung der Interleukinmenge im Zellkulturüber-stand von McA-RH7777 Zellen nach Transfektion mit Ad-1, Ad-2 und Ad-3. Vektormengen, die für die Tierexperimente eingesetzt werden sollten, wurden bezogen auf identische Interleukin-12-Expression hin eingestellt. Fig. 2 zeigt den Zeitverlauf der Expression über 3 Tage in den Ratten Hepatomazellen McA-RH7777.
Verfahren: McA-RH7777 Zellen wurden bei MOIs von 10 mit Ad-1, Ad-2 oder Ad-3 infiziert. Die Überstände wurden an den Tagen 0, 1, 2, und 3 nach Infektion gesammelt. scIL-12 Konzentrationen wurden bestimmt mittels ELISA mit einem anti-Maus IL-12p70 Antikörper (Pharmingen).
- Fig. 3: Nachweis von 4-1BBL in den McA-RH7777 Zellkulturen. Durchflußzytometrische Bestimmung der 4-1BBL Expression. Ad-2 und Ad-3 exprimieren 4-1BBL, Ad-1 exprimiert nicht.
Verfahren: McA-RH7777 Zellen wurden infiziert mit den abgeglichenen Viruskonzentrationen bei MOI 10 mit Ad-1, Ad-2 oder Ad-3. Die Zellen wurden geerntet 24 h nach der Infektion und mit einem Ratte anti-Maus 4-1BBL Monoclonalen Antikörper (TKS-1, Pharmingen ) inkubiert und zum Nachweis mit R-PE-konjugierten Ziege anti-Ratte Ig polyclonalen Antikörper (Pharmingen)angefärbt.
- Fig. 4: Expression von IL-2 in den McA-RH7777 Zellkulturen über 3 Tage. Ad-3 exprimiert molar 466 mal mehr IL-12 als IL-2 (berechnet für Tag 3).
Verfahren: McA-RH7777 Zellen wurden mit Ad-3 bei MOI 10 infiziert. Überstände wurden an Tag 0, 1, 2, 3. IL-2 Konzentrationen wurden mittels ELISA unter Verwendung eines anti-Maus IL-2 Antikörpers bestimmt (Pharmingen).
- Fig. 5: Dosiseskalationsstudie. Änderung der Tumorgröße innerhalb von 9 Tagen nach Behandlung mit Ad3.
Verfahren: Die Tumorvolumina wurden mittels MRT in einem Intervall von 9 Tagen gemessen. Die Bezugsgröße von 100% bezieht sich auf die Größe des Tumors am Tag 3 nach der Virusinjektion (1. MRT), die hier gezeigte Endgröße wurde am Tag 12 nach Virusgabe gemessen (2. MRT). Der Vektor Ad3 (a) wurde in den angegebenen Dosen (i.p.= infektiöse Partikel) in Tumore einer Größe zwischen 7 und 11 mm Durchmesser injiziert (b).
- Fig. 6: MRT Bilder der Dosiseskalationsstudie.
Verfahren: Tumore, die mit 10⁷ bis 10⁹ infektiösen Viruspartikeln Ad-3 bzw. mit 10⁹ infektösen Partikeln Ad-GFP (Kontrolle) behandelt wurden, wurden am Tag 3 und Tag 12 nach Injektion mittels MRT gescannt.
- Fig. 7: Schematische Darstellung des Ablaufes der Tierversuche unter Verwendung von Ad-1, Ad-2 und Ad-3.
- Fig. 8: MRT-Aufnahmen der Tumore vor der Virusinjektion, Woche 0.
- Fig. 9: MRT-Aufnahmen der Tumore nach der Virusinjektion, Woche 3.

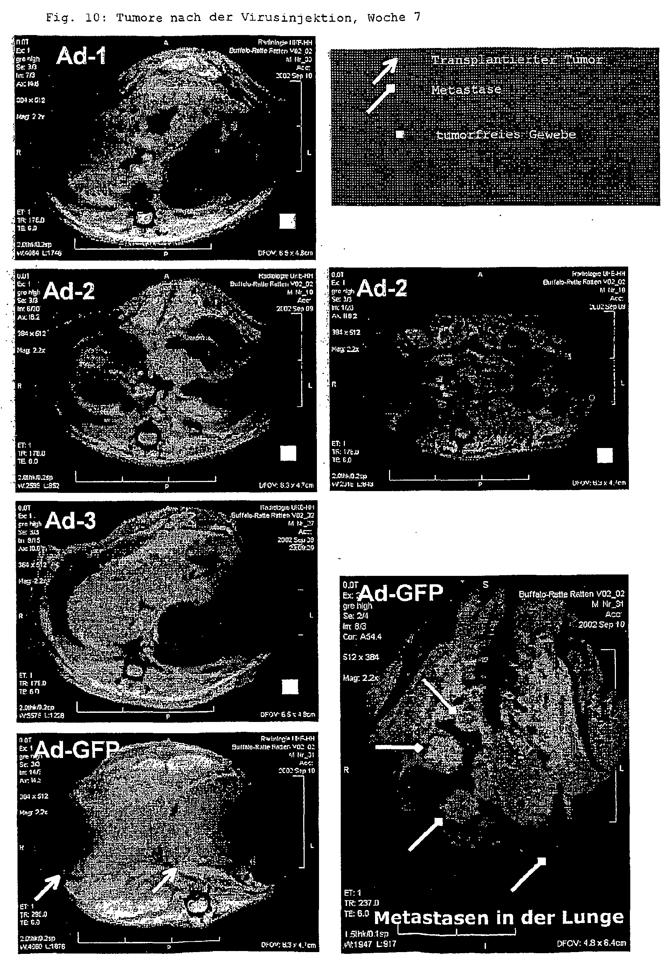

- Fig. 10: MRT-Aufnahmen der Tumore nach der Virusinjektion, Woche 7.
- Fig. 11: Verlaufsdarstellung der Tumorgrößen, berechnet aus den MRT-Daten. Verfahren: Die Gesamttumorvolumina wurden per MRT verfolgt:Ein Tag vor, sowie 3 und 7 Wochen nach Virusgabe wurden die Größen bestimmt. Kontrollgruppe Ad-GFP: 9 Tiere; Immunbehandelte Gruppen: je 10 Tiere in den Gruppen Ad-1, Ad-2 und Ad-3. In der Ad-1 Gruppe zeigte nur eine Ratte fortschreitendes Tumorwachstum. Alle Tiere der Kontrollgruppe verstarben innerhalb von 7 Wochen.
- Fig. 12: Langzeitüberlebensrate der Versuchstiere bis 100 Tage nach Virusinjektion.

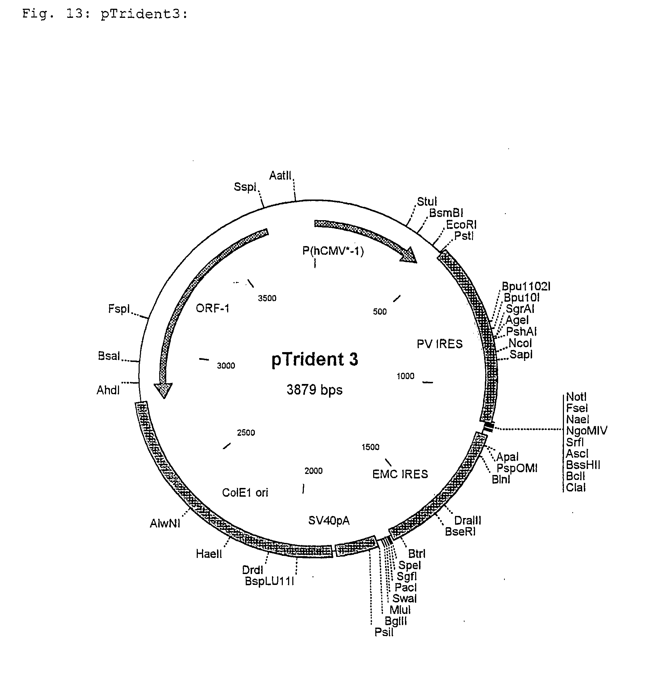

- Fig. 13: Karte des Vektors pTrident3.

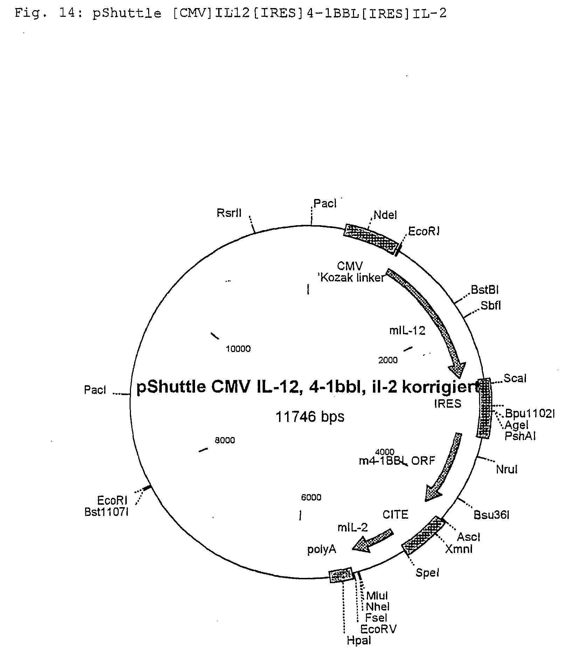

- Fig. 14: Karte des Vektors pShuttle[CMV]IL12[IRES]4-1BBL[IRES]IL-2.

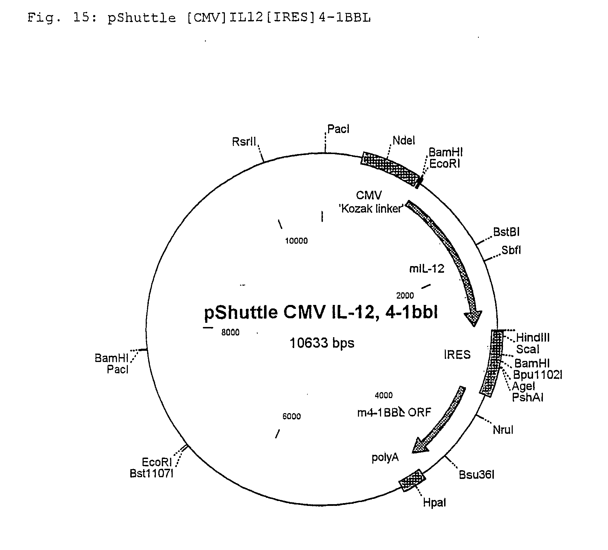

- Fig. 15: Karte des Vektors pShuttle [CMV]IL12[IRES]4-1BBL.

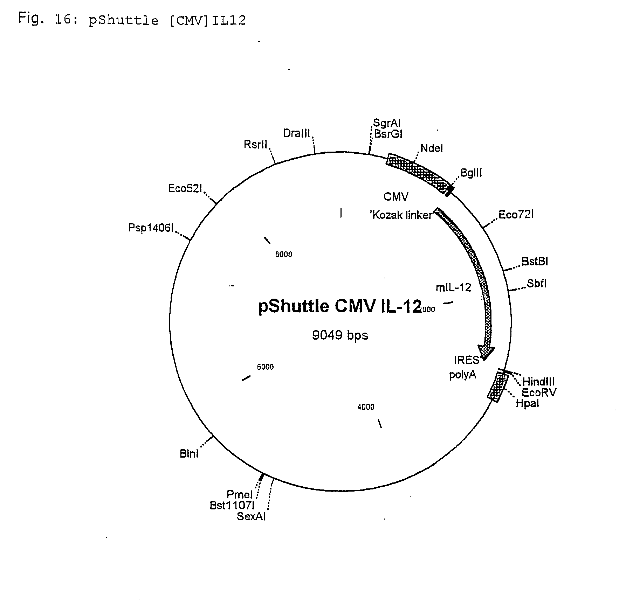

- Fig. 16: Karte des Vektors pShuttle [CMV]IL12.

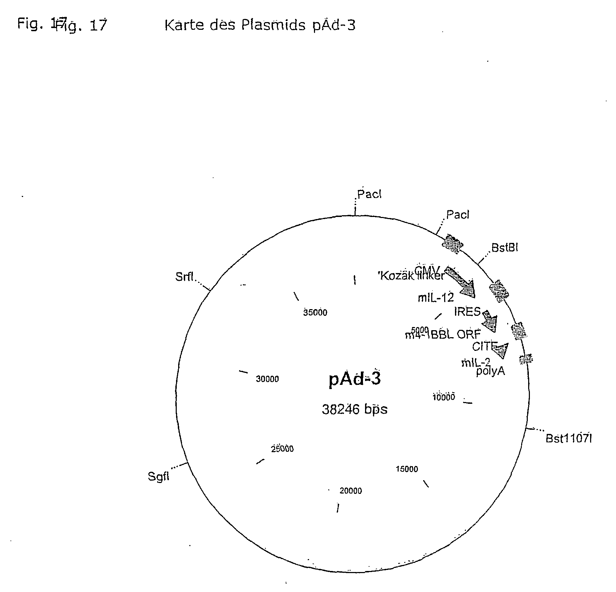

- Fig. 17: Karte des Vektors pAd-3.

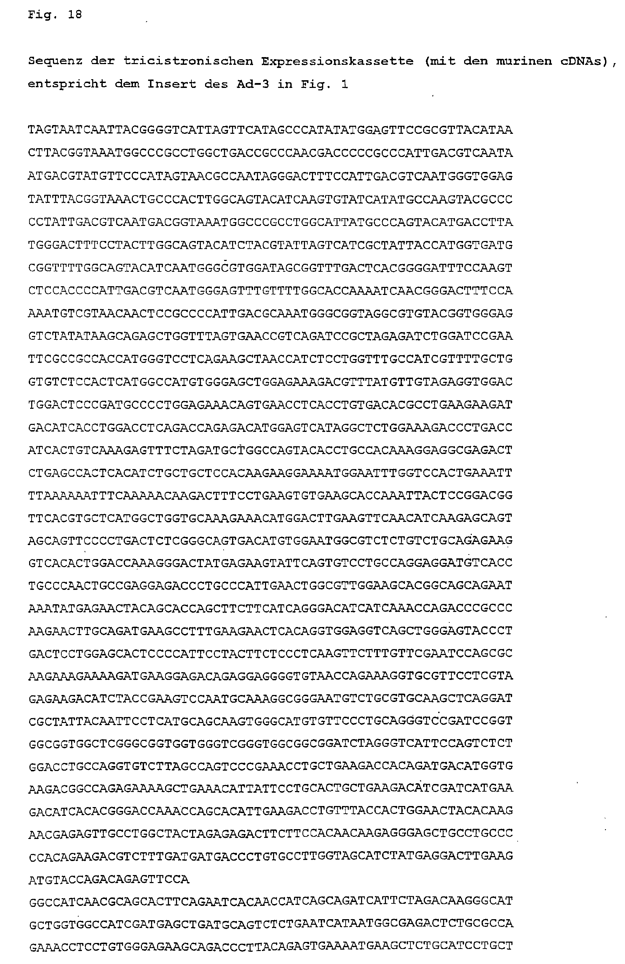

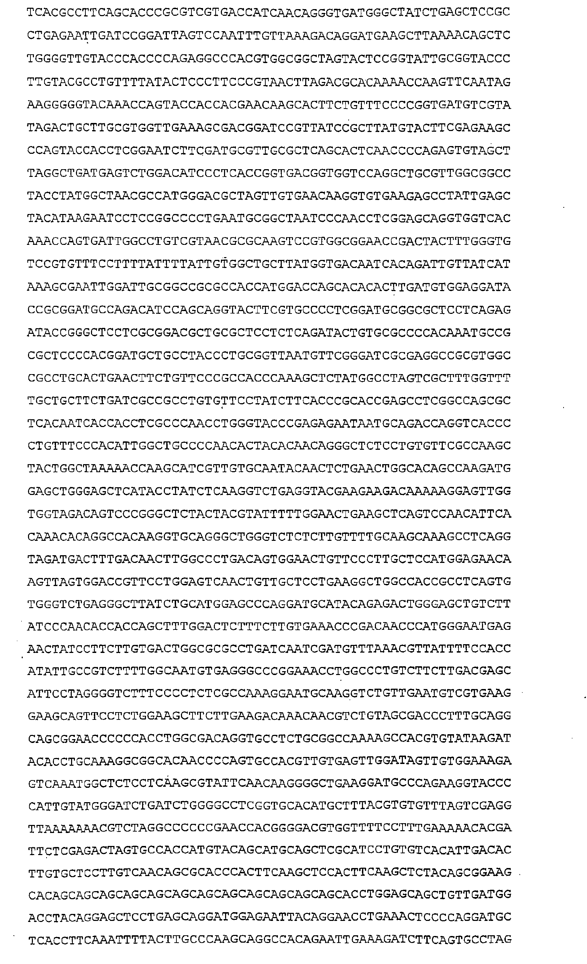

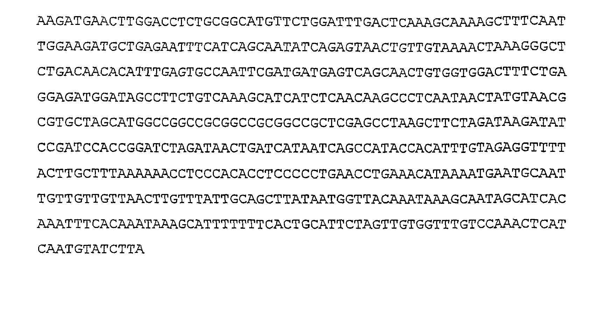

- Fig. 18: Sequenz der tricistronischen Expressionskassette, welche die murinen cDNAs enthält, entspricht dem Insert Ad-3 aus Fig. 1.
- Fig. 19: Kodierende Sequenz des humanen IL-12 40 kDa.
- Fig. 20: Kodierende Sequenz des humanen IL-12 35 kDa.
- Fig. 21: Kodierende Sequenz des humanen 4-1BBL.
- Fig. 22: Kodierende Sequenz des humanen IL-2.
- Fig. 23: Kodierende Sequenz des humanen B7-1 und B7-2.

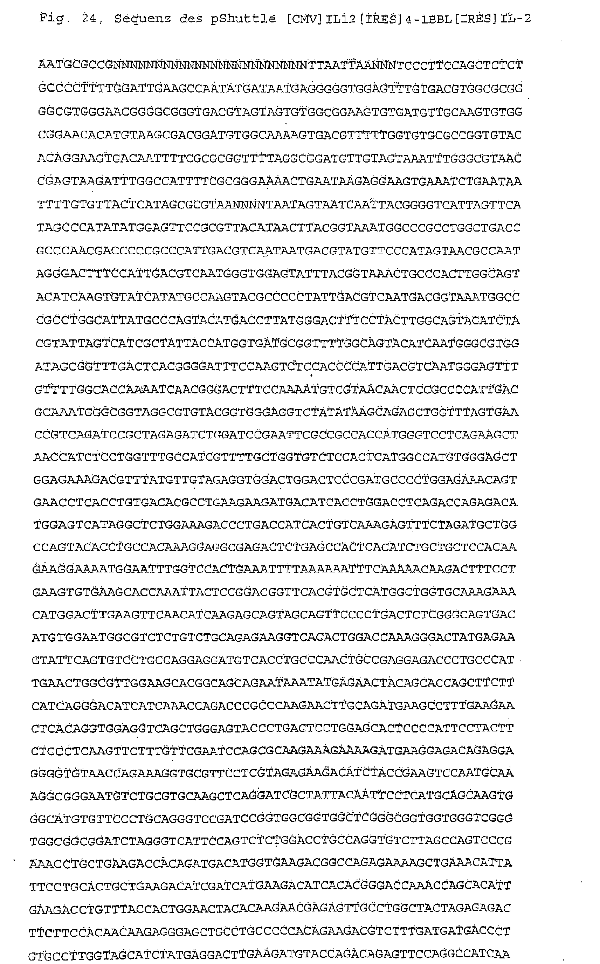

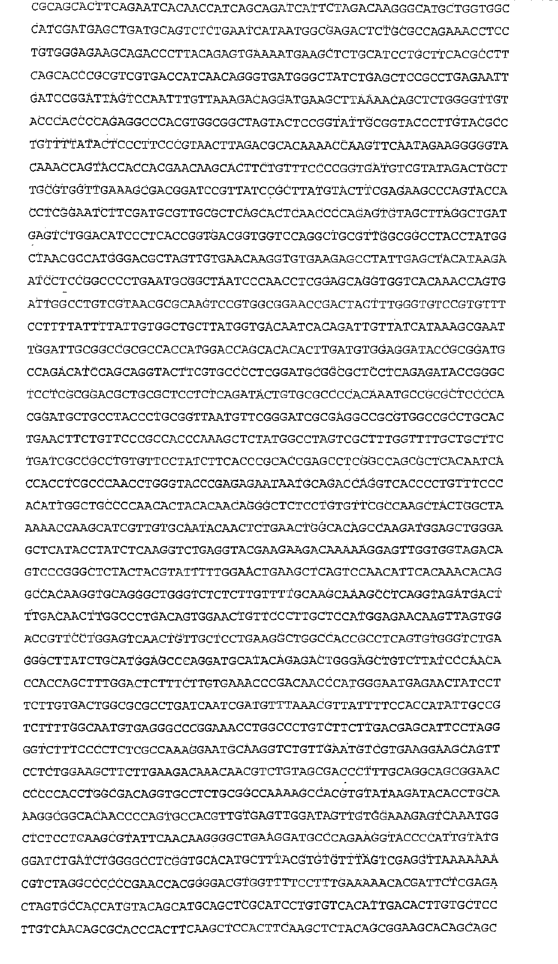

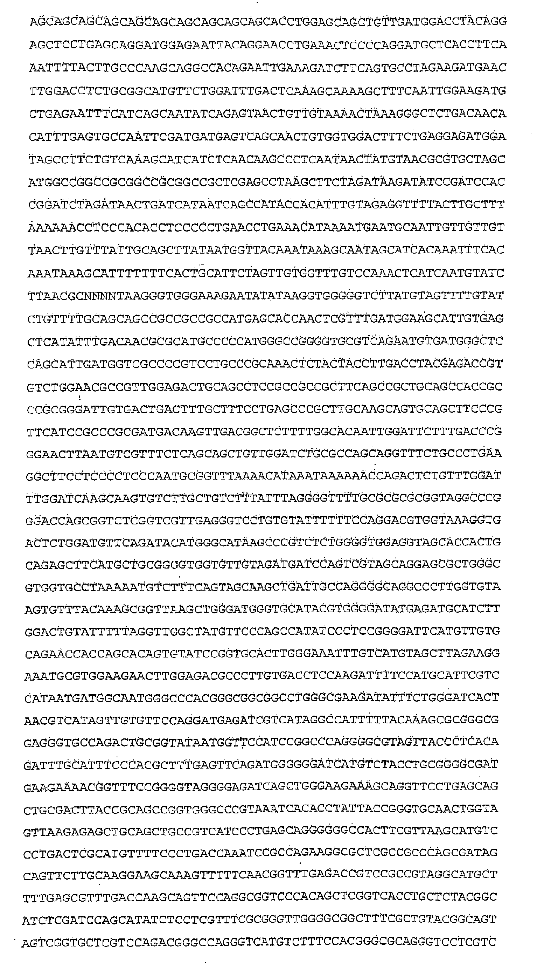

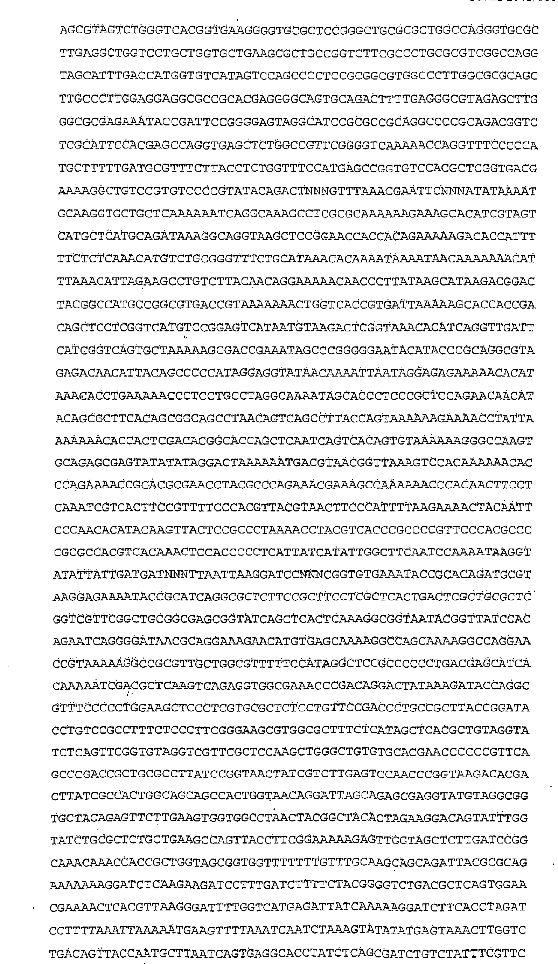

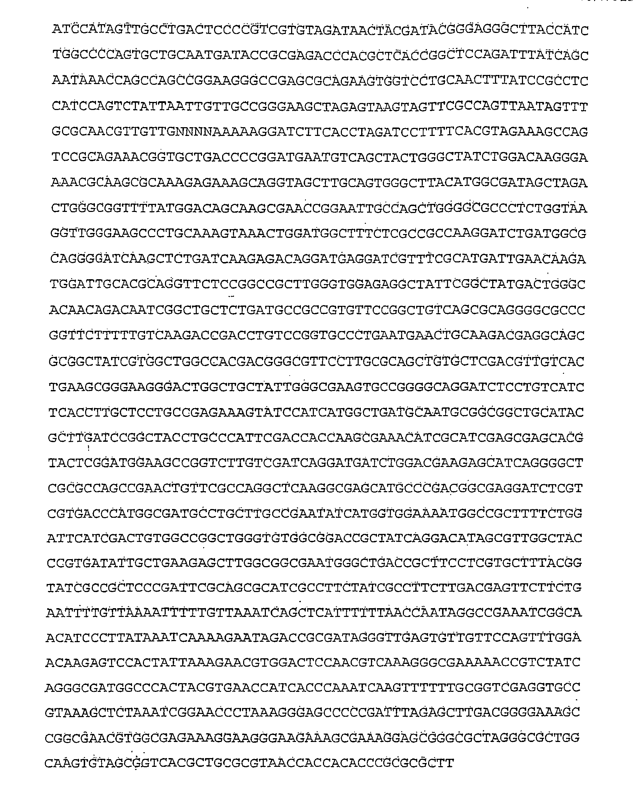

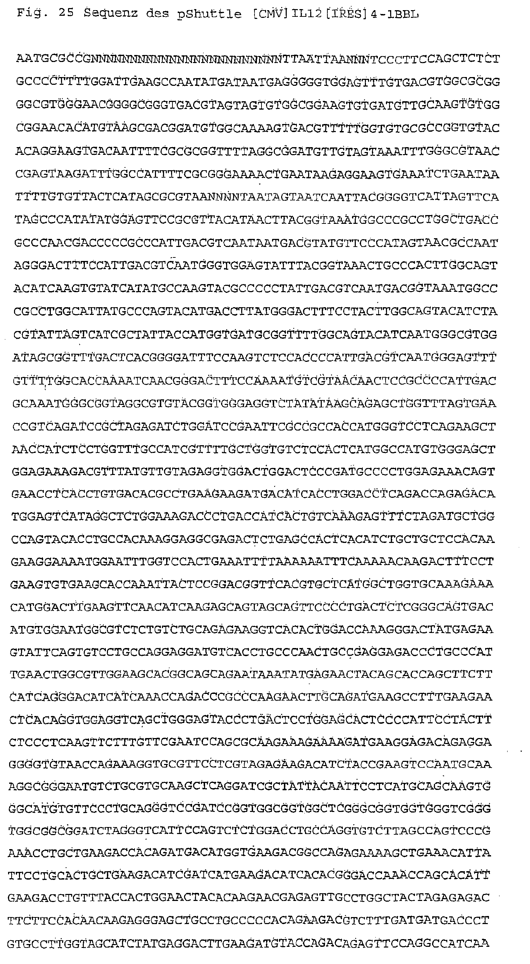

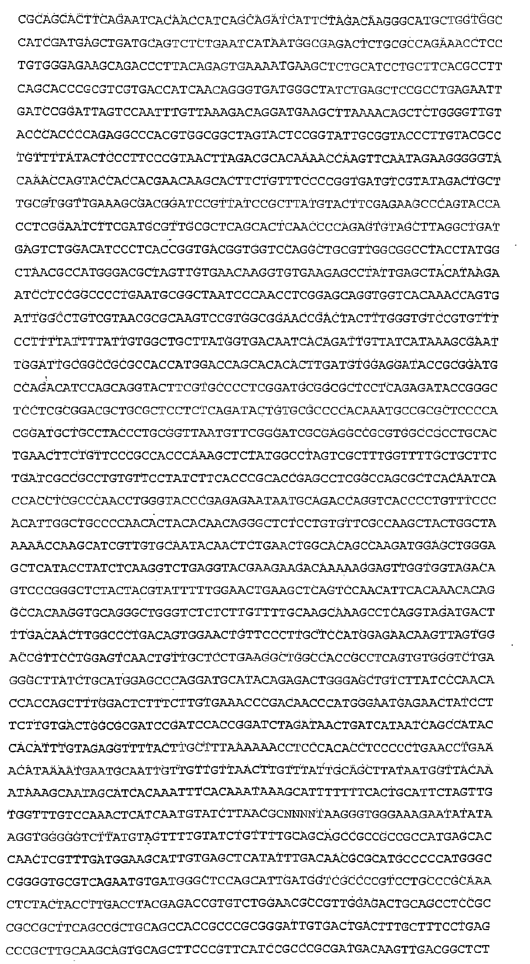

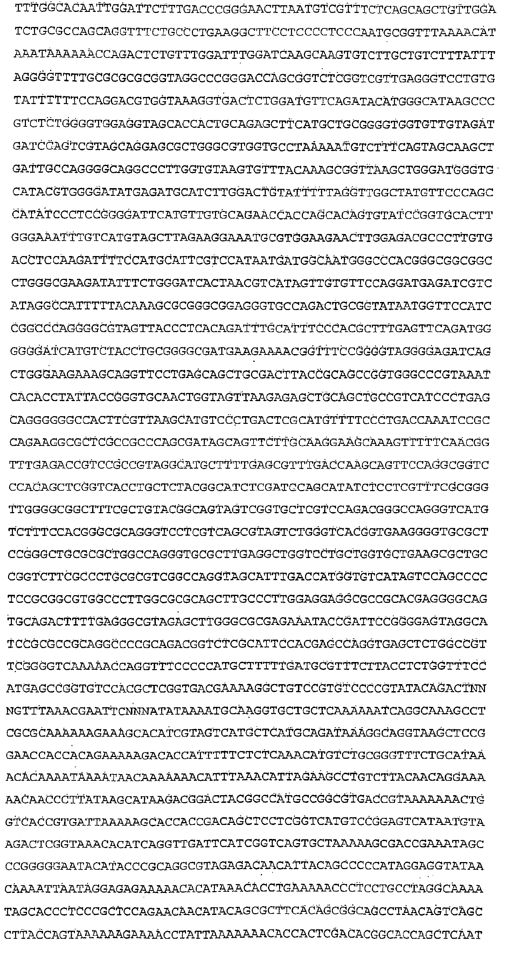

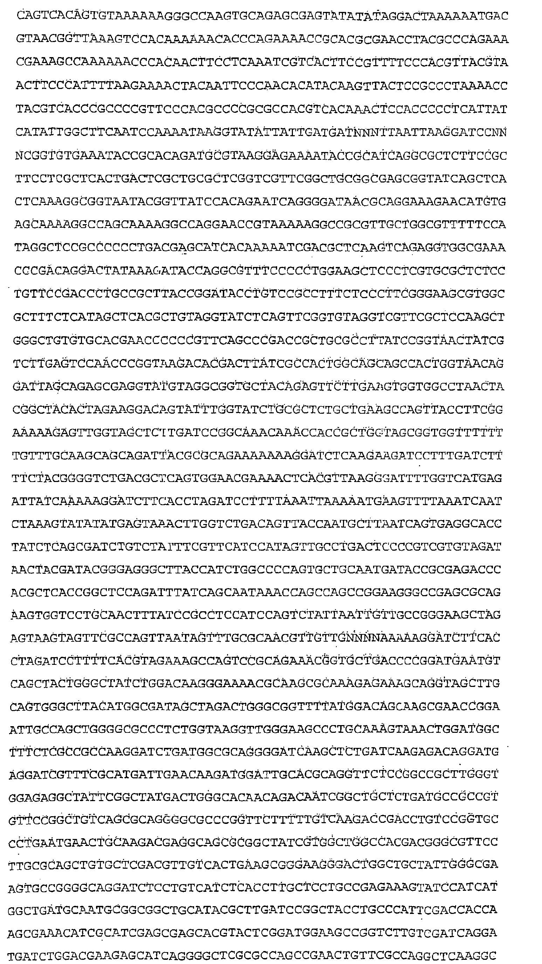

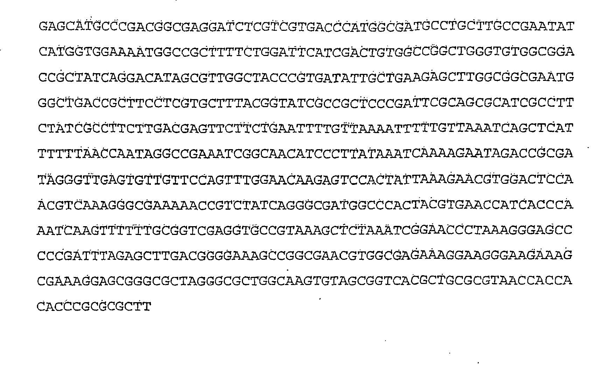

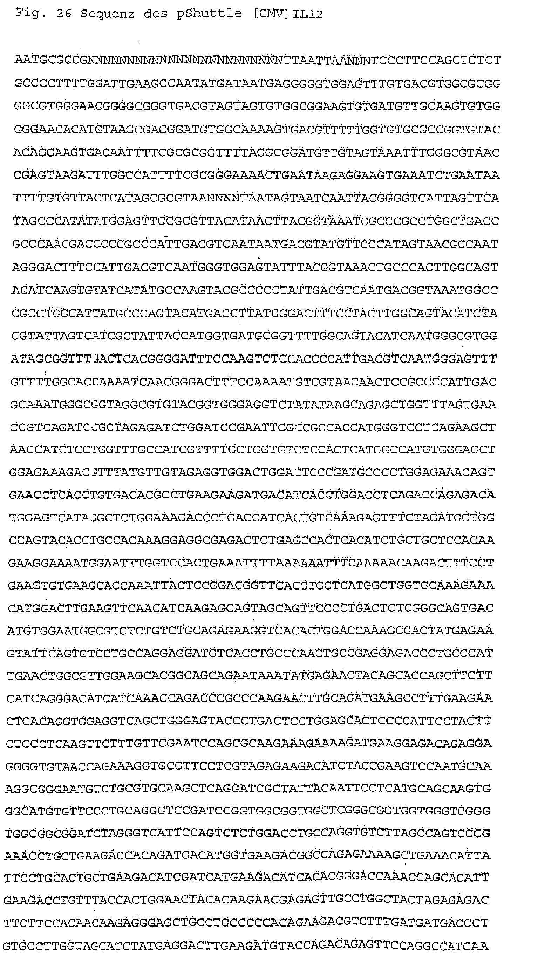

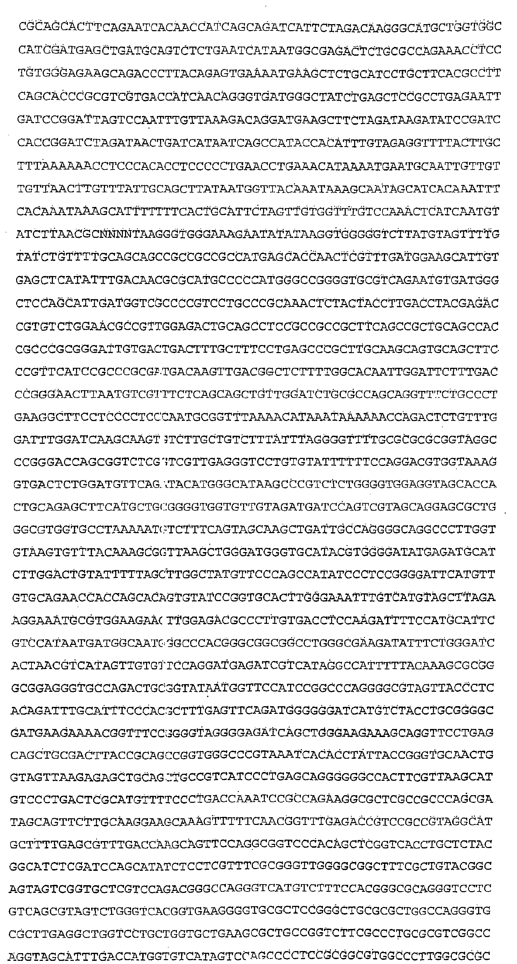

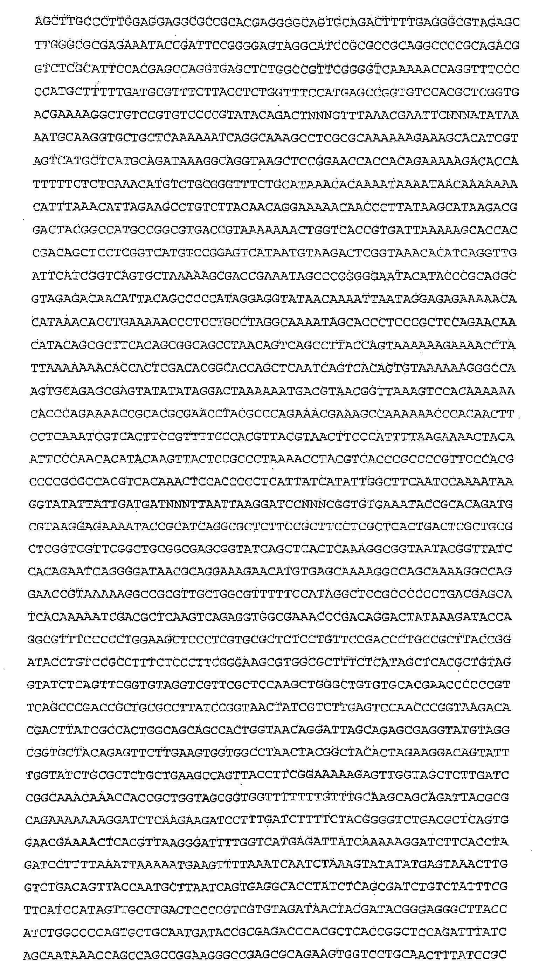

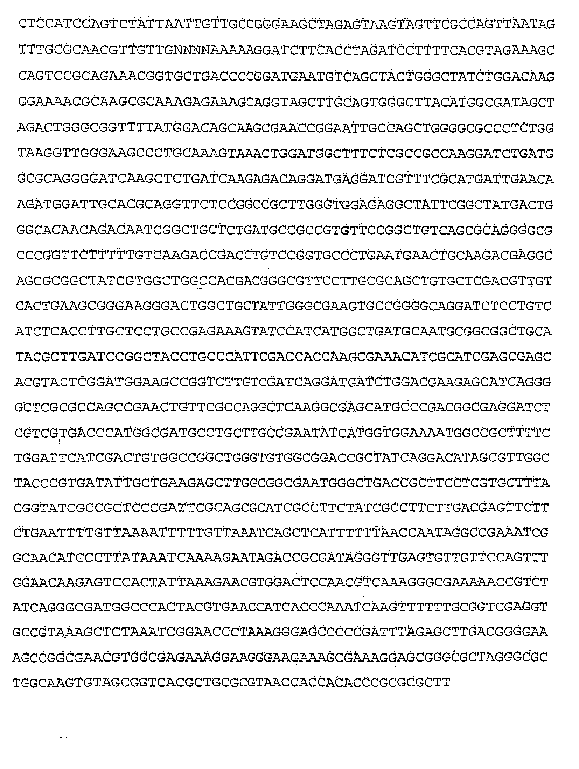

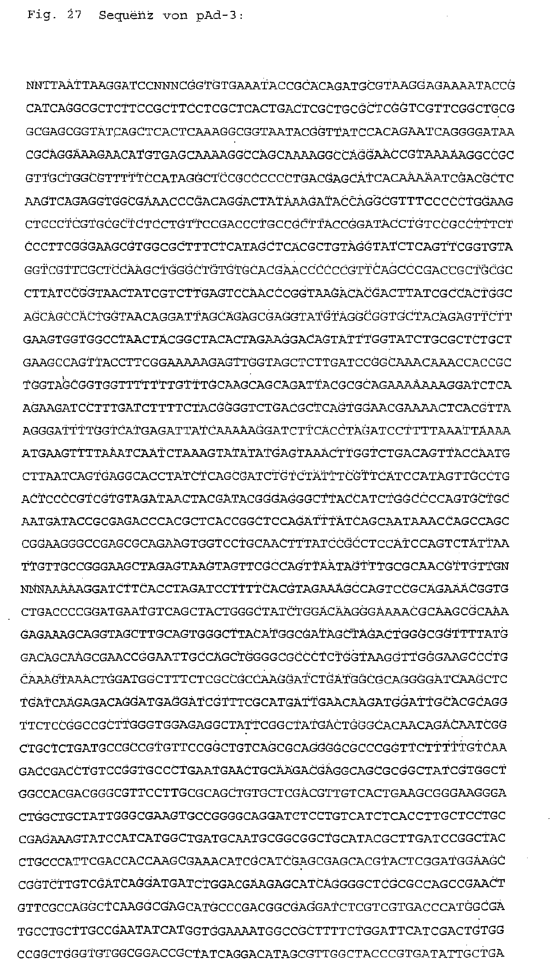

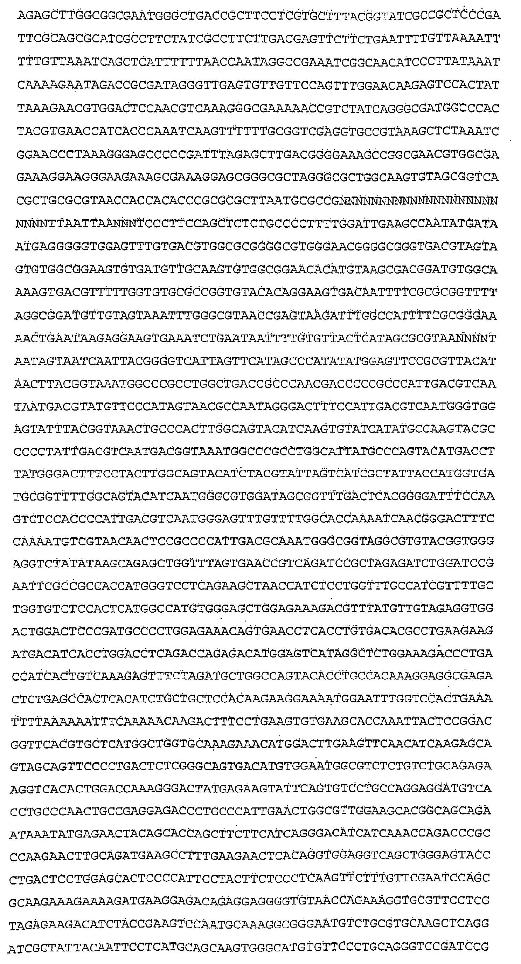

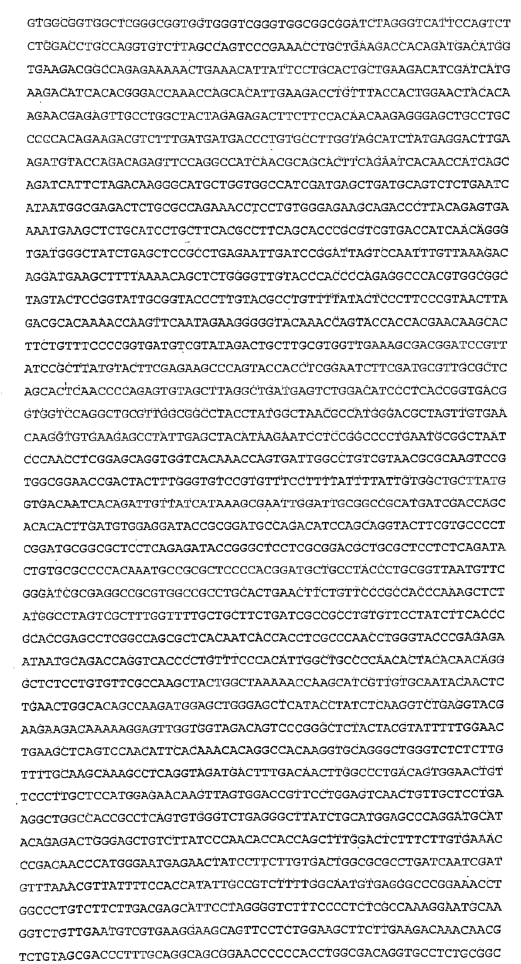

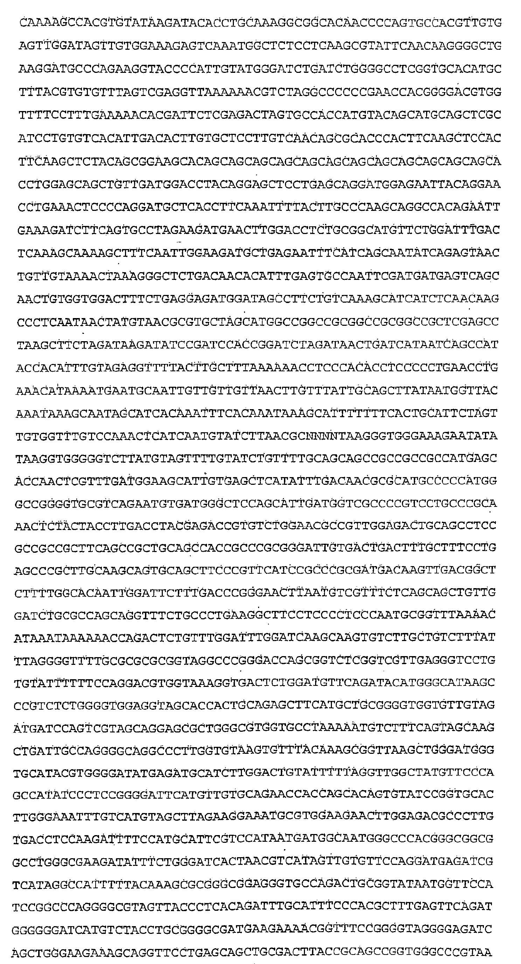

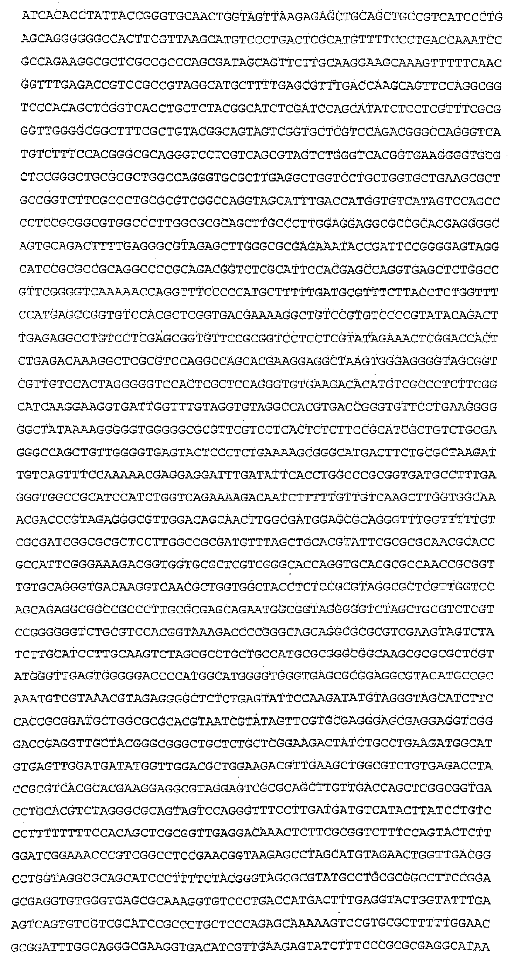

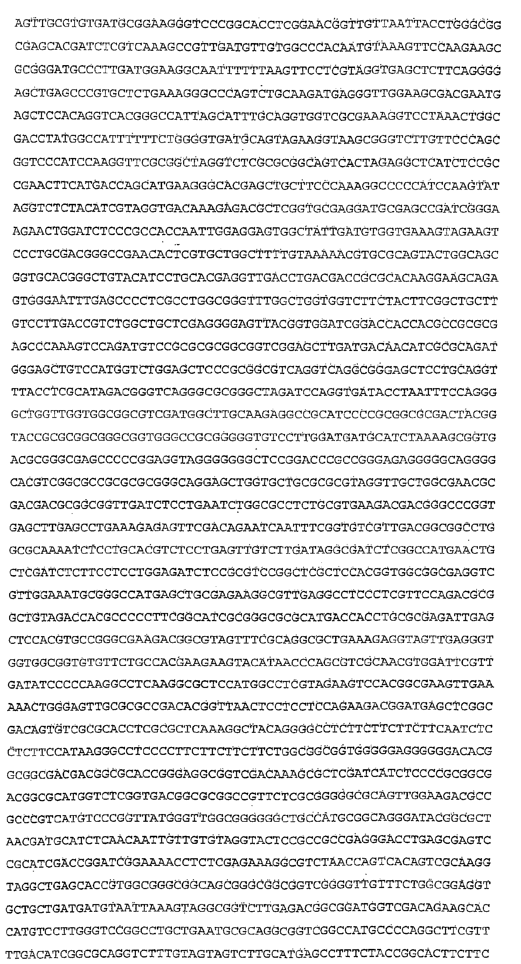

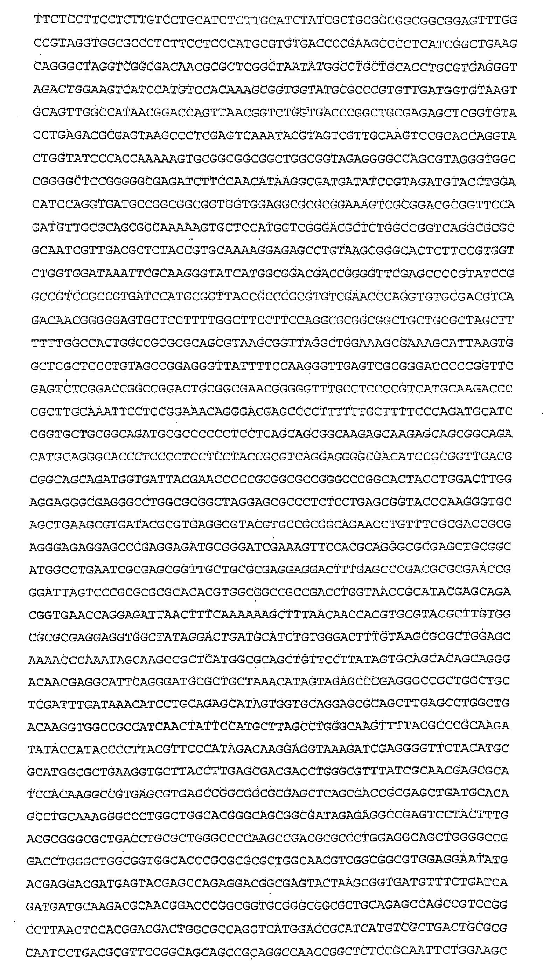

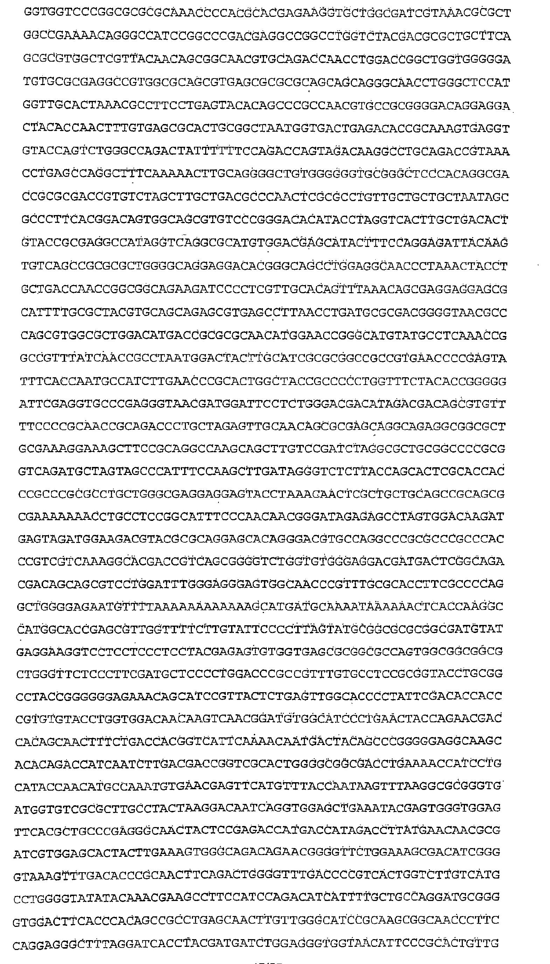

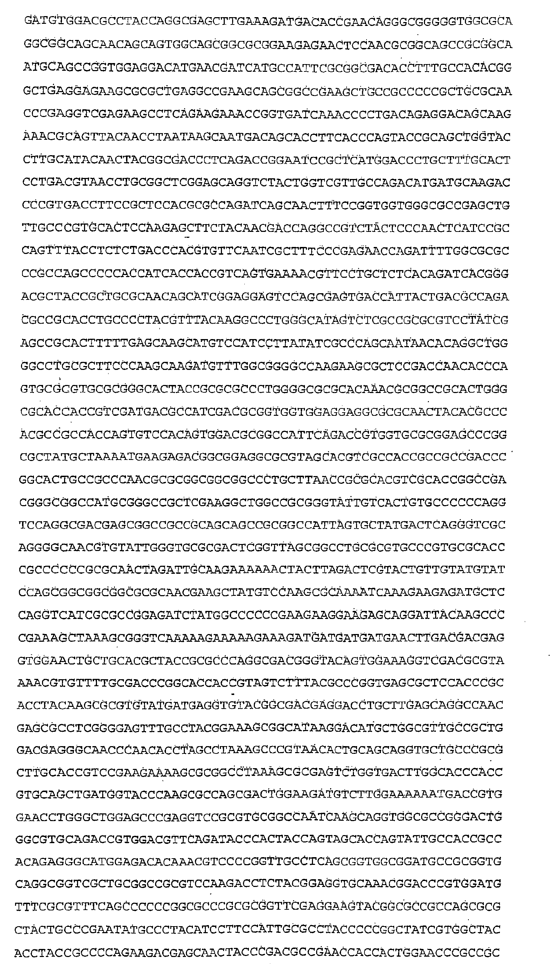

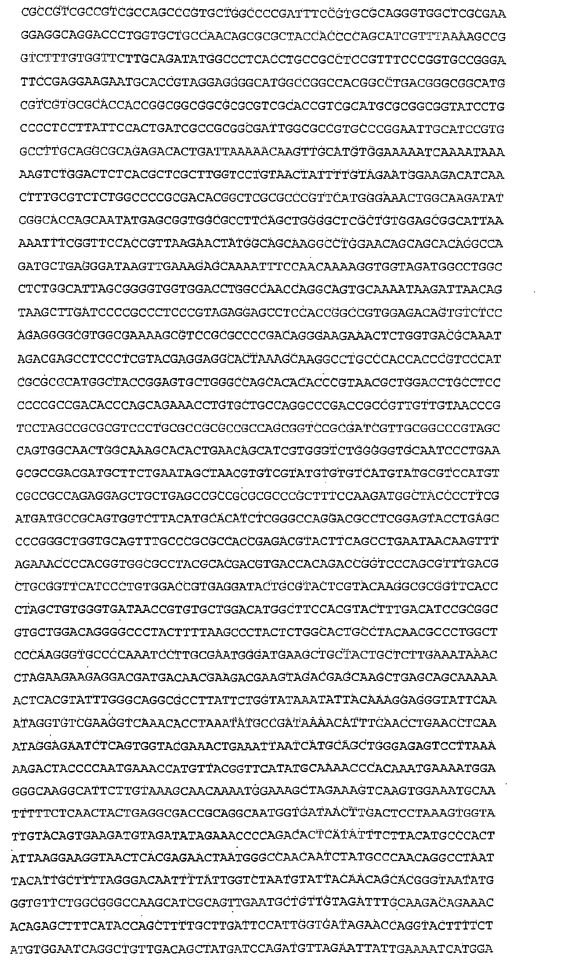

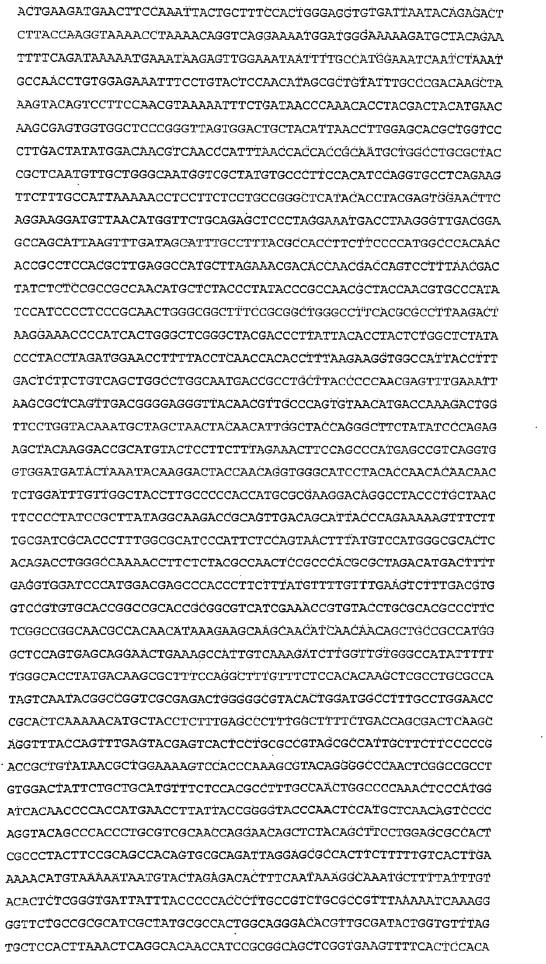

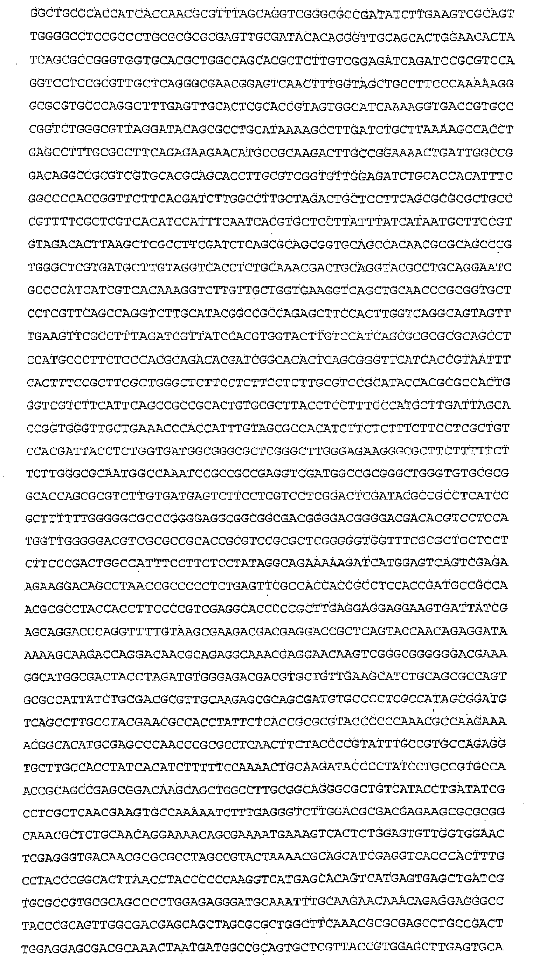

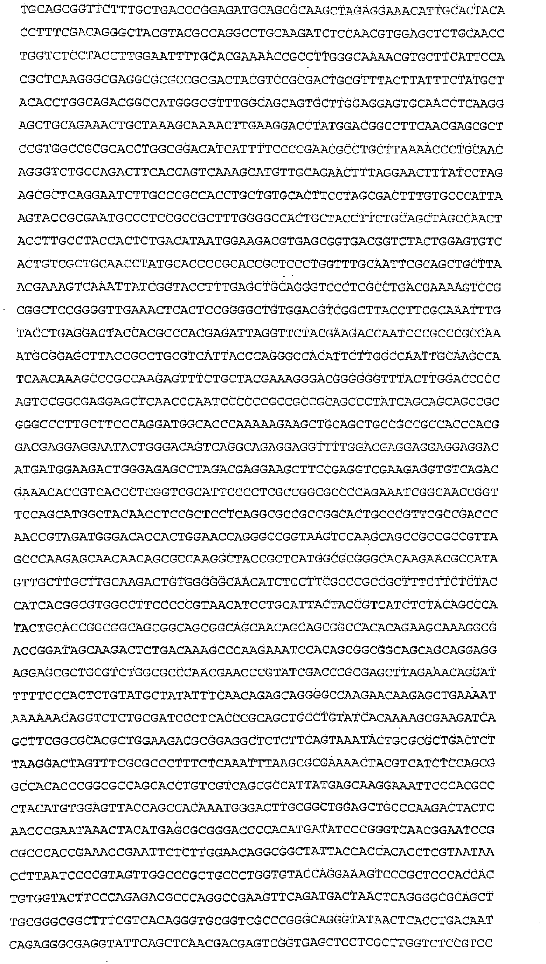

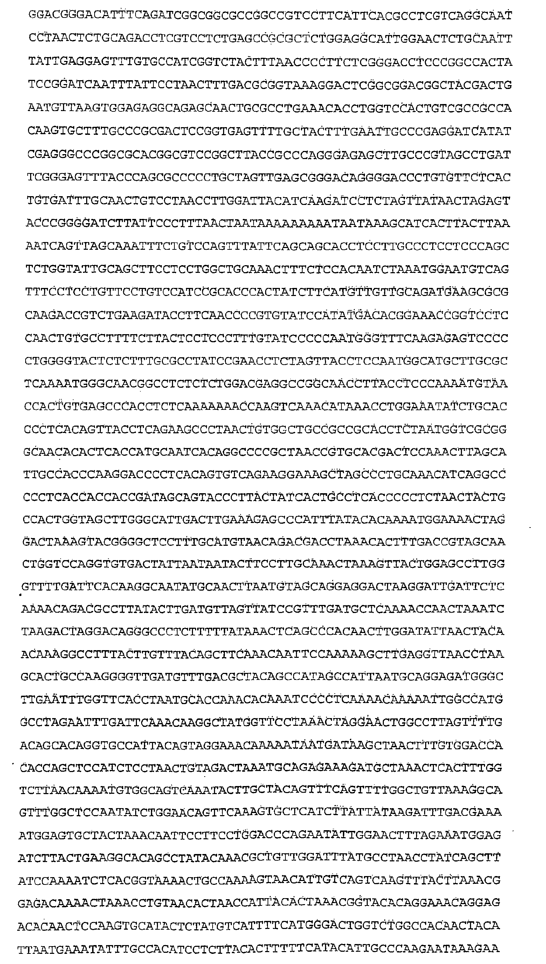

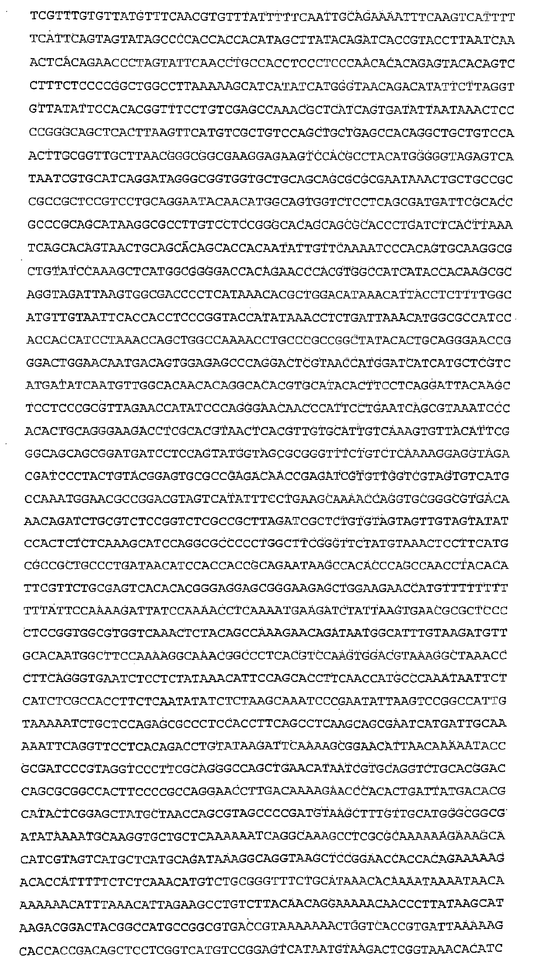

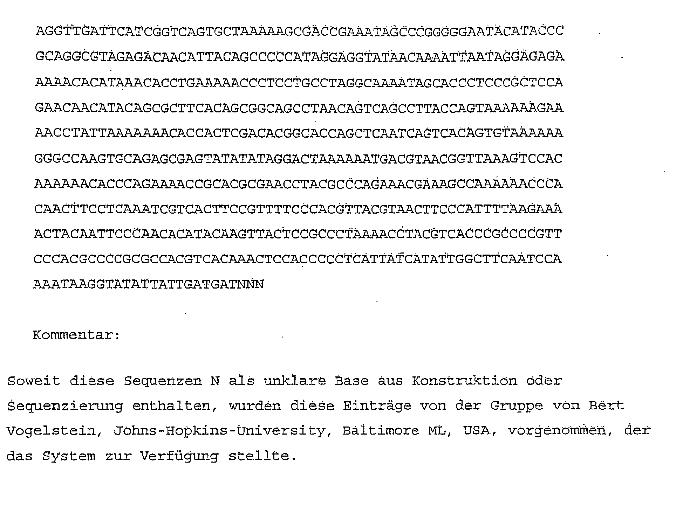

- Fig. 24 bis 27: Sequenz verschiedener Vektoren

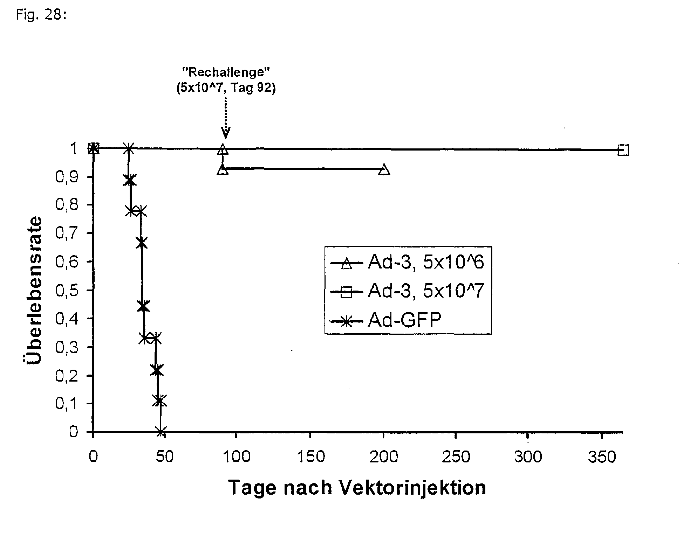

- Fig. 28: Darstellung der Wirksamkeit in einem Beobachtungszeitraum bis zu einem Jahr. Die Grafik zeigt den Anteil der lebenden Ratten zu jedem Zeitpunkt (Überlebensrate 1 = 100% der Tiere einer Gruppe). TherapieGruppen: Ad-3, 5x10⁶ (n=12), Ad-3, 5x10⁷ (n=10). Kontrollgruppe: 5 x 10⁸ Ad-GFP (n=9).
Bei dieser Langzeitstudie wurden zwei Lebertumore in jedem Tier gesetzt. 2 Wochen später (am Tag 0 in der Abbildung) wurde einer der beiden Tumore einmalig durch eine Vektorinjektion behandelt. Das Tumorvolumen lag bei ca. 1 ml zu diesem Zeitpunkt. Die Figur zeigt, daß die Kontrolltiere (mit Ad-GFP behandelt) innerhalb von 47 Tagen nach Vektorinjektion verstarben. In der mit 5 x 10⁶ i.u. ("infectious units" oder infektiöser Partikel) Ad-3 behandelten Gruppe verstarb ein Tier nach 90 Tagen und in der mit 5 x 10⁷ i.u. Ad-3 behandelten Gruppe überlebten alle Tiere. "Rechallenge": In der mit 5 x 10⁷ i.u. Ad-3 behandelten Gruppe wurden in alle Tiere 92 Tage (13 Wochen) nach Vektorinjektion erneut Tumore in die Leber implantiert. Das Tumorzellimplantat verschwand ohne weitere Behandlung bei allen 10 Tieren.

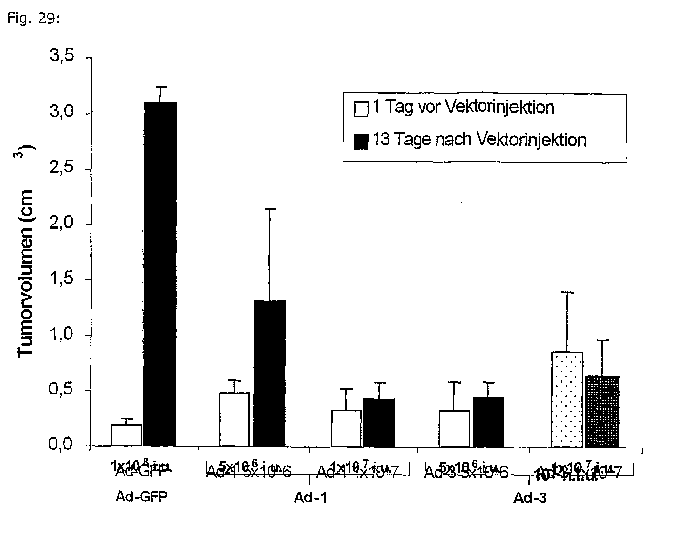

- Fig. 29: Darstellung der Effekte auf das Tumorwachstum nach Behandlung mit Ad-1 und Ad-3 im Vergleich zu einem Kontrollvektor (Ad-GFP).
Hierbei wurden für Ad-1 und Ad-3 zwei verschiedene Dosisstufen appliziert und die Tumorgrößen 2 Wochen nach Behandlung bestimmt. Tierzahl pro Gruppe: n=3. Die Figur zeigt die Änderung der Tumorvolumina bei verschiedenen Vektordosen von Ad-1 und Ad-3. Es wurden 1 x 10⁶ MH-7777A Tumorzellen in den rechten Leberlappen appliziert und zwei Wochen danach der Vektor in den linken Tumor injiziert. MRT-Scans erfolgten einen Tag vor und 13 Tage nach Vektorapplikation. Bei 5 x 10⁶ i.u. Ad-1 nimmt das mittlere Tumorvolumen deutlich zu während es bei 1 x 10⁷ i.u. Ad-1 nur minimal zunimmt. Diese nur minimale Zunahme ist für Ad3 schon bei einer Dosis von 5 x 10⁶ i.u. festzustellen, bei 1 x 10⁷ i.u. hingegen wird bereits eine Verringerung ersichtlich. Damit erweist sich Ad-3 als deutlich effektiver als Ad-1. Die Werte für 1 x 10⁷ i.u. Ad-3 wurden an Tag 3 und Tag 12 nach Vektorinjektion ermittelt. Die Kontrollen (Ad-GFP) nehmen innerhalb des Beobachtungszeitraums von 2 Wochen massiv zu. Extrahepatischen Metastasen wurden bei den Tieren, in denen sie auftraten, nicht für die Berechnung berücksichtig.

## Patentansprüche

1. Ein adenoviraler Vektor, der Nukleinsäure-Sequenzen umfasst, welche für Einzelketten-Interleukin (IL)-12 und das Kostimulator-Protein 4-1BB Ligand kodieren.

2. Vektor gemäß Anspruch 1, **dadurch gekennzeichnet, dass** die Expression der für das Kostimulator-Protein 4-1 BB Ligand kodierenden Sequenz in einer humanen Zelle dazu führt, dass das Kostimulator-Protein 4-1BB Ligand auf der Oberfläche der Zelle vorliegt, und spezifisch von auf der Oberfläche von T-Zellen vorhandenen Rezeptoren gebunden werden kann.

3. Vektor gemäß Anspruch 1 oder 2, **dadurch gekennzeichnet, dass** das Kostimulator-Protein 4-1BB Ligand eine Sequenzhomologie von mindestens 70 %, mindestens 80 % oder mindestens 90 %, zu SEQ ID NO. 4 aufweist, und die Fähigkeit besitzt, T-Zellen spezifisch zu binden und die Immunreaktion zu verstärken.

4. Vektor gemäß einem der Ansprüche 1 bis 3, **dadurch gekennzeichnet, dass** das Einzelketten-IL-12 eine Sequenzhomologie von mindestens 70 %, mindestens 80 % oder mindestens 90 % zu SEQ ID NO. 2 und SEQ ID NO. 3 aufweist und eine immunstimulierende Aktivität besitzt.

5. Vektor gemäß einem der Ansprüche 1 bis 4, **dadurch gekennzeichnet, dass** die Nukleinsäure ferner Sequenzen umfasst, die für Zytokine, für Proteine mit Zytokin-Aktivität und/oder für Kostimulator-Proteine kodieren.

6. Vektor gemäß einem der Ansprüche 1 bis 5, **dadurch gekennzeichnet, dass** die Nukleinsäure ferner eine Sequenz umfasst, die für IL-2 kodiert, wobei diese Sequenz eine Sequenzhomologie von mindestens 70 %, mindestens 80 % oder mindestens 90 %, zu SEQ ID NO. 5 aufweist und das von der Sequenz kodierte Protein eine immunstimulierende Aktivität besitzt.

7. Vektor gemäß einem der Ansprüche 1 bis 6, **dadurch gekennzeichnet, dass** die Nukleinsäure ferner Sequenzen umfasst, die für ein oder mehrere Kostimulator-Proteine kodieren, wobei diese Sequenz eine Sequenzhomologie von mindestens 70 %, mindestens 80 % oder mindestens 90 %, zu SEQ ID NO. 6 (B7-1) oder SEQ ID NO. 7 (B7-2) aufweist, und das von der Sequenz kodierte Protein die Fähigkeit aufweist, T-Zellen spezifisch zu binden und die Immunreaktion zu verstärken.

8. Vektor gemäß einem der Ansprüche 1 bis 7, **dadurch gekennzeichnet, dass** die Nukleinsäure Sequenzen umfasst, welche eine Sequenzhomologie von mindestens 90 % zu der in SEQ ID NO. 2 und 3 (IL-12), in SEQ ID NO. 4 (4-1BB Ligand), in SEQ ID NO. 5 (IL-2) und einer aus der in SEQ ID NO. 6 (B7-1) oder 7 (B7-2) gezeigten Sequenz aufweist und jeweils für ein Proteine kodiert, das die Aktivität des korrespondierenden natürlichen Proteins besitzt.

9. Vektor gemäß einem der Ansprüche 1 bis 8, **dadurch gekennzeichnet, dass** die Nukleinsäure ferner einen oder mehrere Promotoren und einen oder mehrere interne Ribosomeneintrittsstellen umfasst.

10. Vektor gemäß einem der Ansprüche 1 bis 9, **dadurch gekennzeichnet, dass** es sich bei dem adenoviralen Vektor um einen adenoviralen Vektor der ersten oder zweiten Generation (Deletion in E1, E2, E3, E4, etc.) oder einen Helfer-abhängigen adenoviralen Vektor handelt.

11. Vektor gemäß Anspruch 6, **dadurch gekennzeichnet, dass** der Vektor Nukleinsäure-Sequenzen enthält, die eine Sequenzhomologie von mindestens 90 % zu SEQ ID NO. 2 und 3, SEQ ID NO. 4, SEQ. ID NO. 5 und gegebenenfalls SEQ ID NO. 6 oder 7 aufweisen und jeweils für ein Protein kodieren, das die Aktivität des korrespondierenden natürlichen Proteins besitzt.

12. Vektor gemäß Anspruch 11, der ferner durch folgende Merkmale gekennzeichnet ist:
(a) der Vektor weist einen Tumor-unspezifischen Promotor auf, der die Expression aller für immunstimulierende Proteine kodierenden Gensequenzen bewirkt; und
(b) der Vektor weist vor jeder Gensequenz, die nicht unmittelbar hinter dem in (a) genannten Promotor liegt, eine IRES-Sequenz auf.

13. Viruspartikel, **dadurch gekennzeichnet, dass** das Viruspartikel Vektoren gemäß einem der Ansprüche 1 bis 12 umfasst.

14. Arzneimittel, **dadurch gekennzeichnet, dass** es Vektoren gemäß einem der Ansprüche 1 bis 12 oder Viruspartikel gemäß Anspruch 13 umfasst.

15. Arzneimittel gemäß Anspruch 14, **dadurch gekennzeichnet, dass** der Vektor in einer Konzentration von nicht mehr als 1 x 10¹¹, vorzugsweise nicht mehr als 1 x 10¹⁰, nicht mehr als 1 x 10⁹, 1 x 10⁷ oder 1 x 10⁶, pro Dosiereinheit vorliegt.

16. Arzneimittel gemäß Anspruch 14 oder 15, **dadurch gekennzeichnet, dass** das Arzneimittel als Lösung für die intratumorale Injektion oder als Trägermaterial, welches den Vektor nach Implantation in den Tumor über einen gewissen Zeitraum freisetzt, formuliert ist.

17. Arzneimittel, **dadurch gekennzeichnet, dass** es Vektoren gemäß einem der Ansprüche 1 bis 12 oder Viruspartikel gemäß Anspruch 13 umfasst, zur Behandlung von Infektionskrankheiten oder Prionenerkrankungen.

18. Arzneimittel gemäß Anspruch 17 zur Behandlung gemäß Anspruch 17, **dadurch gekennzeichnet, dass** der die Infektion durch das humane Immundefizienzvirus (HIV), durch Hepatitisvirus Typ A, B oder C (HAV, HBV, HCV), durch Cytomegalievirus (CMV) oder durch humane Papillomaviren HPV verursacht wurde.

## Claims

1. An adenoviral vector comprising nucleic acid sequences which code for single chain IL-12 and for the costimulator protein 4-1 BB ligand.

2. The vector according to claim 1, **characterized in that** the expression of the sequence coding for the costimulator protein 4-1 BB ligand within a human cell results in the costimulator protein at the surface of the cell and that can be bound specifically by receptors present on the surface of T-cells.

3. The vector according to claim 1 or 2, **characterized in that** the costimulator protein 4-1 BB ligand has a sequence homology of at least 70 %, at least 80 % or at least 90 % in relation to the sequence of SEQ ID NO: 4 and has the ability to bind T-cells specifically and to stimulate immune reaction.

4. The vector according to claims 1 to 3, **characterized in that** the single chain IL-12 has a sequence homology of at least 70 %, at least 80 % or at least 90 % in relation to the sequences of SEQ ID NOs: 2 and 3 and has an immune stimulating activity.

5. The vector according to claims 1 to 4, **characterized in that** the nucleic acid further comprises sequences coding for cytokines, for proteins with cytokine activity and/or for costimulator proteins.

6. The vector according to claims 1 to 5, **characterized in that** the nucleic acid further comprises a sequence coding for IL-2, wherein said sequence has a sequence homology of at least 70 %, at least 80 % or at least 90 % in relation to the sequence of SEQ ID NO: 5 and that the protein encoded by said sequence has an immune stimulating activity.

7. The vector according to claims 1 to 6, **characterized in that** the nucleic acid further comprises sequences coding for one or several costimulator proteins, wherein said sequence has a sequence homology of at least 70 %, at least 80 % or at least 90 % in relation to the sequences of SEQ ID NO: 6 (B7-1) or SEQ ID NO:7 (B7-2) and that the protein of said sequence has the ability to bind T-cells specifically and to stimulate immune reaction.

8. The vector according to claims 1 to 7, **characterized in that** the nucleic acid comprises sequences having a sequence homology of at least 90 % in relation to the sequences of SEQ ID NOs: 2 and 3 (IL-12), of SEQ ID NO: 4 (4-1 BB ligand), of SEQ ID NO: 5 (IL-2) and to one of the shown sequence of SEQ ID NOs: 6 (B7-1) or 7 (B7-2) and each of the sequences coding for a protein having the activity of the corresponding natural protein.

9. The vector according to claims 1 to 8, **characterized in that** the nucleic acid further comprises one or several promotors and one or several internal ribosome entry sites.

10. The vector according to claims 1 to 9, **characterized in that** the adenoviral vector is an adenoviral vector of the first or second generation (deletion in E1, E2, E3, E4, etc.) or a helper-dependent adenoviral vector.

11. The vector according to claim 6, **characterized in that** the vector contains nucleic acids having a sequence homology of at least 90 % in relation to the sequences of SEQ ID NOs: 2 and 3, of SEQ ID NO: 4, of SEQ ID NO: 5 and optionally of SEQ ID NOs: 6 or 7 and each of the sequences coding for a protein having the activity of the corresponding natural protein.

12. The vector according to claim 11, which is further **characterized by** the following features:
(a) the vector contains a tumor unspecific promotor that mediates the expression of all gene sequences coding for immune stimulatory proteins; and
(b) the vector contains an IRES sequence preceding before each gene sequence that is not located immediately behind the promotor mentioned in (a).

13. Virus particles, **characterized in that** the virus particle comprises vectors according to one of claims 1 to 12.

14. A medicament, **characterized in that** said medicament comprises vectors according to one of claims 1 to 12 or virus particles according to claim 13.

15. The medicament according to claim 14, **characterized in that** the vector is present in a concentration of not more than 1×10¹¹, preferrably not more than 1×10¹⁰, not more than 1×10⁹, 1×10⁷ or 1×10⁶ per dose unit.

16. The medicament according to claims 14 or 15, **characterized in that** the medicament is formulated as solution for the intra-tumoral injection or as a carrier, that releases the vector over a certain time period after implantation into the tumor.

17. The medicament, **characterized in that** said medicament comprises vectors according to one of claims 1 to 12 or virus particles according to claim 13 for the treatment of infectious diseases or prionic diseases.

18. The medicament according to claim 17 for the treatment according to claim 17, **characterized in that** the infection was caused by human immunodeficiency virus (HIV), hepatitis virus type A, B or C (HAV, HBV, HCV), Cytomegalovirus (CMV) or human papilloma virus (HPV).

## Revendications

1. Vecteur adénoviral, qui comprend des séquences d'acide nucléique, qui codent l'interleukine 12 (Il-12) à chaine unique et la protéine co-stimulatrice ligand 4-1BB.

2. Vecteur selon la revendication 1, **caractérisé en ce que** l'expression de la séquence codant la protéine co-stimulatrice ligand 4-1BB dans une cellule humaine entraine le fait que la protéine co-stimulatrice ligand 4-1BB est présente à la surface de la cellule et **en ce qu'**elle peut être liée spécifiquement aux récepteurs présents à la surface de cellules T.

3. Vecteur selon la revendication 1 ou 2, **caractérisé en ce que** la protéine co-stimulatrice ligand 4-1BB présente une homologie de séquence d'au moins 70%, au moins 80% ou au moins 90% avec SEQ ID N°4, et **en ce qu'**elle possède la capacité de lier spécifiquement des cellules T et de renforcer la réaction immune.

4. Vecteur selon l'une des revendications 1 à 3, **caractérisé en ce que** l'IL-12 à chaine unique présente une homologie de séquence d'au moins 70%, au moins 80% ou au moins 90% avec SEQ ID N°2 et SEQ ID N°3, et **en ce qu'**elle possède une activité immunostimulante.

5. Vecteur selon l'une des revendications 1 à 4, **caractérisé en ce que** l'acide nucléique comprend en outre, des séquences qui codent des cytokines, des protéines à activité de cytokine et/ou des protéines co-stimulatrices.

6. Vecteur selon l'une des revendications 1 à 5, **caractérisé en ce que** l'acide nucléique comprend en outre, une séquence qui code IL-2, où cette séquence présente une homologie de séquence d'au moins 70%, au moins 80% ou au moins 90% avec SEQ ID N°5, et **en ce que** la protéine codée par la séquence possède une activité immunostimulante.

7. Vecteur selon l'une des revendications 1 à 6, **caractérisé en ce que** l'acide nucléique comprend en outre, des séquences qui codent une ou plusieurs protéines co-stimulatrices, où cette séquence présente une homologie de séquence d'au moins 70%, au moins 80% ou au moins 90% avec SEQ ID N°6 (B7-1) ou SEQ ID N°7 (B7-2), et **en ce que** la protéine codée par la séquence présente la capacité de lier spécifiquement des cellules T et de renforcer la réaction immune.

8. Vecteur selon l'une des revendications 1 à 7, **caractérisé en ce que** l'acide nucléique comprend en outre, une séquence qui présente une homologie de séquence d'au moins 90% avec la séquence montrée en SEQ ID N°2 et 3 (IL-12), en SEQ ID N°4 (ligand 4-1BB), en SEQ ID N°5 (IL-2) et une des séquences montrées en SEQ ID N°6 (B7-1) ou 7 (B7-2), et **en ce qu'**elle code chaque fois, une protéine qui possède l'activité de la protéine naturelle correspondante.

9. Vecteur selon l'une des revendications 1 à 8, **caractérisé en ce que** l'acide nucléique comprend en outre, un ou plusieurs promoteurs et un ou plusieurs sites internes de liaison au ribosome.

10. Vecteur selon l'une des revendications 1 à 9, **caractérisé en ce que** le vecteur adénoviral consiste en un vecteur adénoviral de première ou de deuxième génération (délétion en E1, E2, E3, E4, etc.) ou en un vecteur adénoviral dépendant d'un auxiliaire.

11. Vecteur selon la revendication 6, **caractérisé en ce que** le vecteur contient des séquences d'acide nucléique, qui présentent une homologie de séquence d'au moins 90% avec SEQ ID N°2 et 3, SEQ ID N°4, SEQ ID N°5 et le cas échéant, SEQ ID N°6 ou 7 et qui codent chaque fois, une protéine qui possède l'activité de la protéine naturelle correspondante.

12. Vecteur selon la revendication 11, qui présente en outre, les caractéristiques suivantes :
(a) le vecteur présente un promoteur non spécifique de tumeur, qui agit sur l'expression de toutes les séquences géniques codant des protéines immunostimulantes ; et
(b) le vecteur présente pour chaque séquence génique, qui ne se trouve pas directement derrière le promoteur cité en (a), une séquence IRES.

13. Particule virale, **caractérisée en ce que** la particule virale comprend le vecteur selon l'une des revendications 1 à 12.

14. Médicament, **caractérisé en ce qu'**il comprend le vecteur selon l'une des revendications 1 à 12 ou la particule virale selon la revendication 13.

15. Médicament selon la revendication 14, **caractérisé en ce que** le vecteur est présent en une concentration non supérieure à 1 x 10¹¹, de préférence non supérieure à 1 x 10¹⁰, non supérieure à 1 x 10⁹, 1 x 10⁷ ou 1 x 10⁶, par unité de dosage.

16. Médicament selon la revendication 14 ou 15, **caractérisé en ce que** le médicament est formulé comme une solution pour l'injection intra-tumorale ou comme un matériau support, qui libère le vecteur pendant une certaine période après implantation dans la tumeur.

17. Médicament, **caractérisé en ce qu'**il comprend le vecteur selon l'une des revendications 1 à 12 ou la particule virale selon la revendication 13, destiné au traitement de maladies infectieuses ou de maladies à prion.

18. Médicament selon la revendication 17 pour un traitement selon la revendication 17, **caractérisé en ce que** l'infection est engendrée par le virus de l'immunodéficience humaine (VIH), par le virus de l'hépatite de type A, B ou C (VHA, VHB, VHC), par le cytomégalovirus (CMV) ou par des papillomavirus humains (HPV).


### SEQUENCE LISTING

<110> Universitätsklinikum Hamburg-Eppendorf
<120> Nukleinsäuren und deren Verwendung für die Gentherapie
<130> P61712
<140> DE 102 48 141.5
   <141> 2002-10-11
<160> 11
<170> PatentIn version 3.1
<210> 1
   <211> 5252
   <212> DNA
   <213> Artificial Sequence
<220>
   <223> Vektor mit tricistronischer Expressionskassette als Insert
<400> 1
   tagtaatcaa ttacggggtc attagttcat agcccatata tggagttccg cgttacataa     60
   cttacggtaa atggcccgcc tggctgaccg cccaacgacc cccgcccatt gacgtcaata    120
   atgacgtatg ttcccatagt aacgccaata gggactttcc attgacgtca atgggtggag    180
   tatttacggt aaactgccca cttggcagta catcaagtgt atcatatgcc aagtacgccc    240
   cctattgacg tcaatgacgg taaatggccc gcctggcatt atgcccagta catgacctta    300
   tgggactttc ctacttggca gtacatctac gtattagtca tcgctattac catggtgatg    360
   cggttttggc agtacatcaa tgggcgtgga tagcggtttg actcacgggg atttccaagt    420
   ctccacccca ttgacgtcaa tgggagtttg ttttggcacc aaaatcaacg ggactttcca    480
   aaatgtcgta acaactccgc cccattgacg caaatgggcg gtaggcgtgt acggtgggag    540
   gtctatataa gcagagctgg tttagtgaac cgtcagatcc gctagagatc tggatccgaa    600
   ttcgccgcca ccatgggtcc tcagaagcta accatctcct ggtttgccat cgttttgctg    660
   gtgtctccac tcatggccat gtgggagctg gagaaagacg tttatgttgt agaggtggac    720
   tggactcccg atgcccctgg agaaacagtg aacctcacct gtgacacgcc tgaagaagat    780
   gacatcacct ggacctcaga ccagagacat ggagtcatag gctctggaaa gaccctgacc    840
   atcactgtca aagagtttct agatgctggc cagtacacct gccacaaagg aggcgagact    900
   ctgagccact cacatctgct gctccacaag aaggaaaatg gaatttggtc cactgaaatt    960
   ttaaaaaatt tcaaaaacaa gactttcctg aagtgtgaag caccaaatta ctccggacgg   1020
   ttcacgtgct catggctggt gcaaagaaac atggacttga agttcaacat caagagcagt   1080
   agcagttccc ctgactctcg ggcagtgaca tgtggaatgg cgtctctgtc tgcagagaag   1140
   gtcacactgg accaaaggga ctatgagaag tattcagtgt cctgccagga ggatgtcacc   1200
   tgcccaactg ccgaggagac cctgcccatt gaactggcgt tggaagcacg gcagcagaat   1260
   aaatatgaga actacagcac cagcttcttc atcagggaca tcatcaaacc agacccgccc   1320
   aagaacttgc agatgaagcc tttgaagaac tcacaggtgg aggtcagctg ggagtaccct   1380
   gactcctgga gcactcccca ttcctacttc tccctcaagt tctttgttcg aatccagcgc   1440
   aagaaagaaa agatgaagga gacagaggag gggtgtaacc agaaaggtgc gttcctcgta   1500
   gagaagacat ctaccgaagt ccaatgcaaa ggcgggaatg tctgcgtgca agctcaggat   1560
   cgctattaca attcctcatg cagcaagtgg gcatgtgttc cctgcagggt ccgatccggt   1620
   ggcggtggct cgggcggtgg tgggtcgggt ggcggcggat ctagggtcat tccagtctct   1680
   ggacctgcca ggtgtcttag ccagtcccga aacctgctga agaccacaga tgacatggtg   1740
   aagacggcca gagaaaagct gaaacattat tcctgcactg ctgaagacat cgatcatgaa   1800
   gacatcacac gggaccaaac cagcacattg aagacctgtt taccactgga actacacaag   1860
   aacgagagtt gcctggctac tagagagact tcttccacaa caagagggag ctgcctgccc   1920
   ccacagaaga cgtctttgat gatgaccctg tgccttggta gcatctatga ggacttgaag   1980
   atgtaccaga cagagttcca ggccatcaac gcagcacttc agaatcacaa ccatcagcag   2040
   atcattctag acaagggcat gctggtggcc atcgatgagc tgatgcagtc tctgaatcat   2100
   aatggcgaga ctctgcgcca gaaacctcct gtgggagaag cagaccctta cagagtgaaa   2160
   atgaagctct gcatcctgct tcacgccttc agcacccgcg tcgtgaccat caacagggtg   2220
   atgggctatc tgagctccgc ctgagaattg atccggatta gtccaatttg ttaaagacag   2280
   gatgaagctt aaaacagctc tggggttgta cccaccccag aggcccacgt ggcggctagt   2340
   actccggtat tgcggtaccc ttgtacgcct gttttatact cccttcccgt aacttagacg   2400
   cacaaaacca agttcaatag aagggggtac aaaccagtac caccacgaac aagcacttct   2460
   gtttccccgg tgatgtcgta tagactgctt gcgtggttga aagcgacgga tccgttatcc   2520
   gcttatgtac ttcgagaagc ccagtaccac ctcggaatct tcgatgcgtt gcgctcagca   2580
   ctcaacccca gagtgtagct taggctgatg agtctggaca tccctcaccg gtgacggtgg   2640
   tccaggctgc gttggcggcc tacctatggc taacgccatg ggacgctagt tgtgaacaag   2700
   gtgtgaagag cctattgagc tacataagaa tcctccggcc cctgaatgcg gctaatccca   2760
   acctcggagc aggtggtcac aaaccagtga ttggcctgtc gtaacgcgca agtccgtggc   2820
   ggaaccgact actttgggtg tccgtgtttc cttttatttt attgtggctg cttatggtga   2880
   caatcacaga ttgttatcat aaagcgaatt ggattgcggc cgcgccacca tggaccagca   2940
   cacacttgat gtggaggata ccgcggatgc cagacatcca gcaggtactt cgtgcccctc   3000
   ggatgcggcg ctcctcagag ataccgggct cctcgcggac gctgcgctcc tctcagatac   3060
   tgtgcgcccc acaaatgccg cgctccccac ggatgctgcc taccctgcgg ttaatgttcg   3120
   ggatcgcgag gccgcgtggc cgcctgcact gaacttctgt tcccgccacc caaagctcta   3180
   tggcctagtc gctttggttt tgctgcttct gatcgccgcc tgtgttccta tcttcacccg   3240
   caccgagcct cggccagcgc tcacaatcac cacctcgccc aacctgggta cccgagagaa   3300
   taatgcagac caggtcaccc ctgtttccca cattggctgc cccaacacta cacaacaggg   3360
   ctctcctgtg ttcgccaagc tactggctaa aaaccaagca tcgttgtgca atacaactct   3420
   gaactggcac agccaagatg gagctgggag ctcataccta tctcaaggtc tgaggtacga   3480
   agaagacaaa aaggagttgg tggtagacag tcccgggctc tactacgtat ttttggaact   3540
   gaagctcagt ccaacattca caaacacagg ccacaaggtg cagggctggg tctctcttgt   3600
   tttgcaagca aagcctcagg tagatgactt tgacaacttg gccctgacag tggaactgtt   3660
   cccttgctcc atggagaaca agttagtgga ccgttcctgg agtcaactgt tgctcctgaa   3720
   ggctggccac cgcctcagtg tgggtctgag ggcttatctg catggagccc aggatgcata   3780
   cagagactgg gagctgtctt atcccaacac caccagcttt ggactctttc ttgtgaaacc   3840
   cgacaaccca tgggaatgag aactatcctt cttgtgactg gcgcgcctga tcaatcgatg   3900
   tttaaacgtt attttccacc atattgccgt cttttggcaa tgtgagggcc cggaaacctg   3960
   gccctgtctt cttgacgagc attcctaggg gtctttcccc tctcgccaaa ggaatgcaag   4020
   gtctgttgaa tgtcgtgaag gaagcagttc ctctggaagc ttcttgaaga caaacaacgt   4080
   ctgtagcgac cctttgcagg cagcggaacc ccccacctgg cgacaggtgc ctctgcggcc   4140
   aaaagccacg tgtataagat acacctgcaa aggcggcaca accccagtgc cacgttgtga   4200
   gttggatagt tgtggaaaga gtcaaatggc tctcctcaag cgtattcaac aaggggctga   4260
   aggatgccca gaaggtaccc cattgtatgg gatctgatct ggggcctcgg tgcacatgct   4320
   ttacgtgtgt ttagtcgagg ttaaaaaaac gtctaggccc cccgaaccac ggggacgtgg   4380
   ttttcctttg aaaaacacga ttctcgagac tagtgccacc atgtacagca tgcagctcgc   4440
   atcctgtgtc acattgacac ttgtgctcct tgtcaacagc gcacccactt caagctccac   4500
   ttcaagctct acagcggaag cacagcagca gcagcagcag cagcagcagc agcagcagca   4560
   cctggagcag ctgttgatgg acctacagga gctcctgagc aggatggaga attacaggaa   4620
   cctgaaactc cccaggatgc tcaccttcaa attttacttg cccaagcagg ccacagaatt   4680
   gaaagatctt cagtgcctag aagatgaact tggacctctg cggcatgttc tggatttgac   4740
   tcaaagcaaa agctttcaat tggaagatgc tgagaatttc atcagcaata tcagagtaac   4800
   tgttgtaaaa ctaaagggct ctgacaacac atttgagtgc caattcgatg atgagtcagc   4860
   aactgtggtg gactttctga ggagatggat agccttctgt caaagcatca tctcaacaag   4920
   ccctcaataa ctatgtaacg cgtgctagca tggccggccg cggccgcggc cgctcgagcc   4980
   taagcttcta gataagatat ccgatccacc ggatctagat aactgatcat aatcagccat   5040
   accacatttg tagaggtttt acttgcttta aaaaacctcc cacacctccc cctgaacctg   5100
   aaacataaaa tgaatgcaat tgttgttgtt aacttgttta ttgcagctta taatggttac   5160
   aaataaagca atagcatcac aaatttcaca aataaagcat ttttttcact gcattctagt   5220
   tgtggtttgt ccaaactcat caatgtatct ta                                 5252
<210> 2
   <211> 987
   <212> DNA
   <213> Homo sapiens
<400> 2
   atgtgtcacc agcagttggt catctcttgg ttttccctgg tttttctggc atctcccctc    60
   gtggccatat gggaactgaa gaaagatgtt tatgtcgtag aattggattg gtatccggat   120
   gcccctggag aaatggtggt cctcacctgt gacacccctg aagaagatgg tatcacctgg   180
   accttggacc agagcagtga ggtcttaggc tctggcaaaa ccctgaccat ccaagtcaaa   240
   gagtttggag atgctggcca gtacacctgt cacaaaggag gcgaggttct aagccattcg   300
   ctcctgctgc ttcacaaaaa ggaagatgga atttggtcca ctgatatttt aaaggaccag   360
   aaagaaccca aaaataagac ctttctaaga tgcgaggcca agaattattc tggacgtttc   420
   acctgctggt ggctgacgac aatcagtact gatttgacat tcagtgtcaa aagcagcaga   480
   ggctcttctg acccccaagg ggtgacgtgc ggagctgcta cactctctgc agagagagtc   540
   agaggggaca acaaggagta tgagtactca gtggagtgcc aggaggacag tgcctgccca   600
   gctgctgagg agagtctgcc cattgaggtc atggtggatg ccgttcacaa gctcaagtat   660
   gaaaactaca ccagcagctt cttcatcagg gacatcatca aacctgaccc acccaagaac   720
   ttgcagctga agccattaaa gaattctcgg caggtggagg tcagctggga gtaccctgac   780
   acctggagta ctccacattc ctacttctcc ctgacattct gcgttcaggt ccagggcaag   840
   agcaagagag aaaagaaaga tagagtcttc acggacaaga cctcagccac ggtcatctgc   900
   cgcaaaaatg ccagcattag cgtgcgggcc caggaccgct actatagctc atcttggagc   960
   gaatgggcat ctgtgccctg cagttag                                        987
<210> 3
   <211> 762
   <212> DNA
   <213> Homo sapiens
<400> 3
   atgtggcccc ctgggtcagc ctcccagcca ccgccctcac ctgccgcggc cacaggtctg      60
   catccagcgg ctcgccctgt gtccctgcag tgccggctca gcatgtgtcc agcgcgcagc     120
   ctcctccttg tggctaccct ggtcctcctg gaccacctca gtttggccag aaacctcccc     180
   gtggccactc cagacccagg aatgttccca tgccttcacc actcccaaaa cctgctgagg     240
   gccgtcagca acatgctcca gaaggccaga caaactctag aattttaccc ttgcacttct     300
   gaagagattg atcatgaaga tatcacaaaa gataaaacca gcacagtgga ggcctgttta     360
   ccattggaat taaccaagaa tgagagttgc ctaaattcca gagagacctc tttcataact     420
   aatgggagtt gcctggcctc cagaaagacc tcttttatga tggccctgtg ccttagtagt     480
   atttatgaag acttgaagat gtaccaggtg gagttcaaga ccatgaatgc aaagcttctg     540
   atggatccta agaggcagat ctttctagat caaaacatgc tggcagttat tgatgagctg     600
   atgcaggccc tgaatttcaa cagtgagact gtgccacaaa aatcctccct tgaagaaccg     660
   gatttttata aaactaaaat caagctctgc atacttcttc atgctttcag aattcgggca     720
   gtgactattg atagagtgat gagctatctg aatgcttcct aa                        762
<210> 4
   <211> 768
   <212> DNA
   <213> Homo sapiens
<400> 4
   gtcatggaat acgcctctga cgcttcactg gaccccgaag ccccgtggcc tcccgcgccc      60
   cgcgctcgcg cctgccgcgt actgccttgg gccctggtcg cggggctgct gctgctgctg     120
   ctgctcgctg ccgcctgcgc cgtcttcctc gcctgcccct gggccgtgtc cggggctcgc     180
   gcctcgcccg gctccgcggc cagcccgaga ctccgcgagg gtcccgagct ttcgcccgac     240
   gatcccgccg gcctcttgga cctgcggcag ggcatgtttg cgcagctggt ggcccaaaat     300
   gttctgctga tcgatgggcc cctgagctgg tacagtgacc caggcctggc aggcgtgtcc     360
   ctgacggggg gcctgagcta caaagaggac acgaaggagc tggtggtggc caaggctgga     420
   gtctactatg tcttctttca actagagctg cggcgcgtgg tggccggcga gggctcaggc     480
   tccgtttcac ttgcgctgca cctgcagcca ctgcgctctg ctgctggggc cgccgccctg     540
   gctttgaccg tggacctgcc acccgcctcc tccgaggctc ggaactcggc cttcggtttc     600
   cagggccgct tgctgcacct gagtgccggc cagcgcctgg gcgtccatct tcacactgag     660
   gccagggcac gccatgcctg gcagcttacc cagggcgcca cagtcttggg actcttccgg     720
   gtgacccccg aaatcccagc cggactccct tcaccgaggt cggaataa                  768
<210> 5
   <211> 473
   <212> DNA
   <213> Homo sapiens
<400> 5
   atgtacagga tgcaactcct gtcttgcatt gcactaattc ttgcacttgt cacaaacagt     60
   gcacctactt caagttcgac aaagaaaaca aagaaaacac agctacaact ggagcattta    120
   ctgctggatt tacagatgat tttgaatgga attaataatt acaagaatcc caaactcacc    180
   aggatgctca catttaagtt ttacatgccc aagaaggcca cagaactgaa acagcttcag    240
   tgtctagaag aagaactcaa acctctggag gaagtgctga atttagctca aagcaaaaac    300
   tttcacttaa gacccaggga cttaatcagc aatatcaacg taatagttct ggaactaaag    360
   ggatctgaaa caacattcat gtgtgaatat gcagatgaga cagcaaccat tgtagaattt    420
   ctgaacagat ggattacctt ttgtcaaagc atcatctcaa cactaacttg ata           473
<210> 6
   <211> 865
   <212> DNA
   <213> Homo sapiens
<400> 6
   atgggccaca cacggaggca gggaacatca ccatccaagt gtccatacct caatttcttt     60
   cagctcttgg tgctggctgg tctttctcac ttctgttcag gtgttatcca cgtgaccaag    120
   gaagtgaaag aagtggcaac gctgtcctgt ggtcacaatg tttctgttga agagctggca    180
   caaactcgca tctactggca aaaggagaag aaaatggtgc tgactatgat gtctggggac    240
   atgaatatat ggcccagtac aagaaccgga ccatctttga tatcactaat aacctctcca    300
   ttgtgatcct ggctctgcgc ccatctgacg agggcacata cgagtgtgtt gttctgaagt    360
   atgaaaaaga cgctttcaag cgggaacacc tggctgaagt gacgttatca gtcaaagctg    420
   acttccctac acctagtata tctgactttg aaattccaac ttctaatatt agaaggataa    480
   tttgctcaac ctctggaggt tttccagagc ctcacctctc ctggttggaa aatggagaag    540
   aattaaatgc catcaacaca acagtttccc aagatcctga aactgagctc tatgctgtta    600
   gcagaaactg gatttcaata tgacaaccaa ccacagcttc atgtgtctca tcaagtatgg    660
   acatttaaga gtgaatcaga ccttcaactg gaatacaacc aagcaagagc attttcctga    720
   taacctgctc ccatcctggg ccattacctt aatctcagta aatggaattt ttgtgatatg    780
   ctgcctgacc tactgctttg ccccaagatg cagagagaga aggaggaatg agagattgag    840
   aagggaaagt gtacgccctg tataa                                          865
<210> 7
   <211> 972
   <212> DNA
   <213> Homo sapiens
<400> 7
   atgggactga gtaacattct ctttgtgatg gccttcctgc tctctggtgc tgctcctctg 60
   aagattcaag cttatttcaa tgagactgca gacctgccat gccaatttgc aaactctcaa 120
   aaccaaagcc tgagtgagct agtagtattt tggcaggacc aggaaaactt ggttctgaat 180
   gaggtatact taggcaaaga gaaatttgac agtgttcatt ccaagtatat gggccgcaca 240
   agttttgatt cggacagttg gaccctgaga cttcacaatc ttcagatcaa ggacaagggc 300
   ttgtatcaat gtatcatcca tcacaaaaag cccacaggaa tgattcgcat ccaccagatg 360
   aattctgaac tgtcagtgct tgctaacttc agtcaacctg aaatagtacc aatttctaat 420
   ataacagaaa atgtgtacat aaatttgacc tgctcatcta tacacggtta cccagaacct 480
   aagaagatga gtgttttgct aagaaccaag aattcaacta tcgagtatga tggtattatg 540
   cagaaatctc aagataatgt cacagaactg tacgacgttt ccatcagctt gtctgtttca 600
   ttccctgatg ttacgagcaa tatgaccatc ttctgtattc tggaaactga caagacgcgg 660
   cttttatctt cacctttctc tatagagctt gaggaccctc agcctccccc agaccacatt 720
   ccttggatta cagctgtact tccaacagtt attatatgtg tgatggtttt ctgtctaatt 780
   ctatggaaat ggaagaagaa gaagcggcct cgcaactctt ataaatgtgg aaccaacaca 840
   atggagaggg aagagagtga acagaccaag aaaagagaaa aaatccatat acctgaaaga 900
   tctgatgaag cccagcgtgt ttttaaaagt tcgaagacat cttcatgcga caaaagtgat 960
   acatgttttt aa 972
<210> 8
   <211> 11746
   <212> DNA
   <213> Artificial Sequence
<220>
   <223> Shuttle fⁿr Ad-3
<220>
   <221> misc_feature
   <222> (1)..(11746)
   <223> "N" = "A", "G", "C" or "T"
<400> 8
   aatgcgccgn nnnnnnnnnn nnnnnnnnnn nnnttaatta annntccctt ccagctctct      60
   gccccttttg gattgaagcc aatatgataa tgagggggtg gagtttgtga cgtggcgcgg      120
   ggcgtgggaa cggggcgggt gacgtagtag tgtggcggaa gtgtgatgtt gcaagtgtgg     180
   cggaacacat gtaagcgacg gatgtggcaa aagtgacgtt tttggtgtgc gccggtgtac     240
   acaggaagtg acaattttcg cgcggtttta ggcggatgtt gtagtaaatt tgggcgtaac     300
   cgagtaagat ttggccattt tcgcgggaaa actgaataag aggaagtgaa atctgaataa     360
   ttttgtgtta ctcatagcgc gtaannnnta atagtaatca attacggggt cattagttca     420
   tagcccatat atggagttcc gcgttacata acttacggta aatggcccgc ctggctgacc     480
   gcccaacgac ccccgcccat tgacgtcaat aatgacgtat gttcccatag taacgccaat     540
   agggactttc cattgacgtc aatgggtgga gtatttacgg taaactgccc acttggcagt     600
   acatcaagtg tatcatatgc caagtacgcc ccctattgac gtcaatgacg gtaaatggcc     660
   cgcctggcat tatgcccagt acatgacctt atgggacttt cctacttggc agtacatcta     720
   cgtattagtc atcgctatta ccatggtgat gcggttttgg cagtacatca atgggcgtgg     780
   atagcggttt gactcacggg gatttccaag tctccacccc attgacgtca atgggagttt     840
   gttttggcac caaaatcaac gggactttcc aaaatgtcgt aacaactccg ccccattgac     900
   gcaaatgggc ggtaggcgtg tacggtggga ggtctatata agcagagctg gtttagtgaa     960
   ccgtcagatc cgctagagat ctggatccga attcgccgcc accatgggtc ctcagaagct     1020
   aaccatctcc tggtttgcca tcgttttgct ggtgtctcca ctcatggcca tgtgggagct     1080
   ggagaaagac gtttatgttg tagaggtgga ctggactccc gatgcccctg gagaaacagt     1140
   gaacctcacc tgtgacacgc ctgaagaaga tgacatcacc tggacctcag accagagaca     1200
   tggagtcata ggctctggaa agaccctgac catcactgtc aaagagtttc tagatgctgg     1260
   ccagtacacc tgccacaaag gaggcgagac tctgagccac tcacatctgc tgctccacaa     1320
   gaaggaaaat ggaatttggt ccactgaaat tttaaaaaat ttcaaaaaca agactttcct     1380
   gaagtgtgaa gcaccaaatt actccggacg gttcacgtgc tcatggctgg tgcaaagaaa     1440
   catggacttg aagttcaaca tcaagagcag tagcagttcc cctgactctc gggcagtgac     1500
   atgtggaatg gcgtctctgt ctgcagagaa ggtcacactg gaccaaaggg actatgagaa     1560
   gtattcagtg tcctgccagg aggatgtcac ctgcccaact gccgaggaga ccctgcccat 1620
   tgaactggcg ttggaagcac ggcagcagaa taaatatgag aactacagca ccagcttctt 1680
   catcagggac atcatcaaac cagacccgcc caagaacttg cagatgaagc ctttgaagaa 1740
   ctcacaggtg gaggtcagct gggagtaccc tgactcctgg agcactcccc attcctactt 1800
   ctccctcaag ttctttgttc gaatccagcg caagaaagaa aagatgaagg agacagagga 1860
   ggggtgtaac cagaaaggtg cgttcctcgt agagaagaca tctaccgaag tccaatgcaa 1920
   aggcgggaat gtctgcgtgc aagctcagga tcgctattac aattcctcat gcagcaagtg 1980
   ggcatgtgtt ccctgcaggg tccgatccgg tggcggtggc tcgggcggtg gtgggtcggg 2040
   tggcggcgga tctagggtca ttccagtctc tggacctgcc aggtgtctta gccagtcccg 2100
   aaacctgctg aagaccacag atgacatggt gaagacggcc agagaaaagc tgaaacatta 2160
   ttcctgcact gctgaagaca tcgatcatga agacatcaca cgggaccaaa ccagcacatt 2220
   gaagacctgt ttaccactgg aactacacaa gaacgagagt tgcctggcta ctagagagac 2280
   ttcttccaca acaagaggga gctgcctgcc cccacagaag acgtctttga tgatgaccct 2340
   gtgccttggt agcatctatg aggacttgaa gatgtaccag acagagttcc aggccatcaa 2400
   cgcagcactt cagaatcaca accatcagca gatcattcta gacaagggca tgctggtggc 2460
   catcgatgag ctgatgcagt ctctgaatca taatggcgag actctgcgcc agaaacctcc 2520
   tgtgggagaa gcagaccctt acagagtgaa aatgaagctc tgcatcctgc ttcacgcctt 2580
   cagcacccgc gtcgtgacca tcaacagggt gatgggctat ctgagctccg cctgagaatt 2640
   gatccggatt agtccaattt gttaaagaca ggatgaagct taaaacagct ctggggttgt 2700
   acccacccca gaggcccacg tggcggctag tactccggta ttgcggtacc cttgtacgcc 2760
   tgttttatac tcccttcccg taacttagac gcacaaaacc aagttcaata gaagggggta 2820
   caaaccagta ccaccacgaa caagcacttc tgtttccccg gtgatgtcgt atagactgct 2880
   tgcgtggttg aaagcgacgg atccgttatc cgcttatgta cttcgagaag cccagtacca 2940
   cctcggaatc ttcgatgcgt tgcgctcagc actcaacccc agagtgtagc ttaggctgat 3000
   gagtctggac atccctcacc ggtgacggtg gtccaggctg cgttggcggc ctacctatgg 3060
   ctaacgccat gggacgctag ttgtgaacaa ggtgtgaaga gcctattgag ctacataaga 3120
   atcctccggc ccctgaatgc ggctaatccc aacctcggag caggtggtca caaaccagtg 3180
   attggcctgt cgtaacgcgc aagtccgtgg cggaaccgac tactttgggt gtccgtgttt 3240
   ccttttattt tattgtggct gcttatggtg acaatcacag attgttatca taaagcgaat 3300
   tggattgcgg ccgcgccacc atggaccagc acacacttga tgtggaggat accgcggatg 3360
   ccagacatcc agcaggtact tcgtgcccct cggatgcggc gctcctcaga gataccgggc 3420
   tcctcgcgga cgctgcgctc ctctcagata ctgtgcgccc cacaaatgcc gcgctcccca 3480
   cggatgctgc ctaccctgcg gttaatgttc gggatcgcga ggccgcgtgg ccgcctgcac 3540
   tgaacttctg ttcccgccac ccaaagctct atggcctagt cgctttggtt ttgctgcttc 3600
   tgatcgccgc ctgtgttcct atcttcaccc gcaccgagcc tcggccagcg ctcacaatca 3660
   ccacctcgcc caacctgggt acccgagaga ataatgcaga ccaggtcacc cctgtttccc 3720
   acattggctg ccccaacact acacaacagg gctctcctgt gttcgccaag ctactggcta 3780
   aaaaccaagc atcgttgtgc aatacaactc tgaactggca cagccaagat ggagctggga 3840
   gctcatacct atctcaaggt ctgaggtacg aagaagacaa aaaggagttg gtggtagaca 3900
   gtcccgggct ctactacgta tttttggaac tgaagctcag tccaacattc acaaacacag 3960
   gccacaaggt gcagggctgg gtctctcttg ttttgcaagc aaagcctcag gtagatgact 4020
   ttgacaactt ggccctgaca gtggaactgt tcccttgctc catggagaac aagttagtgg 4080
   accgttcctg gagtcaactg ttgctcctga aggctggcca ccgcctcagt gtgggtctga 4140
   gggcttatct gcatggagcc caggatgcat acagagactg ggagctgtct tatcccaaca 4200
   ccaccagctt tggactcttt cttgtgaaac ccgacaaccc atgggaatga gaactatcct 4260
   tcttgtgact ggcgcgcctg atcaatcgat gtttaaacgt tattttccac catattgccg 4320
   tcttttggca atgtgagggc ccggaaacct ggccctgtct tcttgacgag cattcctagg 4380
   ggtctttccc ctctcgccaa aggaatgcaa ggtctgttga atgtcgtgaa ggaagcagtt 4440
   cctctggaag cttcttgaag acaaacaacg tctgtagcga ccctttgcag gcagcggaac 4500
   cccccacctg gcgacaggtg cctctgcggc caaaagccac gtgtataaga tacacctgca 4560
   aaggcggcac aaccccagtg ccacgttgtg agttggatag ttgtggaaag agtcaaatgg 4620
   ctctcctcaa gcgtattcaa caaggggctg aaggatgccc agaaggtacc ccattgtatg 4680
   ggatctgatc tggggcctcg gtgcacatgc tttacgtgtg tttagtcgag gttaaaaaaa 4740
   cgtctaggcc ccccgaacca cggggacgtg gttttccttt gaaaaacacg attctcgaga 4800
   ctagtgccac catgtacagc atgcagctcg catcctgtgt cacattgaca cttgtgctcc 4860
   ttgtcaacag cgcacccact tcaagctcca cttcaagctc tacagcggaa gcacagcagc 4920
   agcagcagca gcagcagcag cagcagcagc acctggagca gctgttgatg gacctacagg 4980
   agctcctgag caggatggag aattacagga acctgaaact ccccaggatg ctcaccttca 5040
   aattttactt gcccaagcag gccacagaat tgaaagatct tcagtgccta gaagatgaac 5100
   ttggacctct gcggcatgtt ctggatttga ctcaaagcaa aagctttcaa ttggaagatg 5160
   ctgagaattt catcagcaat atcagagtaa ctgttgtaaa actaaagggc tctgacaaca 5220
   catttgagtg ccaattcgat gatgagtcag caactgtggt ggactttctg aggagatgga 5280
   tagccttctg tcaaagcatc atctcaacaa gccctcaata actatgtaac gcgtgctagc 5340
   atggccggcc gcggccgcgg ccgctcgagc ctaagcttct agataagata tccgatccac 5400
   cggatctaga taactgatca taatcagcca taccacattt gtagaggttt tacttgcttt 5460
   aaaaaacctc ccacacctcc ccctgaacct gaaacataaa atgaatgcaa ttgttgttgt 5520
   taacttgttt attgcagctt ataatggtta caaataaagc aatagcatca caaatttcac 5580
   aaataaagca tttttttcac tgcattctag ttgtggtttg tccaaactca tcaatgtatc 5640
   ttaacgcnnn ntaagggtgg gaaagaatat ataaggtggg ggtcttatgt agttttgtat 5700
   ctgttttgca gcagccgccg ccgccatgag caccaactcg tttgatggaa gcattgtgag 5760
   ctcatatttg acaacgcgca tgcccccatg ggccggggtg cgtcagaatg tgatgggctc 5820
   cagcattgat ggtcgccccg tcctgcccgc aaactctact accttgacct acgagaccgt 5880
   gtctggaacg ccgttggaga ctgcagcctc cgccgccgct tcagccgctg cagccaccgc 5940
   ccgcgggatt gtgactgact ttgctttcct gagcccgctt gcaagcagtg cagcttcccg 6000
   ttcatccgcc cgcgatgaca agttgacggc tcttttggca caattggatt ctttgacccg 6060
   ggaacttaat gtcgtttctc agcagctgtt ggatctgcgc cagcaggttt ctgccctgaa 6120
   ggcttcctcc cctcccaatg cggtttaaaa cataaataaa aaaccagact ctgtttggat 6180
   ttggatcaag caagtgtctt gctgtcttta tttaggggtt ttgcgcgcgc ggtaggcccg 6240
   ggaccagcgg tctcggtcgt tgagggtcct gtgtattttt tccaggacgt ggtaaaggtg 6300
   actctggatg ttcagataca tgggcataag cccgtctctg gggtggaggt agcaccactg 6360
   cagagcttca tgctgcgggg tggtgttgta gatgatccag tcgtagcagg agcgctgggc 6420
   gtggtgccta aaaatgtctt tcagtagcaa gctgattgcc aggggcaggc ccttggtgta 6480
   agtgtttaca aagcggttaa gctgggatgg gtgcatacgt ggggatatga gatgcatctt 6540
   ggactgtatt tttaggttgg ctatgttccc agccatatcc ctccggggat tcatgttgtg 6600
   cagaaccacc agcacagtgt atccggtgca cttgggaaat ttgtcatgta gcttagaagg 6660
   aaatgcgtgg aagaacttgg agacgccctt gtgacctcca agattttcca tgcattcgtc 6720
   cataatgatg gcaatgggcc cacgggcggc ggcctgggcg aagatatttc tgggatcact 6780
   aacgtcatag ttgtgttcca ggatgagatc gtcataggcc atttttacaa agcgcgggcg 6840
   gagggtgcca gactgcggta taatggttcc atccggccca ggggcgtagt taccctcaca 6900
   gatttgcatt tcccacgctt tgagttcaga tggggggatc atgtctacct gcggggcgat 6960
   gaagaaaacg gtttccgggg taggggagat cagctgggaa gaaagcaggt tcctgagcag 7020
   ctgcgactta ccgcagccgg tgggcccgta aatcacacct attaccgggt gcaactggta 7080
   gttaagagag ctgcagctgc cgtcatccct gagcaggggg gccacttcgt taagcatgtc 7140
   cctgactcgc atgttttccc tgaccaaatc cgccagaagg cgctcgccgc ccagcgatag 7200
   cagttcttgc aaggaagcaa agtttttcaa cggtttgaga ccgtccgccg taggcatgct 7260
   tttgagcgtt tgaccaagca gttccaggcg gtcccacagc tcggtcacct gctctacggc 7320
   atctcgatcc agcatatctc ctcgtttcgc gggttggggc ggctttcgct gtacggcagt 7380
   agtcggtgct cgtccagacg ggccagggtc atgtctttcc acgggcgcag ggtcctcgtc 7440
   agcgtagtct gggtcacggt gaaggggtgc gctccgggct gcgcgctggc cagggtgcgc 7500
   ttgaggctgg tcctgctggt gctgaagcgc tgccggtctt cgccctgcgc gtcggccagg 7560
   tagcatttga ccatggtgtc atagtccagc ccctccgcgg cgtggccctt ggcgcgcagc 7620
   ttgcccttgg aggaggcgcc gcacgagggg cagtgcagac ttttgagggc gtagagcttg 7680
   ggcgcgagaa ataccgattc cggggagtag gcatccgcgc cgcaggcccc gcagacggtc 7740
   tcgcattcca cgagccaggt gagctctggc cgttcggggt caaaaaccag gtttccccca 7800
   tgctttttga tgcgtttctt acctctggtt tccatgagcc ggtgtccacg ctcggtgacg 7860
   aaaaggctgt ccgtgtcccc gtatacagac tnnngtttaa acgaattcnn natataaaat 7920
   gcaaggtgct gctcaaaaaa tcaggcaaag cctcgcgcaa aaaagaaagc acatcgtagt 7980
   catgctcatg cagataaagg caggtaagct ccggaaccac cacagaaaaa gacaccattt 8040
   ttctctcaaa catgtctgcg ggtttctgca taaacacaaa ataaaataac aaaaaaacat 8100
   ttaaacatta gaagcctgtc ttacaacagg aaaaacaacc cttataagca taagacggac 8160
   tacggccatg ccggcgtgac cgtaaaaaaa ctggtcaccg tgattaaaaa gcaccaccga 8220
   cagctcctcg gtcatgtccg gagtcataat gtaagactcg gtaaacacat caggttgatt 8280
   catcggtcag tgctaaaaag cgaccgaaat agcccggggg aatacatacc cgcaggcgta 8340
   gagacaacat tacagccccc ataggaggta taacaaaatt aataggagag aaaaacacat 8400
   aaacacctga aaaaccctcc tgcctaggca aaatagcacc ctcccgctcc agaacaacat 8460
   acagcgcttc acagcggcag cctaacagtc agccttacca gtaaaaaaga aaacctatta 8520
   aaaaaacacc actcgacacg gcaccagctc aatcagtcac agtgtaaaaa agggccaagt 8580
   gcagagcgag tatatatagg actaaaaaat gacgtaacgg ttaaagtcca caaaaaacac 8640
   ccagaaaacc gcacgcgaac ctacgcccag aaacgaaagc caaaaaaccc acaacttcct 8700
   caaatcgtca cttccgtttt cccacgttac gtaacttccc attttaagaa aactacaatt 8760
   cccaacacat acaagttact ccgccctaaa acctacgtca cccgccccgt tcccacgccc 8820
   cgcgccacgt cacaaactcc accccctcat tatcatattg gcttcaatcc aaaataaggt 8880
   atattattga tgatnnntta attaaggatc cnnncggtgt gaaataccgc acagatgcgt 8940
   aaggagaaaa taccgcatca ggcgctcttc cgcttcctcg ctcactgact cgctgcgctc 9000
   ggtcgttcgg ctgcggcgag cggtatcagc tcactcaaag gcggtaatac ggttatccac 9060
   agaatcaggg gataacgcag gaaagaacat gtgagcaaaa ggccagcaaa aggccaggaa 9120
   ccgtaaaaag gccgcgttgc tggcgttttt ccataggctc cgcccccctg acgagcatca 9180
   caaaaatcga cgctcaagtc agaggtggcg aaacccgaca ggactataaa gataccaggc 9240
   gtttccccct ggaagctccc tcgtgcgctc tcctgttccg accctgccgc ttaccggata 9300
   cctgtccgcc tttctccctt cgggaagcgt ggcgctttct catagctcac gctgtaggta 9360
   tctcagttcg gtgtaggtcg ttcgctccaa gctgggctgt gtgcacgaac cccccgttca 9420
   gcccgaccgc tgcgccttat ccggtaacta tcgtcttgag tccaacccgg taagacacga 9480
   cttatcgcca ctggcagcag ccactggtaa caggattagc agagcgaggt atgtaggcgg 9540
   tgctacagag ttcttgaagt ggtggcctaa ctacggctac actagaagga cagtatttgg 9600
   tatctgcgct ctgctgaagc cagttacctt cggaaaaaga gttggtagct cttgatccgg 9660
   caaacaaacc accgctggta gcggtggttt ttttgtttgc aagcagcaga ttacgcgcag 9720
   aaaaaaagga tctcaagaag atcctttgat cttttctacg gggtctgacg ctcagtggaa    9780
   cgaaaactca cgttaaggga ttttggtcat gagattatca aaaaggatct tcacctagat    9840
   ccttttaaat taaaaatgaa gttttaaatc aatctaaagt atatatgagt aaacttggtc    9900
   tgacagttac caatgcttaa tcagtgaggc acctatctca gcgatctgtc tatttcgttc    9960
   atccatagtt gcctgactcc ccgtcgtgta gataactacg atacgggagg gcttaccatc   10020
   tggccccagt gctgcaatga taccgcgaga cccacgctca ccggctccag atttatcagc   10080
   aataaaccag ccagccggaa gggccgagcg cagaagtggt cctgcaactt tatccgcctc   10140
   catccagtct attaattgtt gccgggaagc tagagtaagt agttcgccag ttaatagttt   10200
   gcgcaacgtt gttgnnnnaa aaaggatctt cacctagatc cttttcacgt agaaagccag   10260
   tccgcagaaa cggtgctgac cccggatgaa tgtcagctac tgggctatct ggacaaggga   10320
   aaacgcaagc gcaaagagaa agcaggtagc ttgcagtggg cttacatggc gatagctaga   10380
   ctgggcggtt ttatggacag caagcgaacc ggaattgcca gctggggcgc cctctggtaa   10440
   ggttgggaag ccctgcaaag taaactggat ggctttctcg ccgccaagga tctgatggcg   10500
   caggggatca agctctgatc aagagacagg atgaggatcg tttcgcatga ttgaacaaga   10560
   tggattgcac gcaggttctc cggccgcttg ggtggagagg ctattcggct atgactgggc   10620
   acaacagaca atcggctgct ctgatgccgc cgtgttccgg ctgtcagcgc aggggcgccc   10680
   ggttcttttt gtcaagaccg acctgtccgg tgccctgaat gaactgcaag acgaggcagc   10740
   gcggctatcg tggctggcca cgacgggcgt tccttgcgca gctgtgctcg acgttgtcac   10800
   tgaagcggga agggactggc tgctattggg cgaagtgccg gggcaggatc tcctgtcatc   10860
   tcaccttgct cctgccgaga aagtatccat catggctgat gcaatgcggc ggctgcatac   10920
   gcttgatccg gctacctgcc cattcgacca ccaagcgaaa catcgcatcg agcgagcacg   10980
   tactcggatg gaagccggtc ttgtcgatca ggatgatctg gacgaagagc atcaggggct   11040
   cgcgccagcc gaactgttcg ccaggctcaa ggcgagcatg cccgacggcg aggatctcgt   11100
   cgtgacccat ggcgatgcct gcttgccgaa tatcatggtg gaaaatggcc gcttttctgg   11160
   attcatcgac tgtggccggc tgggtgtggc ggaccgctat caggacatag cgttggctac   11220
   ccgtgatatt gctgaagagc ttggcggcga atgggctgac cgcttcctcg tgctttacgg   11280
   tatcgccgct cccgattcgc agcgcatcgc cttctatcgc cttcttgacg agttcttctg   11340
   aattttgtta aaatttttgt taaatcagct cattttttaa ccaataggcc gaaatcggca    11400
   acatccctta taaatcaaaa gaatagaccg cgatagggtt gagtgttgtt ccagtttgga   11460
   acaagagtcc actattaaag aacgtggact ccaacgtcaa agggcgaaaa accgtctatc   11520
   agggcgatgg cccactacgt gaaccatcac ccaaatcaag ttttttgcgg tcgaggtgcc   11580
   gtaaagctct aaatcggaac cctaaaggga gcccccgatt tagagcttga cggggaaagc   11640
   cggcgaacgt ggcgagaaag gaagggaaga aagcgaaagg agcgggcgct agggcgctgg   11700
   caagtgtagc ggtcacgctg cgcgtaacca ccacacccgc gcgctt                  11746
<210> 9
   <211> 10633
   <212> DNA
   <213> Artificial Sequence
<220>
   <223> Shuttle fⁿr Ad-2
<220>
   <221> misc_feature
   <222> (1)..(10633)
   <223> "N" = "A", "C", "G" or "T"
<400> 9
   aatgcgccgn nnnnnnnnnn nnnnnnnnnn nnnttaatta annntccctt ccagctctct      60
   gccccttttg gattgaagcc aatatgataa tgagggggtg gagtttgtga cgtggcgcgg     120
   ggcgtgggaa cggggcgggt gacgtagtag tgtggcggaa gtgtgatgtt gcaagtgtgg     180
   cggaacacat gtaagcgacg gatgtggcaa aagtgacgtt tttggtgtgc gccggtgtac     240
   acaggaagtg acaattttcg cgcggtttta ggcggatgtt gtagtaaatt tgggcgtaac     300
   cgagtaagat ttggccattt tcgcgggaaa actgaataag aggaagtgaa atctgaataa     360
   ttttgtgtta ctcatagcgc gtaannnnta atagtaatca attacggggt cattagttca     420
   tagcccatat atggagttcc gcgttacata acttacggta aatggcccgc ctggctgacc     480
   gcccaacgac ccccgcccat tgacgtcaat aatgacgtat gttcccatag taacgccaat     540
   agggactttc cattgacgtc aatgggtgga gtatttacgg taaactgccc acttggcagt     600
   acatcaagtg tatcatatgc caagtacgcc ccctattgac gtcaatgacg gtaaatggcc     660
   cgcctggcat tatgcccagt acatgacctt atgggacttt cctacttggc agtacatcta     720
   cgtattagtc atcgctatta ccatggtgat gcggttttgg cagtacatca atgggcgtgg     780
   atagcggttt gactcacggg gatttccaag tctccacccc attgacgtca atgggagttt     840
   gttttggcac caaaatcaac gggactttcc aaaatgtcgt aacaactccg ccccattgac     900
   gcaaatgggc ggtaggcgtg tacggtggga ggtctatata agcagagctg gtttagtgaa     960
   ccgtcagatc cgctagagat ctggatccga attcgccgcc accatgggtc ctcagaagct    1020
   aaccatctcc tggtttgcca tcgttttgct ggtgtctcca ctcatggcca tgtgggagct    1080
   ggagaaagac gtttatgttg tagaggtgga ctggactccc gatgcccctg gagaaacagt    1140
   gaacctcacc tgtgacacgc ctgaagaaga tgacatcacc tggacctcag accagagaca    1200
   tggagtcata ggctctggaa agaccctgac catcactgtc aaagagtttc tagatgctgg    1260
   ccagtacacc tgccacaaag gaggcgagac tctgagccac tcacatctgc tgctccacaa    1320
   gaaggaaaat ggaatttggt ccactgaaat tttaaaaaat ttcaaaaaca agactttcct    1380
   gaagtgtgaa gcaccaaatt actccggacg gttcacgtgc tcatggctgg tgcaaagaaa    1440
   catggacttg aagttcaaca tcaagagcag tagcagttcc cctgactctc gggcagtgac    1500
   atgtggaatg gcgtctctgt ctgcagagaa ggtcacactg gaccaaaggg actatgagaa    1560
   gtattcagtg tcctgccagg aggatgtcac ctgcccaact gccgaggaga ccctgcccat 1620
   tgaactggcg ttggaagcac ggcagcagaa taaatatgag aactacagca ccagcttctt 1680
   catcagggac atcatcaaac cagacccgcc caagaacttg cagatgaagc ctttgaagaa 1740
   ctcacaggtg gaggtcagct gggagtaccc tgactcctgg agcactcccc attcctactt 1800
   ctccctcaag ttctttgttc gaatccagcg caagaaagaa aagatgaagg agacagagga 1860
   ggggtgtaac cagaaaggtg cgttcctcgt agagaagaca tctaccgaag tccaatgcaa 1920
   aggcgggaat gtctgcgtgc aagctcagga tcgctattac aattcctcat gcagcaagtg 1980
   ggcatgtgtt ccctgcaggg tccgatccgg tggcggtggc tcgggcggtg gtgggtcggg 2040
   tggcggcgga tctagggtca ttccagtctc tggacctgcc aggtgtctta gccagtcccg 2100
   aaacctgctg aagaccacag atgacatggt gaagacggcc agagaaaagc tgaaacatta 2160
   ttcctgcact gctgaagaca tcgatcatga agacatcaca cgggaccaaa ccagcacatt 2220
   gaagacctgt ttaccactgg aactacacaa gaacgagagt tgcctggcta ctagagagac 2280
   ttcttccaca acaagaggga gctgcctgcc cccacagaag acgtctttga tgatgaccct 2340
   gtgccttggt agcatctatg aggacttgaa gatgtaccag acagagttcc aggccatcaa 2400
   cgcagcactt cagaatcaca accatcagca gatcattcta gacaagggca tgctggtggc 2460
   catcgatgag ctgatgcagt ctctgaatca taatggcgag actctgcgcc agaaacctcc 2520
   tgtgggagaa gcagaccctt acagagtgaa aatgaagctc tgcatcctgc ttcacgcctt 2580
   cagcacccgc gtcgtgacca tcaacagggt gatgggctat ctgagctccg cctgagaatt 2640
   gatccggatt agtccaattt gttaaagaca ggatgaagct taaaacagct ctggggttgt 2700
   acccacccca gaggcccacg tggcggctag tactccggta ttgcggtacc cttgtacgcc 2760
   tgttttatac tcccttcccg taacttagac gcacaaaacc aagttcaata gaagggggta 2820
   caaaccagta ccaccacgaa caagcacttc tgtttccccg gtgatgtcgt atagactgct 2880
   tgcgtggttg aaagcgacgg atccgttatc cgcttatgta cttcgagaag cccagtacca 2940
   cctcggaatc ttcgatgcgt tgcgctcagc actcaacccc agagtgtagc ttaggctgat 3000
   gagtctggac atccctcacc ggtgacggtg gtccaggctg cgttggcggc ctacctatgg 3060
   ctaacgccat gggacgctag ttgtgaacaa ggtgtgaaga gcctattgag ctacataaga 3120
   atcctccggc ccctgaatgc ggctaatccc aacctcggag caggtggtca caaaccagtg 3180
   attggcctgt cgtaacgcgc aagtccgtgg cggaaccgac tactttgggt gtccgtgttt 3240
   ccttttattt tattgtggct gcttatggtg acaatcacag attgttatca taaagcgaat 3300
   tggattgcgg ccgcgccacc atggaccagc acacacttga tgtggaggat accgcggatg 3360
   ccagacatcc agcaggtact tcgtgcccct cggatgcggc gctcctcaga gataccgggc 3420
   tcctcgcgga cgctgcgctc ctctcagata ctgtgcgccc cacaaatgcc gcgctcccca 3480
   cggatgctgc ctaccctgcg gttaatgttc gggatcgcga ggccgcgtgg ccgcctgcac 3540
   tgaacttctg ttcccgccac ccaaagctct atggcctagt cgctttggtt ttgctgcttc 3600
   tgatcgccgc ctgtgttcct atcttcaccc gcaccgagcc tcggccagcg ctcacaatca   3660
   ccacctcgcc caacctgggt acccgagaga ataatgcaga ccaggtcacc cctgtttccc   3720
   acattggctg ccccaacact acacaacagg gctctcctgt gttcgccaag ctactggcta   3780
   aaaaccaagc atcgttgtgc aatacaactc tgaactggca cagccaagat ggagctggga   3840
   gctcatacct atctcaaggt ctgaggtacg aagaagacaa aaaggagttg gtggtagaca   3900
   gtcccgggct ctactacgta tttttggaac tgaagctcag tccaacattc acaaacacag   3960
   gccacaaggt gcagggctgg gtctctcttg ttttgcaagc aaagcctcag gtagatgact   4020
   ttgacaactt ggccctgaca gtggaactgt tcccttgctc catggagaac aagttagtgg   4080
   accgttcctg gagtcaactg ttgctcctga aggctggcca ccgcctcagt gtgggtctga   4140
   gggcttatct gcatggagcc caggatgcat acagagactg ggagctgtct tatcccaaca   4200
   ccaccagctt tggactcttt cttgtgaaac ccgacaaccc atgggaatga gaactatcct   4260
   tcttgtgact ggcgcgatcc gatccaccgg atctagataa ctgatcataa tcagccatac   4320
   cacatttgta gaggttttac ttgctttaaa aaacctccca cacctccccc tgaacctgaa   4380
   acataaaatg aatgcaattg ttgttgttaa cttgtttatt gcagcttata atggttacaa   4440
   ataaagcaat agcatcacaa atttcacaaa taaagcattt ttttcactgc attctagttg   4500
   tggtttgtcc aaactcatca atgtatctta acgcnnnnta agggtgggaa agaatatata   4560
   aggtgggggt cttatgtagt tttgtatctg ttttgcagca gccgccgccg ccatgagcac   4620
   caactcgttt gatggaagca ttgtgagctc atatttgaca acgcgcatgc ccccatgggc   4680
   cggggtgcgt cagaatgtga tgggctccag cattgatggt cgccccgtcc tgcccgcaaa   4740
   ctctactacc ttgacctacg agaccgtgtc tggaacgccg ttggagactg cagcctccgc   4800
   cgccgcttca gccgctgcag ccaccgcccg cgggattgtg actgactttg ctttcctgag   4860
   cccgcttgca agcagtgcag cttcccgttc atccgcccgc gatgacaagt tgacggctct   4920
   tttggcacaa ttggattctt tgacccggga acttaatgtc gtttctcagc agctgttgga   4980
   tctgcgccag caggtttctg ccctgaaggc ttcctcccct cccaatgcgg tttaaaacat   5040
   aaataaaaaa ccagactctg tttggatttg gatcaagcaa gtgtcttgct gtctttattt   5100
   aggggttttg cgcgcgcggt aggcccggga ccagcggtct cggtcgttga gggtcctgtg   5160
   tattttttcc aggacgtggt aaaggtgact ctggatgttc agatacatgg gcataagccc   5220
   gtctctgggg tggaggtagc accactgcag agcttcatgc tgcggggtgg tgttgtagat   5280
   gatccagtcg tagcaggagc gctgggcgtg gtgcctaaaa atgtctttca gtagcaagct   5340
   gattgccagg ggcaggccct tggtgtaagt gtttacaaag cggttaagct gggatgggtg   5400
   catacgtggg gatatgagat gcatcttgga ctgtattttt aggttggcta tgttcccagc   5460
   catatccctc cggggattca tgttgtgcag aaccaccagc acagtgtatc cggtgcactt   5520
   gggaaatttg tcatgtagct tagaaggaaa tgcgtggaag aacttggaga cgcccttgtg   5580
   acctccaaga ttttccatgc attcgtccat aatgatggca atgggcccac gggcggcggc   5640
   ctgggcgaag atatttctgg gatcactaac gtcatagttg tgttccagga tgagatcgtc 5700
   ataggccatt tttacaaagc gcgggcggag ggtgccagac tgcggtataa tggttccatc 5760
   cggcccaggg gcgtagttac cctcacagat ttgcatttcc cacgctttga gttcagatgg 5820
   ggggatcatg tctacctgcg gggcgatgaa gaaaacggtt tccggggtag gggagatcag 5880
   ctgggaagaa agcaggttcc tgagcagctg cgacttaccg cagccggtgg gcccgtaaat 5940
   cacacctatt accgggtgca actggtagtt aagagagctg cagctgccgt catccctgag 6000
   caggggggcc acttcgttaa gcatgtccct gactcgcatg ttttccctga ccaaatccgc 6060
   cagaaggcgc tcgccgccca gcgatagcag ttcttgcaag gaagcaaagt ttttcaacgg 6120
   tttgagaccg tccgccgtag gcatgctttt gagcgtttga ccaagcagtt ccaggcggtc 6180
   ccacagctcg gtcacctgct ctacggcatc tcgatccagc atatctcctc gtttcgcggg 6240
   ttggggcggc tttcgctgta cggcagtagt cggtgctcgt ccagacgggc cagggtcatg 6300
   tctttccacg ggcgcagggt cctcgtcagc gtagtctggg tcacggtgaa ggggtgcgct 6360
   ccgggctgcg cgctggccag ggtgcgcttg aggctggtcc tgctggtgct gaagcgctgc 6420
   cggtcttcgc cctgcgcgtc ggccaggtag catttgacca tggtgtcata gtccagcccc 6480
   tccgcggcgt ggcccttggc gcgcagcttg cccttggagg aggcgccgca cgaggggcag 6540
   tgcagacttt tgagggcgta gagcttgggc gcgagaaata ccgattccgg ggagtaggca 6600
   tccgcgccgc aggccccgca gacggtctcg cattccacga gccaggtgag ctctggccgt 6660
   tcggggtcaa aaaccaggtt tcccccatgc tttttgatgc gtttcttacc tctggtttcc 6720
   atgagccggt gtccacgctc ggtgacgaaa aggctgtccg tgtccccgta tacagactnn 6780
   ngtttaaacg aattcnnnat ataaaatgca aggtgctgct caaaaaatca ggcaaagcct 6840
   cgcgcaaaaa agaaagcaca tcgtagtcat gctcatgcag ataaaggcag gtaagctccg 6900
   gaaccaccac agaaaaagac accatttttc tctcaaacat gtctgcgggt ttctgcataa 6960
   acacaaaata aaataacaaa aaaacattta aacattagaa gcctgtctta caacaggaaa 7020
   aacaaccctt ataagcataa gacggactac ggccatgccg gcgtgaccgt aaaaaaactg 7080
   gtcaccgtga ttaaaaagca ccaccgacag ctcctcggtc atgtccggag tcataatgta 7140
   agactcggta aacacatcag gttgattcat cggtcagtgc taaaaagcga ccgaaatagc 7200
   ccgggggaat acatacccgc aggcgtagag acaacattac agcccccata ggaggtataa 7260
   caaaattaat aggagagaaa aacacataaa cacctgaaaa accctcctgc ctaggcaaaa 7320
   tagcaccctc ccgctccaga acaacataca gcgcttcaca gcggcagcct aacagtcagc 7380
   cttaccagta aaaaagaaaa cctattaaaa aaacaccact cgacacggca ccagctcaat 7440
   cagtcacagt gtaaaaaagg gccaagtgca gagcgagtat atataggact aaaaaatgac 7500
   gtaacggtta aagtccacaa aaaacaccca gaaaaccgca cgcgaaccta cgcccagaaa 7560
   cgaaagccaa aaaacccaca acttcctcaa atcgtcactt ccgttttccc acgttacgta 7620
   acttcccatt ttaagaaaac tacaattccc aacacataca agttactccg ccctaaaacc 7680
   tacgtcaccc gccccgttcc cacgccccgc gccacgtcac aaactccacc ccctcattat 7740
   catattggct tcaatccaaa ataaggtata ttattgatga tnnnttaatt aaggatccnn 7800
   ncggtgtgaa ataccgcaca gatgcgtaag gagaaaatac cgcatcaggc gctcttccgc 7860
   ttcctcgctc actgactcgc tgcgctcggt cgttcggctg cggcgagcgg tatcagctca 7920
   ctcaaaggcg gtaatacggt tatccacaga atcaggggat aacgcaggaa agaacatgtg 7980
   agcaaaaggc cagcaaaagg ccaggaaccg taaaaaggcc gcgttgctgg cgtttttcca 8040
   taggctccgc ccccctgacg agcatcacaa aaatcgacgc tcaagtcaga ggtggcgaaa 8100
   cccgacagga ctataaagat accaggcgtt tccccctgga agctccctcg tgcgctctcc 8160
   tgttccgacc ctgccgctta ccggatacct gtccgccttt ctcccttcgg gaagcgtggc 8220
   gctttctcat agctcacgct gtaggtatct cagttcggtg taggtcgttc gctccaagct 8280
   gggctgtgtg cacgaacccc ccgttcagcc cgaccgctgc gccttatccg gtaactatcg 8340
   tcttgagtcc aacccggtaa gacacgactt atcgccactg gcagcagcca ctggtaacag 8400
   gattagcaga gcgaggtatg taggcggtgc tacagagttc ttgaagtggt ggcctaacta 8460
   cggctacact agaaggacag tatttggtat ctgcgctctg ctgaagccag ttaccttcgg 8520
   aaaaagagtt ggtagctctt gatccggcaa acaaaccacc gctggtagcg gtggtttttt 8580
   tgtttgcaag cagcagatta cgcgcagaaa aaaaggatct caagaagatc ctttgatctt 8640
   ttctacgggg tctgacgctc agtggaacga aaactcacgt taagggattt tggtcatgag 8700
   attatcaaaa aggatcttca cctagatcct tttaaattaa aaatgaagtt ttaaatcaat 8760
   ctaaagtata tatgagtaaa cttggtctga cagttaccaa tgcttaatca gtgaggcacc 8820
   tatctcagcg atctgtctat ttcgttcatc catagttgcc tgactccccg tcgtgtagat 8880
   aactacgata cgggagggct taccatctgg ccccagtgct gcaatgatac cgcgagaccc 8940
   acgctcaccg gctccagatt tatcagcaat aaaccagcca gccggaaggg ccgagcgcag 9000
   aagtggtcct gcaactttat ccgcctccat ccagtctatt aattgttgcc gggaagctag 9060
   agtaagtagt tcgccagtta atagtttgcg caacgttgtt gnnnnaaaaa ggatcttcac 9120
   ctagatcctt ttcacgtaga aagccagtcc gcagaaacgg tgctgacccc ggatgaatgt 9180
   cagctactgg gctatctgga caagggaaaa cgcaagcgca aagagaaagc aggtagcttg 9240
   cagtgggctt acatggcgat agctagactg ggcggtttta tggacagcaa gcgaaccgga 9300
   attgccagct ggggcgccct ctggtaaggt tgggaagccc tgcaaagtaa actggatggc 9360
   tttctcgccg ccaaggatct gatggcgcag gggatcaagc tctgatcaag agacaggatg 9420
   aggatcgttt cgcatgattg aacaagatgg attgcacgca ggttctccgg ccgcttgggt 9480
   ggagaggcta ttcggctatg actgggcaca acagacaatc ggctgctctg atgccgccgt 9540
   gttccggctg tcagcgcagg ggcgcccggt tctttttgtc aagaccgacc tgtccggtgc 9600
   cctgaatgaa ctgcaagacg aggcagcgcg gctatcgtgg ctggccacga cgggcgttcc 9660
   ttgcgcagct gtgctcgacg ttgtcactga agcgggaagg gactggctgc tattgggcga 9720
   agtgccgggg caggatctcc tgtcatctca ccttgctcct gccgagaaag tatccatcat   9780
   ggctgatgca atgcggcggc tgcatacgct tgatccggct acctgcccat tcgaccacca   9840
   agcgaaacat cgcatcgagc gagcacgtac tcggatggaa gccggtcttg tcgatcagga   9900
   tgatctggac gaagagcatc aggggctcgc gccagccgaa ctgttcgcca ggctcaaggc   9960
   gagcatgccc gacggcgagg atctcgtcgt gacccatggc gatgcctgct tgccgaatat  10020
   catggtggaa aatggccgct tttctggatt catcgactgt ggccggctgg gtgtggcgga  10080
   ccgctatcag gacatagcgt tggctacccg tgatattgct gaagagcttg gcggcgaatg  10140
   ggctgaccgc ttcctcgtgc tttacggtat cgccgctccc gattcgcagc gcatcgcctt  10200
   ctatcgcctt cttgacgagt tcttctgaat tttgttaaaa tttttgttaa atcagctcat  10260
   tttttaacca ataggccgaa atcggcaaca tcccttataa atcaaaagaa tagaccgcga  10320
   tagggttgag tgttgttcca gtttggaaca agagtccact attaaagaac gtggactcca  10380
   acgtcaaagg gcgaaaaacc gtctatcagg gcgatggccc actacgtgaa ccatcaccca  10440
   aatcaagttt tttgcggtcg aggtgccgta aagctctaaa tcggaaccct aaagggagcc  10500
   cccgatttag agcttgacgg ggaaagccgg cgaacgtggc gagaaaggaa gggaagaaag  10560
   cgaaaggagc gggcgctagg gcgctggcaa gtgtagcggt cacgctgcgc gtaaccacca  10620
   cacccgcgcg ctt                                                     10633
<210> 10
   <211> 9049
   <212> DNA
   <213> Artificial Sequence
<220>
   <223> Shuttle fⁿr Ad-1
<220>
   <221> misc_feature
   <222> (1)..(9049)
   <223> "N" = "A", "C", "G" or "T"
<400> 10
   aatgcgccgn nnnnnnnnnn nnnnnnnnnn nnnttaatta annntccctt ccagctctct     60
   gccccttttg gattgaagcc aatatgataa tgagggggtg gagtttgtga cgtggcgcgg    120
   ggcgtgggaa cggggcgggt gacgtagtag tgtggcggaa gtgtgatgtt gcaagtgtgg    180
   cggaacacat gtaagcgacg gatgtggcaa aagtgacgtt tttggtgtgc gccggtgtac    240
   acaggaagtg acaattttcg cgcggtttta ggcggatgtt gtagtaaatt tgggcgtaac    300
   cgagtaagat ttggccattt tcgcgggaaa actgaataag aggaagtgaa atctgaataa    360
   ttttgtgtta ctcatagcgc gtaannnnta atagtaatca attacggggt cattagttca    420
   tagcccatat atggagttcc gcgttacata acttacggta aatggcccgc ctggctgacc    480
   gcccaacgac ccccgcccat tgacgtcaat aatgacgtat gttcccatag taacgccaat    540
   agggactttc cattgacgtc aatgggtgga gtatttacgg taaactgccc acttggcagt    600
   acatcaagtg tatcatatgc caagtacgcc ccctattgac gtcaatgacg gtaaatggcc 660
   cgcctggcat tatgcccagt acatgacctt atgggacttt cctacttggc agtacatcta 720
   cgtattagtc atcgctatta ccatggtgat gcggttttgg cagtacatca atgggcgtgg 780
   atagcggttt gactcacggg gatttccaag tctccacccc attgacgtca atgggagttt 840
   gttttggcac caaaatcaac gggactttcc aaaatgtcgt aacaactccg ccccattgac 900
   gcaaatgggc ggtaggcgtg tacggtggga ggtctatata agcagagctg gtttagtgaa 960
   ccgtcagatc cgctagagat ctggatccga attcgccgcc accatgggtc ctcagaagct 1020
   aaccatctcc tggtttgcca tcgttttgct ggtgtctcca ctcatggcca tgtgggagct 1080
   ggagaaagac gtttatgttg tagaggtgga ctggactccc gatgcccctg gagaaacagt 1140
   gaacctcacc tgtgacacgc ctgaagaaga tgacatcacc tggacctcag accagagaca 1200
   tggagtcata ggctctggaa agaccctgac catcactgtc aaagagtttc tagatgctgg 1260
   ccagtacacc tgccacaaag gaggcgagac tctgagccac tcacatctgc tgctccacaa 1320
   gaaggaaaat ggaatttggt ccactgaaat tttaaaaaat ttcaaaaaca agactttcct 1380
   gaagtgtgaa gcaccaaatt actccggacg gttcacgtgc tcatggctgg tgcaaagaaa 1440
   catggacttg aagttcaaca tcaagagcag tagcagttcc cctgactctc gggcagtgac 1500
   atgtggaatg gcgtctctgt ctgcagagaa ggtcacactg gaccaaaggg actatgagaa 1560
   gtattcagtg tcctgccagg aggatgtcac ctgcccaact gccgaggaga ccctgcccat 1620
   tgaactggcg ttggaagcac ggcagcagaa taaatatgag aactacagca ccagcttctt 1680
   catcagggac atcatcaaac cagacccgcc caagaacttg cagatgaagc ctttgaagaa 1740
   ctcacaggtg gaggtcagct gggagtaccc tgactcctgg agcactcccc attcctactt 1800
   ctccctcaag ttctttgttc gaatccagcg caagaaagaa aagatgaagg agacagagga 1860
   ggggtgtaac cagaaaggtg cgttcctcgt agagaagaca tctaccgaag tccaatgcaa 1920
   aggcgggaat gtctgcgtgc aagctcagga tcgctattac aattcctcat gcagcaagtg 1980
   ggcatgtgtt ccctgcaggg tccgatccgg tggcggtggc tcgggcggtg gtgggtcggg 2040
   tggcggcgga tctagggtca ttccagtctc tggacctgcc aggtgtctta gccagtcccg 2100
   aaacctgctg aagaccacag atgacatggt gaagacggcc agagaaaagc tgaaacatta 2160
   ttcctgcact gctgaagaca tcgatcatga agacatcaca cgggaccaaa ccagcacatt 2220
   gaagacctgt ttaccactgg aactacacaa gaacgagagt tgcctggcta ctagagagac 2280
   ttcttccaca acaagaggga gctgcctgcc cccacagaag acgtctttga tgatgaccct 2340
   gtgccttggt agcatctatg aggacttgaa gatgtaccag acagagttcc aggccatcaa 2400
   cgcagcactt cagaatcaca accatcagca gatcattcta gacaagggca tgctggtggc 2460
   catcgatgag ctgatgcagt ctctgaatca taatggcgag actctgcgcc agaaacctcc 2520
   tgtgggagaa gcagaccctt acagagtgaa aatgaagctc tgcatcctgc ttcacgcctt 2580
   cagcacccgc gtcgtgacca tcaacagggt gatgggctat ctgagctccg cctgagaatt 2640
   gatccggatt agtccaattt gttaaagaca ggatgaagct tctagataag atatccgatc   2700
   caccggatct agataactga tcataatcag ccataccaca tttgtagagg ttttacttgc    2760
   tttaaaaaac ctcccacacc tccccctgaa cctgaaacat aaaatgaatg caattgttgt    2820
   tgttaacttg tttattgcag cttataatgg ttacaaataa agcaatagca tcacaaattt   2880
   cacaaataaa gcattttttt cactgcattc tagttgtggt ttgtccaaac tcatcaatgt   2940
   atcttaacgc nnnntaaggg tgggaaagaa tatataaggt gggggtctta tgtagttttg   3000
   tatctgtttt gcagcagccg ccgccgccat gagcaccaac tcgtttgatg gaagcattgt   3060
   gagctcatat ttgacaacgc gcatgccccc atgggccggg gtgcgtcaga atgtgatggg   3120
   ctccagcatt gatggtcgcc ccgtcctgcc cgcaaactct actaccttga cctacgagac   3180
   cgtgtctgga acgccgttgg agactgcagc ctccgccgcc gcttcagccg ctgcagccac   3240
   cgcccgcggg attgtgactg actttgcttt cctgagcccg cttgcaagca gtgcagcttc   3300
   ccgttcatcc gcccgcgatg acaagttgac ggctcttttg gcacaattgg attctttgac   3360
   ccgggaactt aatgtcgttt ctcagcagct gttggatctg cgccagcagg tttctgccct   3420
   gaaggcttcc tcccctccca atgcggttta aaacataaat aaaaaaccag actctgtttg   3480
   gatttggatc aagcaagtgt cttgctgtct ttatttaggg gttttgcgcg cgcggtaggc   3540
   ccgggaccag cggtctcggt cgttgagggt cctgtgtatt ttttccagga cgtggtaaag   3600
   gtgactctgg atgttcagat acatgggcat aagcccgtct ctggggtgga ggtagcacca   3660
   ctgcagagct tcatgctgcg gggtggtgtt gtagatgatc cagtcgtagc aggagcgctg   3720
   ggcgtggtgc ctaaaaatgt ctttcagtag caagctgatt gccaggggca ggcccttggt   3780
   gtaagtgttt acaaagcggt taagctggga tgggtgcata cgtggggata tgagatgcat   3840
   cttggactgt atttttaggt tggctatgtt cccagccata tccctccggg gattcatgtt   3900
   gtgcagaacc accagcacag tgtatccggt gcacttggga aatttgtcat gtagcttaga   3960
   aggaaatgcg tggaagaact tggagacgcc cttgtgacct ccaagatttt ccatgcattc   4020
   gtccataatg atggcaatgg gcccacgggc ggcggcctgg gcgaagatat ttctgggatc   4080
   actaacgtca tagttgtgtt ccaggatgag atcgtcatag gccattttta caaagcgcgg   4140
   gcggagggtg ccagactgcg gtataatggt tccatccggc ccaggggcgt agttaccctc   4200
   acagatttgc atttcccacg ctttgagttc agatgggggg atcatgtcta cctgcggggc   4260
   gatgaagaaa acggtttccg gggtagggga gatcagctgg gaagaaagca ggttcctgag   4320
   cagctgcgac ttaccgcagc cggtgggccc gtaaatcaca cctattaccg ggtgcaactg   4380
   gtagttaaga gagctgcagc tgccgtcatc cctgagcagg ggggccactt cgttaagcat   4440
   gtccctgact cgcatgtttt ccctgaccaa atccgccaga aggcgctcgc cgcccagcga   4500
   tagcagttct tgcaaggaag caaagttttt caacggtttg agaccgtccg ccgtaggcat   4560
   gcttttgagc gtttgaccaa gcagttccag gcggtcccac agctcggtca cctgctctac   4620
   ggcatctcga tccagcatat ctcctcgttt cgcgggttgg ggcggctttc gctgtacggc   4680
   agtagtcggt gctcgtccag acgggccagg gtcatgtctt tccacgggcg cagggtcctc 4740
   gtcagcgtag tctgggtcac ggtgaagggg tgcgctccgg gctgcgcgct ggccagggtg 4800
   cgcttgaggc tggtcctgct ggtgctgaag cgctgccggt cttcgccctg cgcgtcggcc 4860
   aggtagcatt tgaccatggt gtcatagtcc agcccctccg cggcgtggcc cttggcgcgc 4920
   agcttgccct tggaggaggc gccgcacgag gggcagtgca gacttttgag ggcgtagagc 4980
   ttgggcgcga gaaataccga ttccggggag taggcatccg cgccgcaggc cccgcagacg 5040
   gtctcgcatt ccacgagcca ggtgagctct ggccgttcgg ggtcaaaaac caggtttccc 5100
   ccatgctttt tgatgcgttt cttacctctg gtttccatga gccggtgtcc acgctcggtg 5160
   acgaaaaggc tgtccgtgtc cccgtataca gactnnngtt taaacgaatt cnnnatataa 5220
   aatgcaaggt gctgctcaaa aaatcaggca aagcctcgcg caaaaaagaa agcacatcgt 5280
   agtcatgctc atgcagataa aggcaggtaa gctccggaac caccacagaa aaagacacca 5340
   tttttctctc aaacatgtct gcgggtttct gcataaacac aaaataaaat aacaaaaaaa 5400
   catttaaaca ttagaagcct gtcttacaac aggaaaaaca acccttataa gcataagacg 5460
   gactacggcc atgccggcgt gaccgtaaaa aaactggtca ccgtgattaa aaagcaccac 5520
   cgacagctcc tcggtcatgt ccggagtcat aatgtaagac tcggtaaaca catcaggttg 5580
   attcatcggt cagtgctaaa aagcgaccga aatagcccgg gggaatacat acccgcaggc 5640
   gtagagacaa cattacagcc cccataggag gtataacaaa attaatagga gagaaaaaca 5700
   cataaacacc tgaaaaaccc tcctgcctag gcaaaatagc accctcccgc tccagaacaa 5760
   catacagcgc ttcacagcgg cagcctaaca gtcagcctta ccagtaaaaa agaaaaccta 5820
   ttaaaaaaac accactcgac acggcaccag ctcaatcagt cacagtgtaa aaaagggcca 5880
   agtgcagagc gagtatatat aggactaaaa aatgacgtaa cggttaaagt ccacaaaaaa 5940
   cacccagaaa accgcacgcg aacctacgcc cagaaacgaa agccaaaaaa cccacaactt 6000
   cctcaaatcg tcacttccgt tttcccacgt tacgtaactt cccattttaa gaaaactaca 6060
   attcccaaca catacaagtt actccgccct aaaacctacg tcacccgccc cgttcccacg 6120
   ccccgcgcca cgtcacaaac tccaccccct cattatcata ttggcttcaa tccaaaataa 6180
   ggtatattat tgatgatnnn ttaattaagg atccnnncgg tgtgaaatac cgcacagatg 6240
   cgtaaggaga aaataccgca tcaggcgctc ttccgcttcc tcgctcactg actcgctgcg 6300
   ctcggtcgtt cggctgcggc gagcggtatc agctcactca aaggcggtaa tacggttatc 6360
   cacagaatca ggggataacg caggaaagaa catgtgagca aaaggccagc aaaaggccag 6420
   gaaccgtaaa aaggccgcgt tgctggcgtt tttccatagg ctccgccccc ctgacgagca 6480
   tcacaaaaat cgacgctcaa gtcagaggtg gcgaaacccg acaggactat aaagatacca 6540
   ggcgtttccc cctggaagct ccctcgtgcg ctctcctgtt ccgaccctgc cgcttaccgg 6600
   atacctgtcc gcctttctcc cttcgggaag cgtggcgctt tctcatagct cacgctgtag 6660
   gtatctcagt tcggtgtagg tcgttcgctc caagctgggc tgtgtgcacg aaccccccgt 6720
   tcagcccgac cgctgcgcct tatccggtaa ctatcgtctt gagtccaacc cggtaagaca 6780
   cgacttatcg ccactggcag cagccactgg taacaggatt agcagagcga ggtatgtagg 6840
   cggtgctaca gagttcttga agtggtggcc taactacggc tacactagaa ggacagtatt 6900
   tggtatctgc gctctgctga agccagttac cttcggaaaa agagttggta gctcttgatc 6960
   cggcaaacaa accaccgctg gtagcggtgg tttttttgtt tgcaagcagc agattacgcg 7020
   cagaaaaaaa ggatctcaag aagatccttt gatcttttct acggggtctg acgctcagtg 7080
   gaacgaaaac tcacgttaag ggattttggt catgagatta tcaaaaagga tcttcaccta 7140
   gatcctttta aattaaaaat gaagttttaa atcaatctaa agtatatatg agtaaacttg 7200
   gtctgacagt taccaatgct taatcagtga ggcacctatc tcagcgatct gtctatttcg 7260
   ttcatccata gttgcctgac tccccgtcgt gtagataact acgatacggg agggcttacc 7320
   atctggcccc agtgctgcaa tgataccgcg agacccacgc tcaccggctc cagatttatc 7380
   agcaataaac cagccagccg gaagggccga gcgcagaagt ggtcctgcaa ctttatccgc 7440
   ctccatccag tctattaatt gttgccggga agctagagta agtagttcgc cagttaatag 7500
   tttgcgcaac gttgttgnnn naaaaaggat cttcacctag atccttttca cgtagaaagc 7560
   cagtccgcag aaacggtgct gaccccggat gaatgtcagc tactgggcta tctggacaag 7620
   ggaaaacgca agcgcaaaga gaaagcaggt agcttgcagt gggcttacat ggcgatagct 7680
   agactgggcg gttttatgga cagcaagcga accggaattg ccagctgggg cgccctctgg 7740
   taaggttggg aagccctgca aagtaaactg gatggctttc tcgccgccaa ggatctgatg 7800
   gcgcagggga tcaagctctg atcaagagac aggatgagga tcgtttcgca tgattgaaca 7860
   agatggattg cacgcaggtt ctccggccgc ttgggtggag aggctattcg gctatgactg 7920
   ggcacaacag acaatcggct gctctgatgc cgccgtgttc cggctgtcag cgcaggggcg 7980
   cccggttctt tttgtcaaga ccgacctgtc cggtgccctg aatgaactgc aagacgaggc 8040
   agcgcggcta tcgtggctgg ccacgacggg cgttccttgc gcagctgtgc tcgacgttgt 8100
   cactgaagcg ggaagggact ggctgctatt gggcgaagtg ccggggcagg atctcctgtc 8160
   atctcacctt gctcctgccg agaaagtatc catcatggct gatgcaatgc ggcggctgca 8220
   tacgcttgat ccggctacct gcccattcga ccaccaagcg aaacatcgca tcgagcgagc 8280
   acgtactcgg atggaagccg gtcttgtcga tcaggatgat ctggacgaag agcatcaggg 8340
   gctcgcgcca gccgaactgt tcgccaggct caaggcgagc atgcccgacg gcgaggatct 8400
   cgtcgtgacc catggcgatg cctgcttgcc gaatatcatg gtggaaaatg gccgcttttc 8460
   tggattcatc gactgtggcc ggctgggtgt ggcggaccgc tatcaggaca tagcgttggc 8520
   tacccgtgat attgctgaag agcttggcgg cgaatgggct gaccgcttcc tcgtgcttta 8580
   cggtatcgcc gctcccgatt cgcagcgcat cgccttctat cgccttcttg acgagttctt 8640
   ctgaattttg ttaaaatttt tgttaaatca gctcattttt taaccaatag gccgaaatcg 8700
   gcaacatccc ttataaatca aaagaataga ccgcgatagg gttgagtgtt gttccagttt 8760
   ggaacaagag tccactatta aagaacgtgg actccaacgt caaagggcga aaaaccgtct 8820
   atcagggcga tggcccacta cgtgaaccat cacccaaatc aagttttttg cggtcgaggt 8880
   gccgtaaagc tctaaatcgg aaccctaaag ggagcccccg atttagagct tgacggggaa 8940
   agccggcgaa cgtggcgaga aaggaaggga agaaagcgaa aggagcgggc gctagggcgc 9000
   tggcaagtgt agcggtcacg ctgcgcgtaa ccaccacacc cgcgcgctt 9049
<210> 11
   <211> 38246
   <212> DNA
   <213> Artificial Sequence
<220>
   <223> Plasmid, enthΣlt die gesamte DNA fⁿr Ad-3
<220>
   <221> misc_feature
   <222> (1)..(38246)
   <223> "N" = "A", "C", "G" or "T"
<400> 11